# EUROPÄISCHE PATENTSCHRIFT

(11) **EP 2 477 999 B1**
(45) Veröffentlichungstag und Bekanntmachung des Hinweises auf die Patenterteilung: **23.01.2019**
(21) Anmeldenummer: 10757570.6
(22) Anmeldetag: 15.09.2010
(51) Int. Cl.: C07F 15/00, H01L 51/54

(54) **FORMULIERUNGEN ZUR HERSTELLUNG VON ELEKTRONISCHEN VORRICHTUNGEN**
FORMULATIONS FOR ELECTRONIC DEVICES
FORMULATIONS POUR DISPOSITIFS ÉLECTRONIQUES

(30) Priorität: 16.09.2009 DE 102009041414; 17.11.2009 DE 102009053644; 17.11.2009 DE 102009053645; 03.05.2010 WO PCT/EP2010/002683; 15.06.2010 EP 10006208
(43) Veröffentlichungstag der Anmeldung: 25.07.2012
(73) Patentinhaber: Merck Patent GmbH, 64293 Darmstadt (DE)
(72) Erfinder: ANÉMIAN, Rémi, Manouk, 657-169 Seoul (KR); HEUN, Susanne, 65812 Bad Soden (DE); EBERLE, Thomas, 76829 Landau (DE); STOESSEL, Philipp, 60487 Frankfurt am Main (DE)
(86) Internationale Anmeldenummer: PCT/EP2010/005648
(87) Internationale Veröffentlichungsnummer: WO 2011/032686

(56) Entgegenhaltungen:
- WO-A1-2009/021107
- WO-A1-2010/006680
- DE-A1- 10 238 903
- JP-A- 2008 127 316
- US-A1- 2006 014 046
- US-A1- 2007 013 296
- US-A1- 2008 113 101
- US-A2- 2009 191 426
- MILLER T M ET AL: "SYNTHESIS AND CHARACTERIZATION OF A SERIES OF MONODISPERSE, 1,3,5-PHENYLENE-BASED HYDROCARBON DENDRIMERS INCLUDING C276H186 AND THEIR FLUORINATED ANALOGUES", JOURNAL OF THE AMERICAN CHEMICAL SOCIETY, AMERICAN CHEMICAL SOCIETY, vol. 114, no. 3, 29 January 1992 (1992-01-29), pages 1018-1025, XP001017807, ISSN: 0002-7863, DOI: 10.1021/JA00029A034

## Beschreibung

Die vorliegende Erfindung betrifft Formulierungen zur Herstellung von elektronischen Vorrichtungen.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische FeldeffektTransistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:
(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Ein Problem, das sich in einem derartigen "Dreischichtsystem" ergibt, ist jedoch die fehlende Steuerung der Ladungstrennung oder die fehlende Möglichkeit, die einzelnen Bestandteile in unterschiedlichen Schichten bezüglich ihrer Eigenschaften zu optimieren, wie es beispielsweise bei SMOLEDs ("small-molecule OLEDs") durch einen mehrschichtigen Aufbau einfach gelöst ist.

Eine "small molecule OLED" umfasst vielfach eine oder mehrere organische Lochinjektionsschichten, Lochtransportschichten, Emissionsschichten, Elektronentransportschichten und/oder Elektroneninjektionsschichten sowie eine Anode und eine Kathode, wobei sich das ganze System gewöhnlich auf einem Glassubstrat befindet. Der Vorteil einer solchen Mehrlagenstruktur besteht darin, dass die verschiedenen Funktionen der Ladungsinjektion, des Ladungstransports und der Emission auf die verschiedenen Schichten verteilt und somit die Eigenschaften der jeweiligen Schichten separat modifiziert werden können. Durch diese Modifikation kann die Leistungsfähigkeit der elektronischen Vorrichtungen stark verbessert werden.

Nachteilig an elektronischen Vorrichtungen, die auf den zuvor beschriebenen "small molecules", also nicht polymeren Verbindungen beruhen, ist deren Herstellung. Üblicherweise werden nicht polymere Verbindungen über Verdampfungstechniken zu elektronischen Vorrichtungen verarbeitet. Dies stellt vor allem für großflächige Vorrichtungen einen großen Kostennachteil dar, da ein mehrstufiger Vakuumprozeß in verschiedenen Kammern sehr kostenintensiv ist und sehr genau gesteuert werden muß. Hier wären kostengünstigere und etablierte Beschichtungsverfahren aus Lösung wie z.B.Tintenstrahldruck, Airbrushverfahren, Rolle-zu-Rolle-Prozesse, usw., von großem Vorteil. Die zuvor beschriebenen Vorrichtungen aus "small molecules" können jedoch im allgemeinen aufgrund der geringen Löslichkeit der nicht polymeren Verbindungen in den üblichen Lösungsmitteln nicht auf diese Art und Weise hergestellt werden. Zwar kann die Löslichkeit dieser Verbindungen durch Modifikation verbessert werden, jedoch zeigen die erhaltenen elektronischen Vorrichtungen eine im Vergleich zu den über Gasphasenabscheidung erhaltenen Vorrichtungen verringerte Leistungsfähigkeit und Lebensdauer.

So werden beispielsweise in der WO 2009/021107 A1 und der WO 2010/006680 A1 organische Verbindungen beschrieben, die zur Herstellung von elektronischen Vorrichtungen geeignet sind, wobei diese Verbindungen sowohl über Gasphasenabscheidung als auch aus Lösung verarbeitet werden können. Allerdings zeigen die elektronischen Vorrichtungen, die über Gasphasenabscheidung erhalten werden, ein günstigeres Eigenschaftsprofil.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern. Zu diesen Eigenschaften gehört insbesondere die Lebensdauer der elektronischen Vorrichtungen. Ein weiteres Problem stellt insbesondere die Energieeffizienz dar, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein.

Eine weitere Aufgabe kann darin gesehen werden, elektronische Vorrichtungen mit einer ausgezeichneten Leistungsfähigkeit möglichst kostengünstig und in konstanter Qualität bereitzustellen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Formulierungen mit allen Merkmalen des Patentanspruchs 1 gelöst werden. Zweckmäßige Abwandlungen der erfindungsgemäßen Formulierungen werden in den auf Anspruch 1 rückbezogenen abhängigen Ansprüchen unter Schutz gestellt.

Gegenstand der vorliegenden Erfindung ist dementsprechend eine Formulierung umfassend mindestens ein Lösungsmittel und mindestens eine funktionale Verbindung der allgemeinen Formel (I)

A⁅B]ₖ (I)

wobei
A ein funktionales Strukturelement,
B ein löslichkeitsvermittelndes Strukturelement und
k eine ganze Zahl im Bereich von 1 bis 20 ist,
wobei das Molekulargewicht der funktionalen Verbindung mindestens 800 g/mol beträgt und
   das löslichkeitsvermittelnde Strukturelement B der allgemeinen Formel (L-II) entspricht 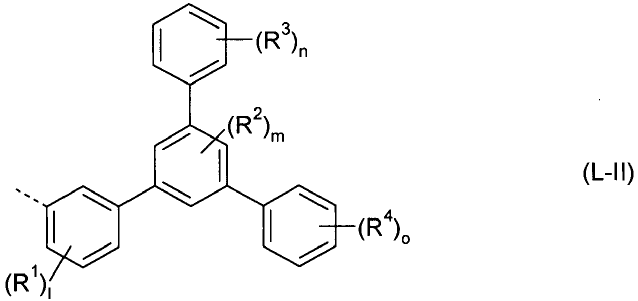
wobei
- R¹, R², R³, R⁴: jeweils unabhängig voneinander eine geradkettige Alkyl- oder Alkoxygruppe mit 1 bis 20 C-Atomen oder eine verzweigte Alkyl- oder Alkoxygruppe mit 3 bis 20 C-Atomen ist,
- l: 0, 1, 2, 3 oder 4 ist,
- m: 0, 1, 2 oder 3 ist und
- n, o: jeweils unabhängig voneinander 0, 1, 2, 3, 4 oder 5 ist;
wobei die gestrichelte Bindung die Bindung an das funktionale Strukturelement andeutet, und
die Formulierung mindestens 80 Gew.-% aromatisches oder heteroaromatisches Lösungsmittel umfasst, und wobei Verbindungen der Formel (A-III) laut Anspruch 1 ausgeschlossen sind.

Ähnliche funktionale Verbindungen werden in US2006/0014046, US2008/0113101, JP2008127316, WO2010/006680 und WO2009/021107 beschrieben.

Das Lösungsmittel umfasst mindestens eine aromatische oder heteroaromatische Verbindung. Besonders bevorzugt umfasst das Lösungsmittel mindestens einen aromatischen Kohlenwasserstoff und/oder einen Halogenaromaten, die besonders bevorzugt mindestens eine Alkylgruppe und/oder eine Cycloalkylgruppe mit 1 bis 8 Kohlenstoffatomen, vorzugsweise mit 1 bis 6 Kohlenstoffatomen aufweisen. Hierzu gehören insbesondere Toluol, Dimethylbenzol (Xylole), Trimethylbenzole, Methylnaphthaline, Tetralin, Cyclopentylbenzol und Cyclohexylbenzol.

Gemäß einer weiteren Ausgestaltung der vorliegenden Erfindung können aromatische oder heteroaromatische Verbindungen eingesetzt werden, die in der Seitengruppe Heteroatome aufweisen, insbesondere Ester, Ether, Nitrile und/oder Amide. Zu den bevorzugten Verbindungen dieser Klasse zählen aromatische Alkoxyverbindungen, wie zum Beispiel 3-Methylanisol, 2-Isopropylanisol, 5-Methoxyindan, 2-Ethoxynaphthalin, und aromatische Ester, wie zum Beispiel Butylbenzoat und Ethylbenzoat. Weiterhin sind heteroaromatische Lösungsmittel mit einem O, N oder S Atom in dem aromatischen Ring geeignet, wie zum Beispiel 2-Methylindol und 6-Methylchinolin.
Weiterhin können heterocyclische Verbindungen als Lösungsmittel eingesetzt werden, wie zum Beispiel 1-Cyclohexyl-2-pyrrolidinon (N-Cyclohexyl-pyrrolidinon).
Ferner stellen Alkohole eine geeignete Lösungsmittelklasse dar. Zu den bevorzugten Alkoholen zählen Naphthole, beispielsweise Decahydro-2-naphthol oder 1,2,3,4-Tetrahydro-1-naphthol, Terpenoide, wie zum Beispiel Nonylphenol, 1-Indanol und 2-Indanol.

Die Lösungsmittel können einzeln oder als Mischung von zwei, drei oder mehreren Verbindungen eingesetzt werden.
Zu den bevorzugten Lösungsmitteln zählen unter anderem Toluol, Anisol, Xylole, Methylbenzoat, Dimethylanisole, Mesitylene, Tetralin, Veratrol, und Chlorbenzol sowie Gemische derselben. Besonders bevorzugt werden aromatische Lösungsmittel, insbesondere aromatische Kohlenwasserstoffe eingesetzt. Die erfindungsgemäße Formulierung umfasst mindestens 80 Gew.-% und besonders bevorzugt mindestens 90 Gew-% aromatisches Lösungsmittel. Überraschende Vorteile können insbesondere durch Lösungsmittel erzielt werden, deren Löslichkeitsparameter nach Hansen vorzugsweise in den folgenden Bereichen liegen:
H_{d} (dispergierende Anteil; dispersion contribution) im Bereich von 17,0 bis 23,2 MPa^{0,5}, besonders bevorzugt im Bereich von 18,5 bis 21,0 MPa^{0,5}; Hₚ (polare Anteil; polar contribution) im Bereich von 0,2 bis 12,5 MPa^{0,5}, besonders bevorzugt im Bereich von 2,0 bis 6,0 MPa^{0,5} und
Hₕ (Wasserstoffbrückenanteil; hydrogen bonding contribution) im Bereich von 0,9 bis 14,2 MPa^{0,5}, besonders bevorzugt im Bereich von 2,0 bis 6,0 MPa^{0,5}. Die Löslichkeitsparameter nach Hansen können mit dem Computer Programm "the Hansen Solubility Parameters in Practice (HSPiP) program (2nd edition)" bestimmt werden, welches von Hanson and Abbot et al. bereitgestellt wird.
Bevorzugte funktionale Verbindungen gemäß Formel (I) können zwei, drei oder mehr der löslichkeitsvermittelnden Strukturelemente B aufweisen. Dementsprechend kann der Index k in Formel (I) eine ganze Zahl größer oder gleich 2, besonders bevorzugt größer oder gleich 3 sein.
Überraschende Vorteile können insbesondere mit funktionalen Verbindungen der allgemeinen Formel (I) mit einem relativ hohen Molekulargewicht erzielt werden. So zeichnen sich funktionale Verbindungen der allgemeinen Formel (I) durch ein Molekulargewicht von mindestens 800 g/mol, bevorzugt mindestens 900 g/mol und besonders bevorzugt mindestens 950 g/mol aus.

Weiterhin können bevorzugte funktionale Verbindungen der Formel (I) ein Molekulargewicht von höchstens 10000 g/mol, besonders bevorzugt von höchstens 5000 g/mol und ganz besonders bevorzugt von höchstens 3000 g/mol aufweisen.

Von besonderem Interesse sind des Weiteren funktionale Verbindungen, die sich durch eine hohe Glasübergangstemperatur auszeichnen. In diesem Zusammenhang sind insbesondere funktionale Verbindungen der allgemeinen Formel (I) bevorzugt, die eine Glasübergangstemperatur von mindestens 70°C, besonders bevorzugt von mindestens 100°C, ganz besonders bevorzugt von mindestens 125°C und insbesondere bevorzugt von mindestens 150°C aufweisen, bestimmt nach DIN 51005.

Das funktionale Strukturelement A der funktionalen Verbindung der Formel (I) unterliegt keiner besonderen Begrenzung, so dass die vorliegende Erfindung geeignet ist, bekannte Stoffe, die in elektronischen Vorrichtungen zum Erzielen funktionaler Eigenschaften eingesetzt werden, in eine lösliche Form zu überführen, ohne dass sich hierdurch die ursprünglichen elektronischen Eigenschaften der bekannten Stoffe in inakzeptabler Weise verändern.
Dies sind u.a. solche, wie sie in der WO 02/077060 A1 und in der WO 2005/014689 A2 offenbart und umfangreich aufgelistet sind. Die funktionalen Strukturelemente A können beispielsweise aus den folgenden Klassen stammen:

| | |
|---|---|
| Gruppe 1: | Einheiten, die Lochinjektions- und/oder Lochtransporteigenschaften erzeugen können; |
| Gruppe 2: | Einheiten, die Elektroneninjektions- und/oder Elektronentransporteigenschaften erzeugen können; |
| Gruppe 3: | Einheiten, welche lichtemittierende Eigenschaften aufweisen; |
| Gruppe 4: | Einheiten, die als Host-Materialien oder Co-Host-Materialien dienen können; |
| Gruppe 5: | Einheiten, welche den Übergang vom so genannten Singulettzum Triplettzustand verbessern. |

Strukturelemente aus der Gruppe 1, die Lochinjektions- und/oder Lochtransporteigenschaften aufweisen, sind beispielsweise Triarylamin-, Benzidin-, Tetraaryl-para-phenylendiamin-, Triarylphosphin-, Phenothiazin-, Phenoxazin-, Dihydrophenazin-, Thianthren-, Dibenzo-para-dioxin-, Phenoxathiin-, Carbazol-, Azulen-, Thiophen-, Pyrrol- und Furanderivate und weitere O-, S- oder N-haltige Heterocyclen mit hoch liegendem HOMO (HOMO = höchstes besetztes Molekülorbital).

Insbesondere zu nennen sind als Strukturelemente aus der Gruppe 1, die Lochinjektions- und/oder Lochtransporteigenschaften aufweisen, Phenylendiamin-Derivate (US 3615404), Arylamin-Derivate (US 3567450), Amino-substituierte Chalcon-Derivate (US 3526501), Styrylanthracen-Derivate (JP-A-56-46234), Polyzyklische aromatische Verbindungen (EP 1009041), Polyarylalkan-Derivate (US 3615402), Fluorenon-Derivate (JP-A-54-110837), Hydrazon-Derivate (US 3717462), Acylhydrazone, Stilben-Derivate (JP-A-61-210363), Silazan-Derivate (US 4950950), Polysilane (JP-A-2-204996), Anilin-Copolymere (JP-A-2-282263), Thiophen-Oligomere (JP Heisei 1 (1989) 211399), Polythiophene, Poly(N-vinylcarbazol) (PVK), Polypyrrole, Polyaniline und andere elektrisch leitende Makromoleküle, Porphyrin-Verbindungen (JP-A-63-2956965, US 4720432), aromatische Dimethyliden-Typ-Verbindungen, Carbazol-Verbindungen wie z.B. CDBP, CBP, mCP, aromatische tertiäre Amin- und Styrylamin-Verbindungen (US 4127412) wie z.B. Triphenylamine vom Benzidin-Typ, Triphenylamine vom Styrylamin-Typ und Triphenylamine vom Diamin-Typ. Auch Arylamin-Dendrimere können verwendet werden (JP Heisei 8 (1996) 193191), monomere Triarylamine (US 3180730), Triarylamine mit einem oder mehreren Vinylradikalen und/oder mindestens einer funktionellen Gruppe mit aktivem Wasserstoff (US 3567450 und US 3658520) oder Tetraaryldiamine (die zwei Tertiäramineinheiten sind über eine Arylgruppe verbunden). Es können auch noch mehr Triarylaminogruppen im Molekül vorhanden sein. Auch Phthalocyanin-Derivate, Naphthalocyanin-Derivate, Butadien-Derivate und Chinolinderivate wie z.B. Dipyrazino[2,3-f:2',3'-h]quinoxalinhexacarbonitril sind geeignet.

Bevorzugt sind aromatische tertiäre Amine mit mindestens zwei Tertiäramin-Einheiten (US 2008/0102311 A1, US 4720432 und US 5061569), wie z.B. NPD (α-NPD = 4,4'-bis[N-(1-naphthyl)-N-phenylamino]biphenyl) (US 5061569), TPD 232 (= N,N'-Bis-(N,N'-diphenyl-4-aminophenyl)-N,N-diphenyl-4,4'-diamino-1,1'-biphenyl) oder MTDATA (MTDATA oder m-MTDATA= 4, 4', 4"-Tris[3-methylphenyl)phenyl-amino]triphenylamin) (JP-A-4-308688), TBDB (= N,N,N',N'-Tetra(4-biphenyl)diaminobiphenylen), TAPC (= 1,1-Bis(4-di-p-tolylaminophenyl)-cyclohexan), TAPPP (= 1,1-Bis(4-di-p-tolylaminophenyl)-3-phenylpropan), BDTAPVB (= 1,4-Bis[2-[4-[N,N-di(p-tolyl)amino]phenyl]vinyl]benzol), TTB (= N,N,N',N'-Tetra-p-tolyl-4,4'-diaminobiphenyl), TPD (= 4,4'-Bis[N-3-methylphenyl]-N-phenylamino)biphenyl), N,N,N',N'-Tetraphenyl-4,4"'-diamino-1,1',4',1",4",1'"-quaterphenyl, ebenso tertiäre Amine mit Carbazol-Einheiten wie z.B. TCTA (= 4-(9H-Carbazol-9-yl)-N,N-bis[4-(9H-carbazol-9-yl)phenyl]benzolamin). Ebenfalls bevorzugt sind Hexaaza-Triphenylen-Verbindungen gemäß US 2007/0092755 A1 sowie Phthalocyanin-Derivate (z.B. H₂Pc, CuPc (= Kupfer-Phthalocyanin), CoPc, NiPc, ZnPc, PdPc, FePc, MnPc, ClAlPc, ClGaPc, ClInPc, ClSnPc, Cl₂SiPc, (HO)AlPc, (HO)GaPc, VOPc, TiOPc, MoOPc, GaPc-O-GaPc).

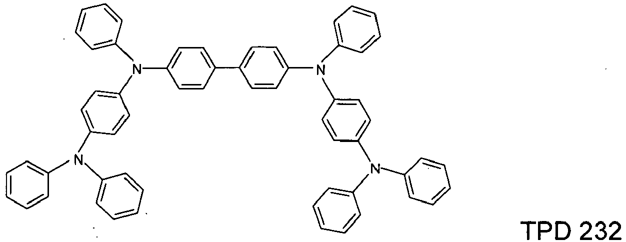

Besonders bevorzugt sind folgende Triarylamin-Verbindungen, die auch substituiert sein können: 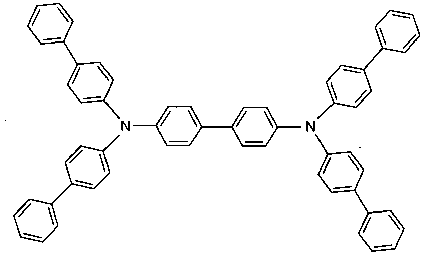
TBDB: EP 1162193 B1 und EP 650955 B1 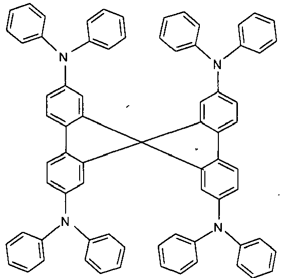
Synth.Metals 1997, 91(1-3), 209 und DE 19646119 A1 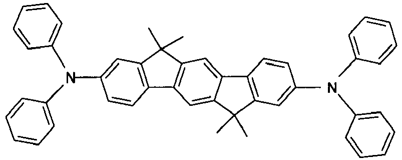
WO 2006 122630 A1 und EP 1860097 A1 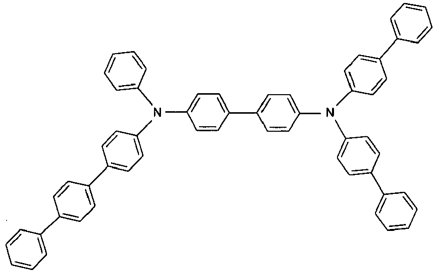
EP 1834945 A1 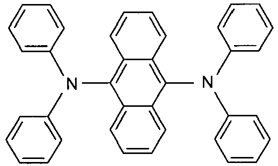
JP 08053397 A und US 6251531 B1 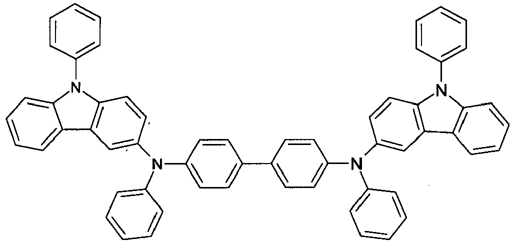
EP 1661888 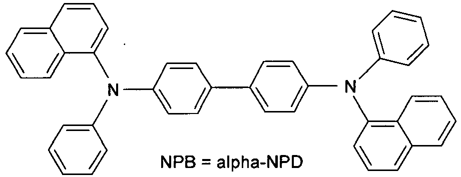
NPB = 4,4'-Bis[N-(1-naphthyl)-N-phenylamino]biphenyl 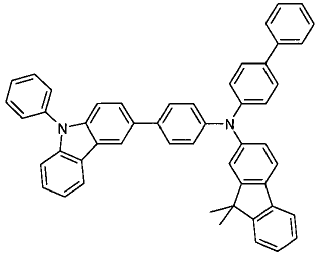
US 2005/0221124, WO 09/041635 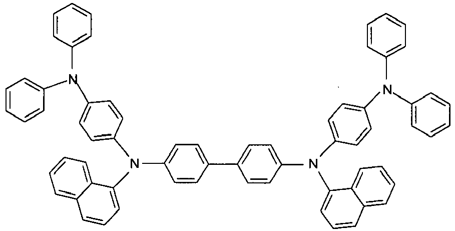
US 7399537 B2, US 2006 0061265 A1 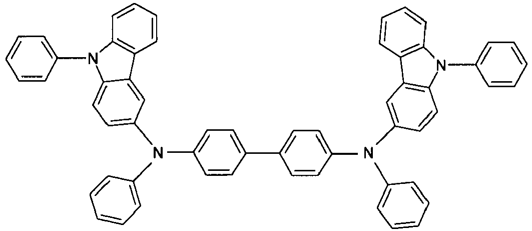
EP 1661888 B1 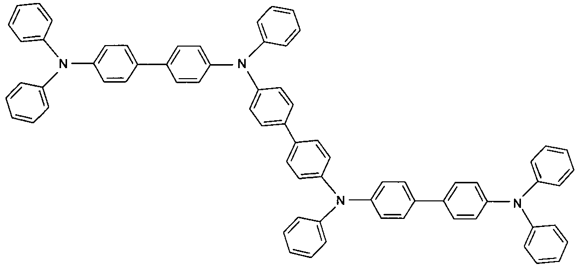
JP 08292586 A

Weitere Strukturelemente, die als Lochinjektionsmaterialien eingesetzt werden können, sind beschrieben in der EP 0891121 A1 und der EP 1029909 A1, Injektionsschichten allgemein in der US 2004/0174116 A1.

Vorzugsweise führen diese Arylamine und Heterocyclen, die im allgemeinen als Strukturelemente der Gruppe 1 eingesetzt werden, zu einem HOMO im Polymer von mehr als -5,8 eV (gegen Vakuumlevel), besonders bevorzugt von mehr als -5,5 eV.

Strukturelemente aus der Gruppe 2, die Elektroneninjektions- und/oder Elektronentransporteigenschaften aufweisen, sind beispielsweise Pyridin-, Pyrimidin-, Pyridazin-, Pyrazin-, Oxadiazol-, Chinolin-, Chinoxalin-, Anthracen-, Benzanthracen-, Pyren-, Perylen-, Benzimidazol-, Triazin-, Keton-, Phosphinoxid- und Phenazinderivate, aber auch Triarylborane und weitere O-, S- oder N-haltige Heterocyclen mit niedrig liegendem LUMO (LUMO = niedrigstes unbesetztes Molekülorbital).

Besonders geeignete Strukturelemente für elektronentransportierende und elektroneninjizierende Schichten sind Metallchelate von 8-Hydroxychinolin (z.B. LiQ, AlQ₃, GaQ₃, MgQ₂, ZnQ₂, InQ₃, ZrQ₄), BAIQ, Ga-Oxinoid-Komplexe, 4-Azaphenanthren-5-ol-Be-Komplexe (US 5529853 A),

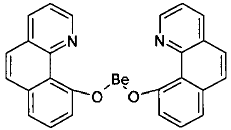

Butadienderivate (US 4356429), heterozyklische optische Aufheller (US 4539507), Benzimidazol-Derivate (US 2007/0273272 A1), wie z.B. TPBI (US 5766779), 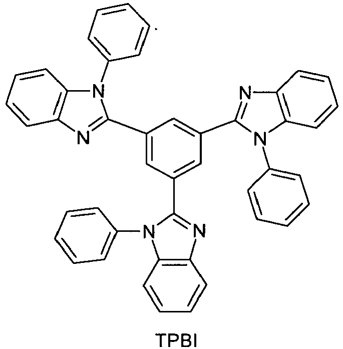 2,2',2"-(1,3,5-benzenetriyl)tris(1-phenyl-1H-benzimidazole)

1,3,5-Triazine, z.B. Spirobifluoren-Triazin-Derivate (z.B. gemäß der DE 102008064200), Pyrene, Anthracene, Tetracene, Fluorene, Spirofluorene, Dendrimere, Tetracene (z.B. Rubren-Derivate), 1,10-Phenanthrolin-Derivate (JP 2003-115387, JP 2004-311184, JP-2001-267080, WO 2002/043449), Sila-Cyclopentadien-Derivate (EP 1480280, EP 1478032, EP 1469533), Boran-Derivate wie z.B. Triarylboranderivate mit Si, 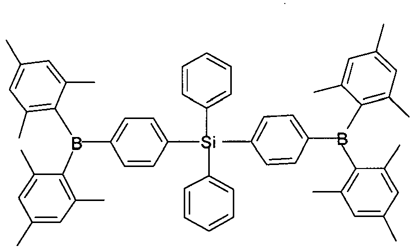 US 2007/0087219 A1

Pyridin-Derivate (JP 2004-200162), Phenanthroline, vor allem 1,10-Phenanthrolinderivate, wie z.B. BCP und Bphen, auch mehrere über Biphenyl oder andere aromatische Gruppen verbundene Phenanthroline (US-2007-0252517 A1) oder mit Anthracen verbundene Phenanthroline (US 2007-0122656 A1).

| | |
|---|---|
| 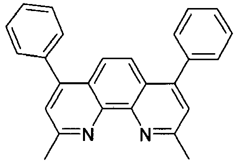 | 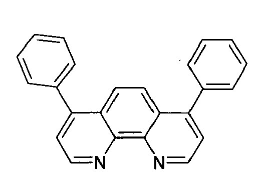 |
| Bathocuproin | Bphen |

Ebenfalls geeignet sind heterozyklische organische Verbindungen wie z.B. Thiopyrandioxide, Oxazole, Triazole, Imidazole oder Oxadiazole. Beispiele für die Verwendung von Fünfringen mit N wie z.B. Oxazole, Thiazole, Oxadiazole, Thiadiazole, Triazole u.a. siehe US 2008/0102311 A1. Bevorzugte Verbindungen sind folgende:
Triazole, z.B. 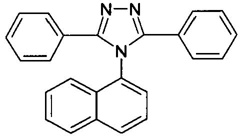
Y.A. Levin, M.S. Skorobogatova, Khimiya Geterotsiklicheskikh Soedinenii 1967 (2), 339-341.
1,3,4-Oxadiazole, z.B. 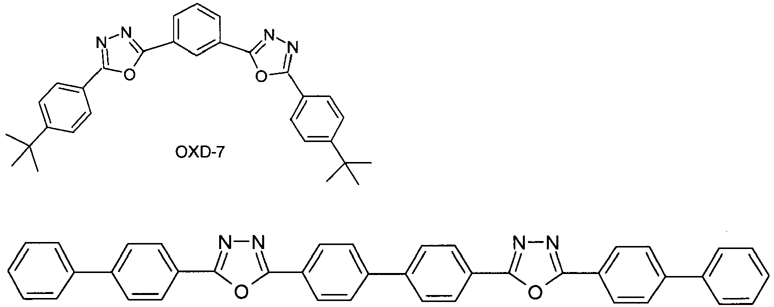
US 2007/0273272 A1 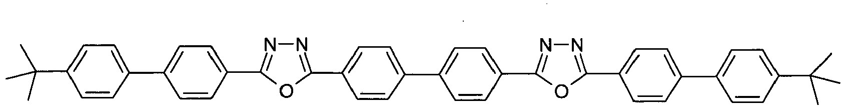
US 2007/0273272 A1 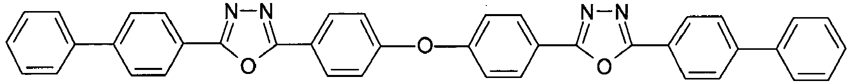
US 2007/0273272 A1

Auch organische Verbindungen wie Derivate von Fluorenon, Fluorenylidenmethan, Perylentetrakohlensäure, Anthrachinondimethan, Diphenochinon, Anthron und Anthrachinondiethylendiamin können eingesetzt werden.

Bevorzugt sind 2,9,10-substituierte Anthracene (mit 1- oder 2-Naphthyl und 4- oder 3-Biphenyl) oder Moleküle, die zwei Anthraceneinheiten enthalten (US2008/0193796 A1). Sehr vorteilhaft ist auch die Verbindung von 9,10-substituierten Anthracen-Einheiten mit Benzimidazol-Derivaten.

| | |
|---|---|
| 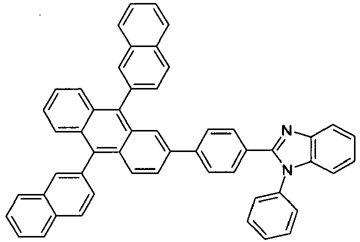 | |
| US 6878469 B2 | |
| 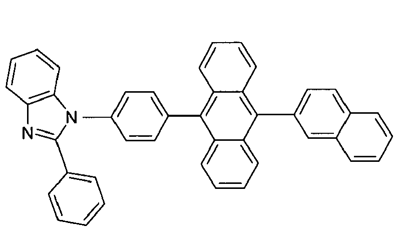 | 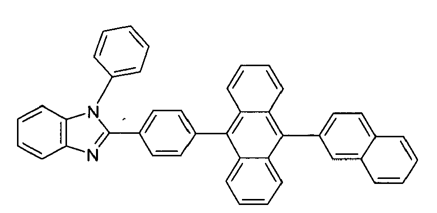 |
| US 2006 147747 A | EP 1551206 A1 |

Vorzugsweise führen die Strukturelemente aus der Gruppe 2 in einer erfindungsgemäß einzusetzenden Verbindung gemäß Formel (I) zu einem LUMO von weniger als -2,5 eV (gegen Vakuumlevel), besonders bevorzugt von weniger als -2,7 eV.

Strukturelemente aus der Gruppe 3 sind solche, welche Licht emittieren können. Hierzu gehören unter anderem Verbindungen mit Stilben-, Stilbenamin-, Styrylamin-, Coumarin-, Rubren-, Rhodamin-, Thiazol-, Thiadiazol-, Cyanin-, Thiophen-, Paraphenylen-, Perylen-, Phatolocyanin-, Porphyrin-, Keton-, Chinolin-, Imin-, Anthracen- und/oder Pyren-Strukturen. Besonders bevorzugt sind Verbindungen, die auch bei Raumtemperatur mit hoher Effizienz aus dem Triplettzustand Licht emittieren können, also Elektrophosphoreszenz statt Elektrofluoreszenz zeigen, was häufig eine Steigerung der Energieeffizienz bewirkt. Hierfür eignen sich zunächst Verbindungen, welche Schweratome mit einer Ordnungszahl von mehr als 36 enthalten. Bevorzugt sind Verbindungen, welche d- oder f-Übergangsmetalle enthalten, die die o.g. Bedingung erfüllen. Besonders bevorzugt sind hier entsprechende Strukturelemente, welche Elemente der Gruppe 8 bis 10 (Ru, Os, Rh, Ir, Pd, Pt) enthalten. Als funktionale Strukturelemente A für die Verbindungen gemäß Formel (I) kommen hier z.B. verschiedene Komplexe in Frage, wie sie z.B. in der WO 02/068435 A1, der WO 02/081488 A1, der EP 1239526 A2 und der WO 04/026886 A2 beschrieben werden.

Nachfolgend werden beispielhaft bevorzugte Strukturelemente aus der Gruppe 3 dargelegt, die als fluoreszierende Emitter dienen können. Bevorzugte Strukturelemente aus der Gruppe 3 sind ausgewählt aus der Klasse der Monostyrylamine, der Distyrylamine, der Tristyrylamine, der Tetrastyrylamine, der Styrylphosphine, der Styrylether und der Arylamine.

Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne der vorliegenden Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, vorzugsweise mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 2,6- oder 9,10-Position. Aromatische Pyrenamine, Pyrendiamine, Chrysenamine und Chrysendiamine sind analog dazu definiert, wobei die Diarylaminogruppen am Pyren vorzugsweise in 1-Position bzw. in 1,6-Position gebunden sind.

Weitere bevorzugte Strukturelemente aus der Gruppe 3 sind ausgewählt aus Indenofluorenaminen bzw. -diaminen, beispielsweise gemäß der WO 06/122630, Benzoindenofluorenaminen bzw. -diaminen, beispielsweise gemäß der WO 08/006449, und Dibenzoindenofluorenaminen bzw. -diaminen, beispielsweise gemäß der WO 07/140847.

Beispiele für Strukturelemente der Gruppe 3 aus der Klasse der Styrylamine sind substituierte oder unsubstituierte Tristilbenamine oder die Dotanden, die in der WO 06/000388, der WO 06/058737, der WO 06/000389, der WO 07/065549 und der WO 07/115610 beschrieben sind. Distyrylbenzol- und Distyrylbiphenyl-Derivate sind beschrieben in der US 5121029. Weitere Styrylamine sind in der US 2007/0122656 A1 zu finden.

Besonders bevorzugte Styrylamin-Strukturelemente der Gruppe 3 sind: 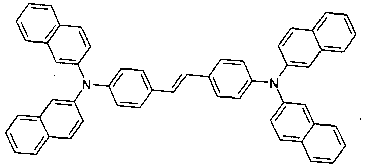
US 7250532 B2 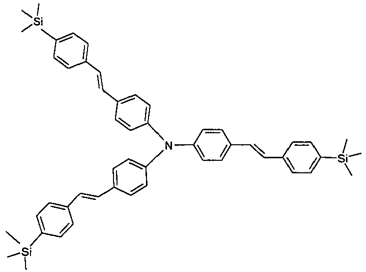
DE 10 2005 058557 A1

Besonders bevorzugte Triarylamin-Strukturelemente der Gruppe 3 sind:

| | |
|---|---|
| 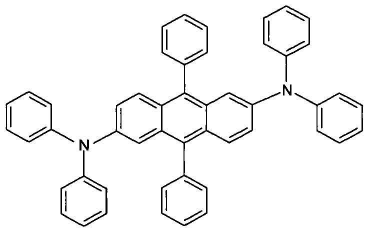 | 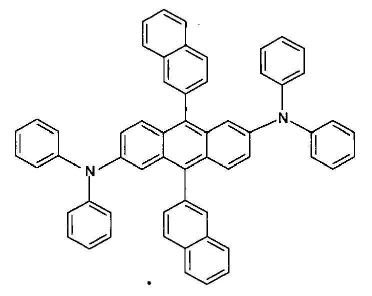 |
| CN 1583691 A | CN 1583691 A |
| 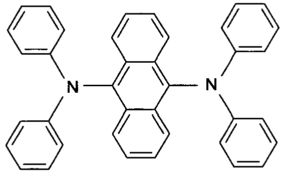 | |
| JP 08/053397 A und US 6251531 B1, Derivate in EP 1957606 A1 und US 2008/0113101 A1. | |
| 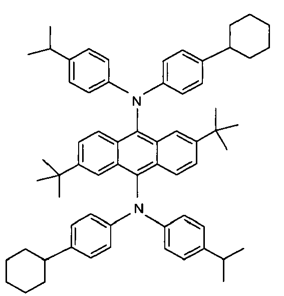 | 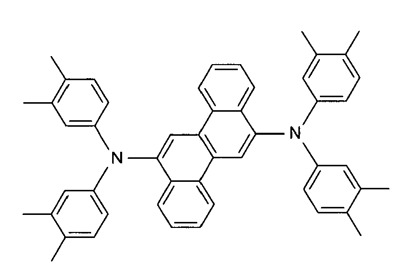 |
| EP 1957606 A1 | US 2006/210830 A |
| 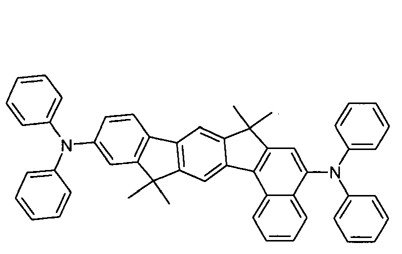 | 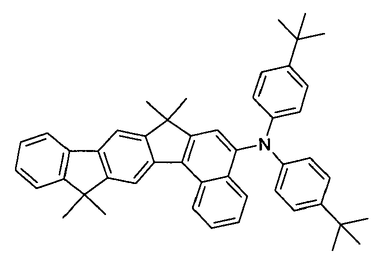 |
| WO 08/006449 | WO 08/006449 |
| 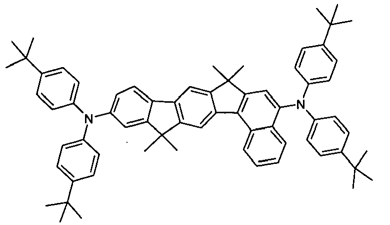 | 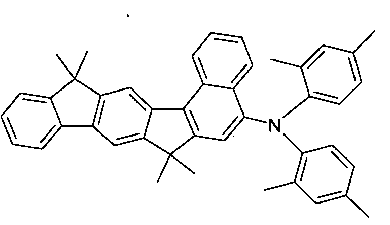 |
| WO 08/006449 | WO 08/006449 |
| 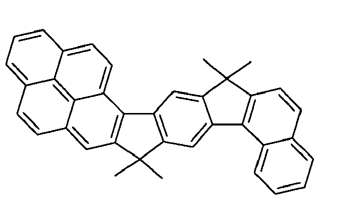 | 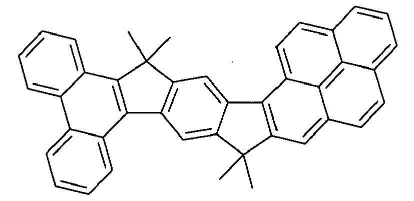 |
| DE 102008035413 | DE 102008035413 |
| 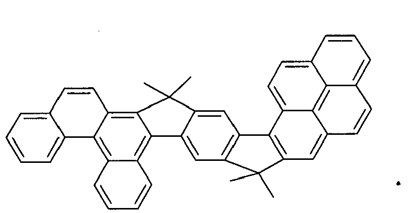 | 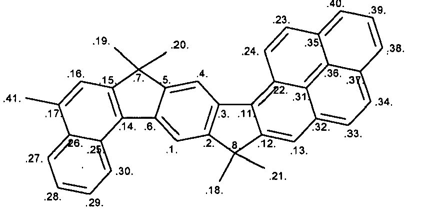 |
| DE 102008035413 | DE 102008035413 |

Weitere bevorzugte Strukturelemente der Gruppe 3 sind ausgewählt aus Derivaten von Naphthalin, Anthracen, Tetracen, Benzanthracen, Benzphenanthren (DE 10 2009 005746), Fluoren, Fluoranthen, Periflanthen, Indenoperylen, Phenanthren, Perylen (US 2007/0252517 A1), Pyren, Chrysen, Decacyclen, Coronen, Tetraphenylcyclopentadien, Pentaphenylcyclopentadien, Fluoren, Spirofluoren, Rubren, Cumarin (US 4769292, US 6020078, US 2007/0252517 A1), Pyran, Oxazol, Benzoxazol, Benzothiazol, Benzimidazol, Pyrazin, Zimtsäureestern, Diketopyrrolopyrrol, Acridon und Chinacridon (US 2007/0252517 A1).

Von den Anthracenverbindungen sind besonders bevorzugt in 9,10-Position substituierte Anthracene wie z.B. 9,10-Diphenylanthracen und 9,10-Bis(phenylethynyl)anthracen. Auch 1,4-Bis(9'-ethynylanthracenyl)-benzol ist ein bevorzugter Dotand.

Ebenfalls bevorzugt sind Derivate von Rubren, Cumarin, Rhodamin, Chinacridon wie z.B. DMQA (= N,N'-dimethylchinacridon), Dicyanomethylenpyran wie z.B. DCM (= 4-(dicyanoethylen)-6-(4-dimethylamino-styryl-2-methyl)-4H-pyran), Thiopyran, Polymethin, Pyrylium- und Thiapyryliumsalzen, Periflanthen und Indenoperylen.

Blaue Fluoreszenzemitter sind vorzugsweise Polyaromaten wie z.B. 9,10-Di(2-naphthylanthracen) und andere Anthracen-Derivate, Derivate von Tetracen, Xanthen, Perylen wie z.B. 2,5,8,11-Tetra-*t*-butyl-perylen, Phenylen, z.B. 4,4'-(Bis(9-ethyl-3-carbazovinylen)-1,1'-biphenyl, Fluoren, Fluoranthen, Arylpyrene (US 2006/0222886 A1), Arylenvinylene (US 5121029, US 5130603), Bis(azinyl)imin-Bor-Verbindungen (US 2007/0092753 A1), Bis(azinyl)methenverbindungen und Carbostyryl-Verbindungen.

Weitere bevorzugte blaue Fluoreszenzemitter sind in C.H.Chen et al.: "Recent developments in organic electroluminescent materials" Macromol. Symp. 125, (1997) 1-48 und "Recent progress of molecular organic electroluminescent materials and devices" Mat. Sci. and Eng. R, 39 (2002), 143-222 beschrieben.

Weitere bevorzugte blau fluoreszierende Emitter sind die in der DE 102008035413 offenbarten Kohlenwasserstoffe.

Nachfolgend werden beispielhaft bevorzugte Strukturelemente aus der Gruppe 3 dargelegt, die als phosphoreszierende Emitter dienen können.

Beispiele für phosphoreszierende Emitter können der WO 00/70655, der WO 01/41512, der WO 02/02714, der WO 02/15645, der EP 1191613, der EP 1191612, der EP 1191614 und der WO 05/033244 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Phosphoreszierende Metallkomplexe enthalten vorzugsweise Ir, Ru, Pd, Pt, Os oder Re.

Bevorzugte Liganden sind 2-Phenylpyridin-Derivate, 7,8-Benzochinolin-Derivate, 2-(2-Thienyl)pyridin-Derivate, 2-(1-Naphthyl)pyridin-Derivate, 1-Phenylisochinolin-Derivate, 3-Phenylisochinolin-Derivate oder 2-Phenylchinolin-Derivate. Alle diese Verbindungen können substituiert sein, z.B. für Blau mit Fluor-, Cyano- und/oder Trifluormethylsubstituenten. Auxiliäre Liganden sind vorzugsweise Acetylacetonat oder Picolinsäure.

Insbesondere sind Komplexe von Pt oder Pd mit tetradentaten Liganden,

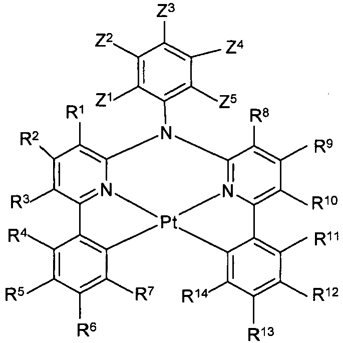

(US 2007/0087219 A1, wobei zur Erläuterung der Substituenten und Indices in obiger Formel auf diese Druckschrift zu Offenbarungszwecken verwiesen wird), Pt-Porphyrinkomplexe mit vergrößertem Ringsystem (US 2009/0061681 A1) und Ir-Komplexe geeignet, z.B. 2,3,7,8,12,13,17,18-Octaethyl-21H, 23H-porphyrin-Pt(II), Tetraphenyl-Pt(II)-tetrabenzoporphyrin (US 2009/0061681 A1), *cis*-Bis(2-phenylpyridinato-N,C^{2'})Pt(II), *cis*-Bis(2-(2'-thienyl)pyridinato-N,C^{3'})Pt(II), *cis*-Bis-(2-(2'-thienyl)chinolinato-N,C^{5'})Pt(II), (2-(4,6-Difluorophenyl)pyridinato-N,C^{2'})Pt(II)(acetylacetonat), oder Tris(2-phenylpyridinato-N,C^{2'})Ir(III) (= Ir(ppy)₃, grün), Bis(2-phenyl-pyridinato-N,C²)Ir(III)(acetylacetonat) (= Ir(ppy)₂acetylacetonat, grün, US 2001/0053462 A1, Baldo, Thompson et al. Nature 403, (2000), 750-753), Bis(1-phenylisochinolinato-N,C^{2'})(2-phenylpyridinato-N,C^{2'})Iridium(III), Bis(2-phenylpyridinato-N,C^{2'})(1-phenylisochinolinato-N,C^{2'})Iridium(III), Bis(2-(2'-benzothienyl)pyridinato-N,C^{3'})Iridium(III)(acetylacetonat), Bis(2-(4',6'-difluorophenyl)pyridinato-N,C^{2'})Iridium(III)(piccolinat) (FIrpic, blau), Bis(2-(4',6'-difluorophenyl)pyridinato-N,C^{2'})Ir(III)(tetrakis(1-pyrazolyl)borat), Tris(2-(biphenyl-3-yl)-4-tertbutylpyridin)iridium(III), (ppz)₂Ir(5phdpym) (US 2009/0061681 A1), (45ooppz)₂Ir(5phdpym) (US 2009/0061681 A1), Derivate von 2-Phenylpyridin-Ir-Komplexen, wie z.B. PQIr (= Iridium(III)-bis(2-phenyl-quinolyl-N,C^{2'})acetylacetonat), Tris(2-phenylisochinolinato-N,C)Ir(III) (rot), Bis(2-(2'-benzo[4,5-a]thienyl)pyridinato-N,C³)Ir(acetylacetonat) ([Btp₂Ir(acac)], rot, Adachi et al. Appl. Phys. Lett. 78 (2001), 1622-1624).

Ebenfalls geeignet sind Komplexe von trivalenten Lanthaniden wie z.B. Tb³⁺ und Eu³⁺ (J.Kido et al. Appl. Phys. Lett. 65 (1994), 2124, Kido et al. Chem. Lett. 657, 1990, US 2007/0252517 A1) oder phosphoreszente Komplexe von Pt(II), Ir(I), Rh(I) mit Maleonitrildithiolat (Johnson et al., JACS 105, 1983, 1795), Re(I)-Tricarbonyl-diimin-Komplexe (Wrighton, JACS 96, 1974, 998 u.a.), Os(II)-Komplexe mit Cyanoliganden und Bipyridyl- oder Phenanthrolin-Liganden (Ma et al., Synth. Metals 94, 1998, 245).

Weitere phosphoreszierende Emitter mit tridentaten Liganden werden beschrieben in der US 6824895 und der US 10/729238. Rot emittierende phosphoreszente Komplexe findet man in der US 6835469 und der US 6830828.

Besonders bevorzugte Strukturelemente, die als phosphoreszierende Dotanden Verwendung finden, sind: 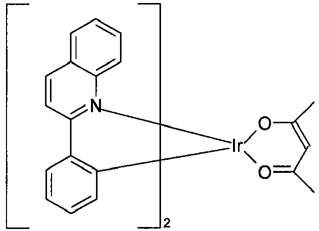 US 2001/0053462 A1 und Inorg. Chem. 2001, 40(7), 1704-1711, JACS 2001, 123(18), 4304-4312.

Derivate sind beschrieben in der US 7378162 B2, der US 6835469 B2 und der JP 2003/253145 A.

| | |
|---|---|
| 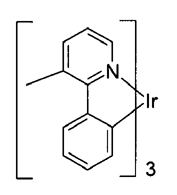 | 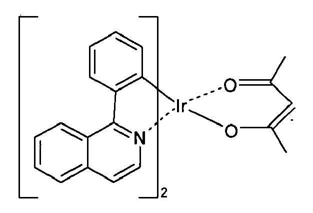 |
| US 7238437 B2 | US 2009/008607 A1 |
| 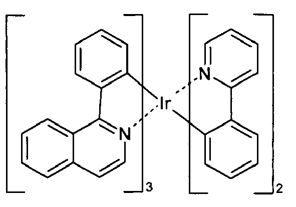 | 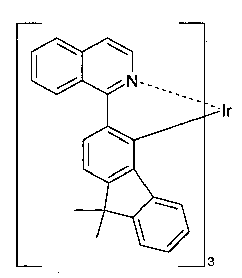 |
| US 2009/008607 | A1 EP 1348711 |

Strukturelemente aus der Gruppe 4, die als Hostmaterialien, insbesondere zusammen mit emittierenden Verbindungen eingesetzt werden, umfassen Materialien verschiedener Stoffklassen.

Bevorzugte Strukturelemente aus der Gruppe 4, die insbesondere zusammen mit fluoreszierenden Dotanden eingesetzt werden, sind ausgewählt aus den Klassen der Oligoarylene (z.B. 2,2',7,7'-Tetraphenylspirobifluoren gemäß EP 676461 oder Dinaphthylanthracen), insbesondere der Oligoarylene enthaltend kondensierte aromatische Gruppen wie z.B. Anthracen, Benzanthracen, Benzphenanthren (DE 10 2009 005746, WO 09/069566), Phenanthren, Tetracen, Coronen, Chrysen, Fluoren, Spirofluoren, Perylen, Phthaloperylen, Naphthaloperylen, Decacyclen, Rubren, der Oligoarylenvinylene (z.B. DPVBi = 4,4'-Bis(2,2-diphenyl-ethenyl)-1,1'-biphenyl) oder Spiro-DPVBi gemäß EP 676461), der polypodalen Metallkomplexe (z.B. gemäß WO 04/081017), insbesondere Metallkomplexe von 8-Hydroxychinolin, z.B. AlQ₃ (= Aluminium(III)tris(8-hydroxychinolin)) oder Bis(2-methyl-8-chinolinolato)-4-(phenylphenolinolato)aluminium, auch mit Imidazol-Chelat (US 2007/0092753 A1) sowie der Chinolin-Metallkomplexe, Aminochinolin-Metallkomplexe, Benzochinolin-Metallkomplexe, der lochleitenden Verbindungen (z.B. gemäß WO 04/058911), der elektronenleitenden Verbindungen, insbesondere Ketone, Phosphinoxide, Sulfoxide, etc. (z.B. gemäß der WO 05/084081 und der WO 05/084082), der Atropisomere (z.B. gemäß der WO 06/048268), der Boronsäurederivate (z.B. gemäß der WO 06/117052) oder der Benzanthracene (z.B. gemäß der WO 08/145239).

Besonders bevorzugte Strukturelemente der Gruppe 4 sind ausgewählt aus den Klassen der Oligoarylene, enthaltend Anthracen, Benzanthracen und/oder Pyren oder Atropisomere dieser Verbindungen. Unter einem Oligoarylen im Sinne der vorliegenden Erfindung soll eine Verbindung verstanden werden, in der mindestens drei Aryl- bzw. Arylengruppen aneinander gebunden sind.

Bevorzugte Hostmaterialien sind insbesondere ausgewählt aus Verbindungen der Formel (H-1),

Ar³-(Ar⁴)ₚ-Ar⁵ (H-1)

wobei Ar³, Ar⁴, Ar⁵ bei jedem Auftreten gleich oder verschieden eine Aryl- oder Heteroarylgruppe mit 5 bis 30 aromatischen Ringatomen ist, die mit einem oder mehreren Resten R¹ substituiert sein kann, und R¹ dieselbe Bedeutung hat, wie oben beschrieben, und p eine ganze Zahl im Bereich von 1 bis 5 darstellt; dabei gilt, dass die Summe der π-Elektronen in Ar³, Ar⁴ und Ar⁵ mindestens 30 beträgt, wenn p = 1 ist, und mindestens 36 beträgt, wenn p = 2 ist, und mindestens 42 beträgt, wenn p = 3 ist.

Besonders bevorzugt steht in den Verbindungen der Formel (H-1) die Gruppe Ar⁴ für Anthracen, welches durch einen oder mehrere Reste R¹ substituiert sein kann, und die Gruppen Ar³ und Ar⁵ sind in 9- und 10-Position gebunden. Ganz besonders bevorzugt ist mindestens eine der Gruppen Ar³ und/oder Ar⁵ eine kondensierte Arylgruppe, ausgewählt aus 1- oder 2-Naphthyl, 2-, 3- oder 9-Phenanthrenyl oder 2-, 3-, 4-, 5-, 6- oder 7-Benzanthracenyl, welche jeweils durch einen oder mehrere Reste R¹ substituiert sein kann. Anthracen-basierte Verbindungen sind beschrieben in der US 2007/0092753 A1 und der US 2007/0252517 A1, z.B. 2-(4-Methylphenyl)-9,10-di-(2-naphthyl)anthracen, 9-(2-Naphthyl)-10-(1,1'-biphenyl)anthracen und 9,10-Bis[4-(2,2-diphenylethenyl)phenyl]anthracen, 9,10-Diphenylanthracen, 9,10-Bis(phenylethynyl)anthracen und 1,4-Bis(9'-ethynylanthracenyl)benzol. Bevorzugt sind auch Verbindungen mit zwei Anthraceneinheiten (US 2008/0193796 A1), z.B. 10,10'-Bis[1,1',4', 1"]terphenyl-2-yl-9,9'-bisanthracenyl.

Weitere bevorzugte Verbindungen sind Derivate von Arylamin, Styrylamin, Fluorescein, Diphenylbutadien, Tetraphenylbutadien, Cyclopentadiene, Tetraphenylcyclopentadien, Pentaphenylcyclopentadien, Cumarin, Oxadiazol, Bisbenzoxazolin, Oxazol, Pyridin, Pyrazin, Imin, Benzothiazol, Benzoxazol, Benzimidazol (US 2007/0092753 A1), z.B. 2,2',2"-(1,3,5-Phenylen)tris[1-phenyl-1H-benzimidazol], Aldazin, Stilben, Styrylarylenderivate, z.B. 9,10-Bis[4-(2,2-diphenylethenyl)phenyl]anthracen und Distyrylarylen-Derivate (US 5121029), Diphenylethylen, Vinylanthracen, Diaminocarbazol, Pyran, Thiopyran, Diketopyrrolopyrrol, Polymethin, Zimtsäureestern und Fluoreszenzfarbstoffen.

Besonders bevorzugt sind Derivate von Arylamin und Styrylamin, z.B. TNB (= 4,4'-Bis[N-(1-naphthyl)-N-(2-naphthyl)amino]biphenyl). Metall-Oxinoid-Komplexe wie LiQ oder AlQ₃ können als Co-Hosts verwendet werden.

Bevorzugte Verbindung mit Oligoarylen als Matrix:

| | |
|---|---|
| 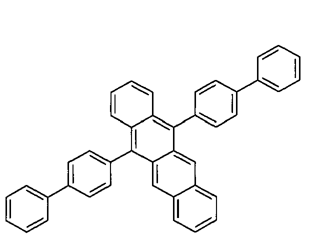 | 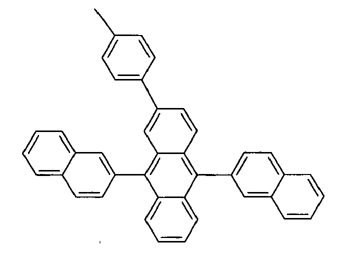 |
| US 2003/0027016 A1 | US 7326371 B2 |
| 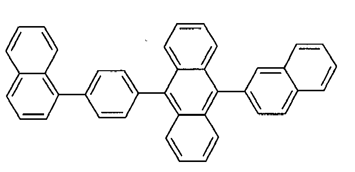 | 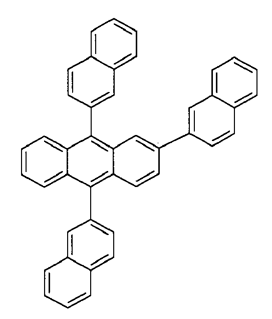 |
| US 2006/043858 A | US 7326371 B2 |
| 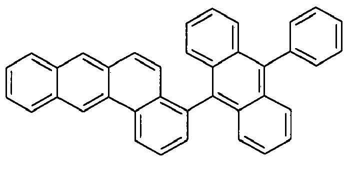 | 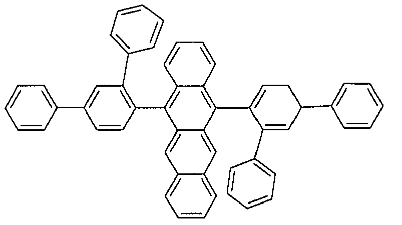 |
| WO 08/145239 | US 2003/0027016 A1 |
| 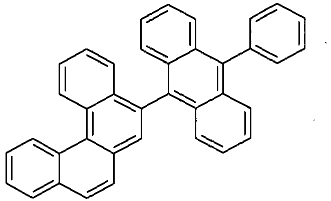 | |
| DE 102009005746 | |

Weiterhin umfassen Strukturelemente der Gruppe 4 Materialien, die zusammen mit phosphoreszierenden Emittern eingesetzt werden. Zu diesen Strukturelementen gehören CBP (N,N-Biscarbazolylbiphenyl), Carbazolderivate (z.B. gemäß WO 05/039246, US 2005/0069729, JP 2004/288381, EP 1205527 oder WO 08/086851), Azacarbazole (z.B. gemäß EP 1617710, EP 1617711, EP 1731584, JP 2005/347160), Ketone (z.B. gemäß WO 04/093207 oder gemäß der DE 102008033943), Phosphinoxide, Sulfoxide und Sulfone (z.B. gemäß WO 05/003253), Oligophenylene, aromatische Amine (z.B. gemäß US 2005/0069729), bipolare Matrixmaterialien (z.B. gemäß WO 07/137725), Silane (z.B. gemäß WO 05/111172), 9,9-Diarylfluorenderivate (z.B. gemäß der DE 102008017591), Azaborole oder Boronester (z.B. gemäß WO 06/117052), Triazin-Derivate (z.B. gemäß der DE 102008036982), Indolocarbazolderivate (z.B. gemäß WO 07/063754 oder WO 08/056746), Indenocarbazolderivate (z.B. gemäß der DE 102009023155 und der DE 102009031021), Diazaphospholderivate (z.B. gemäß der DE 102009022858), Triazol-Derivate, Oxazole und Oxazol-Derivate, ImidazolDerivate, Polyarylalkan-Derivate, Pyrazolin-Derivate, Pyrazolon-Derivate, Distyrylpyrazin-Derivate, Thiopyrandioxid-Derivate, Phenylendiamin-Derivate, tertiäre aromatische Amine, Styrylamine, Amino-substituierte Chalcon-Derivate, Indole, Hydrazon-Derivate, Stilben-Derivate, Silazan-Derivate, aromatische Dimethyliden-Verbindungen, Carbodiimid-Derivate, Metallkomplexe von 8-Hydroxychinolin-Derivaten wie z.B. AlQ₃, die 8-Hydroxychinolin-Komplexe können auch Triarylaminophenol-Liganden enthalten (US 2007/0134514 A1), Metallkomplex-Polysilan-Verbindungen sowie Thiophen-, Benzothiophen- und Dibenzothiophen-Derivate.

Beispiele für bevorzugte Carbazol-Strukturelemente sind mCP (= 1,3-N,N-dicarbazol-benzol (= 9,9'-(1,3-Phenylen)bis-9H-carbazol)), CDBP (= 9,9'-(2,2'-Dimethyl[1,1'-biphenyl]-4,4'-diyl)bis-9H-carbazol), 1,3-Bis(N,N'-dicarbazol)benzol (= 1,3-Bis(carbazol-9-yl)benzol), PVK (Polyvinylcarbazol), 3,5-Di(9H-carbazol-9-yl)biphenyl sowie die unten abgebildeten Verbindungen. 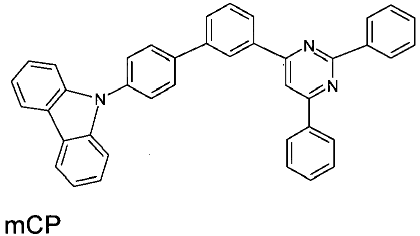 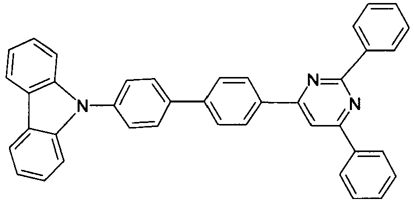
US 2005/0249976 A1 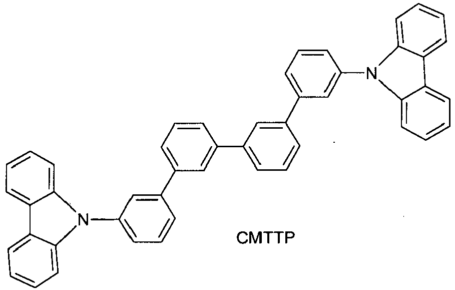 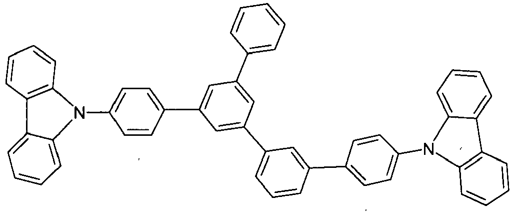
US 2007/0128467 A1 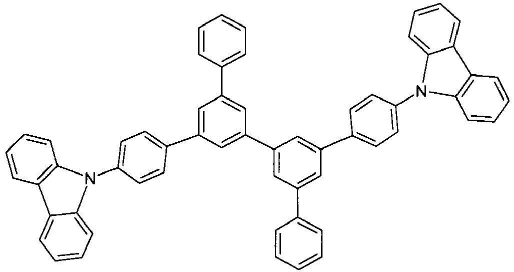
US 2007/0128467 A1

Bevorzugte Si-Tetraaryle sind z.B. (US 2004/0209115, US 2004/0209116)

| | |
|---|---|
| 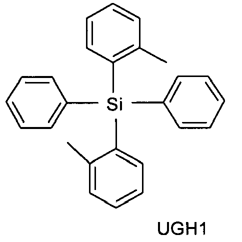 | 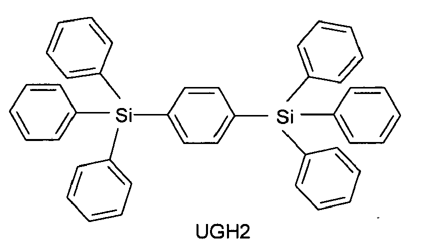 |
| 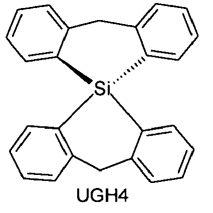 | 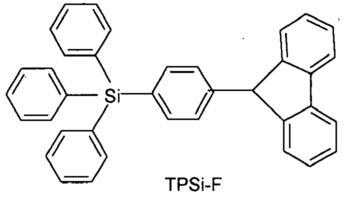 |
| | Triphenyl-[4-(9-phenyl-9H-fluoren-9-yl)phenyl]silane |
| 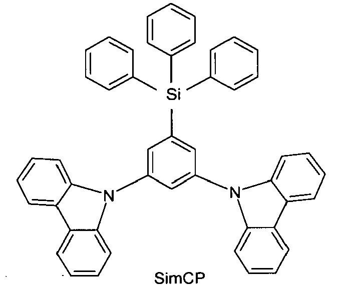 | |
| 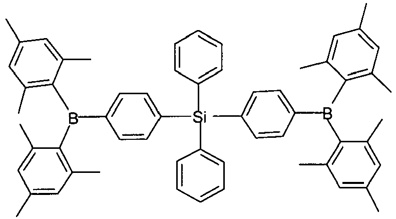 | |
| US 2007/0087219 A1 | |
| 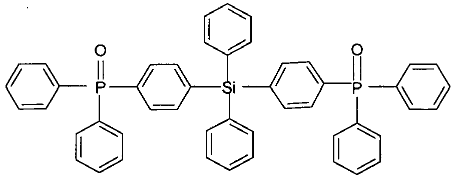 | |
| US 2007/0087219 A1 | |
| 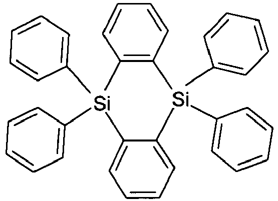 | |
| H. Gilman, E.A. Zuech, Chemistry & Industry (London, United Kingdom), 1960, 120. | |

Besonders bevorzugte Strukturelemente gemäß Gruppe 4 zur Herstellung der Matrix für phosphoreszierende Dotanden sind:

| | |
|---|---|
| 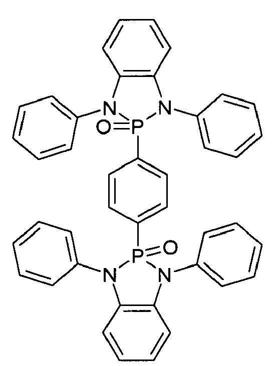 | 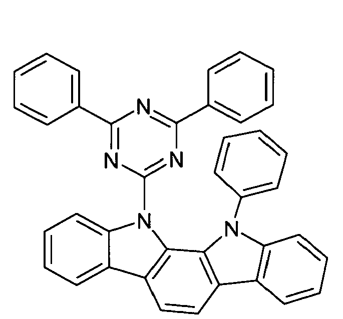 |
| DE 102009022858 | WO 07/063754 und WO 08/056746 |
| 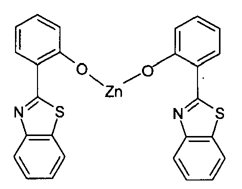 | 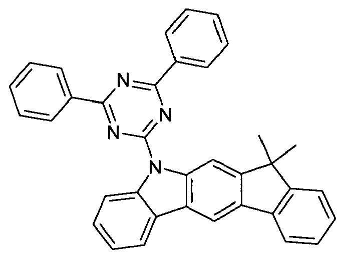 |
| EP 652273 B1 | DE 102009023155 |

Strukturelemente der Gruppe 5 sind solche, welche den Übergang vom Singulett- zum Triplettzustand verbessern und welche, unterstützend zu den funktionalen Strukturelementen der Gruppe 3 eingesetzt, die Phosphoreszenzeigenschaften dieser Strukturelemente verbessern. Hierfür kommen insbesondere Carbazol- und überbrückte Carbazoldimereinheiten in Frage, wie sie z.B. in der WO 04/070772 A2 und der WO 04/113468 A1 beschrieben werden. Weiterhin kommen hierfür Ketone, Phosphinoxide, Sulfoxide, Sulfone, Silan-Derivate und ähnliche Verbindungen in Frage, wie sie z.B. in der WO 05/040302 A1 beschrieben werden.

Die zuvor dargelegten funktionalen Strukturelemente A können vorzugsweise über eine aromatische und/oder heteroaromatische Gruppe mit mindestens einem löslichkeitsvermittelnden Strukturelement B verbunden sein. Die Bindungsstelle ist im Allgemeinen unkritisch, so dass eine oder mehrere Bindungen zu mindestens einem der nachfolgend beschriebenen löslichkeitsvermittelnden Strukturelemente B vorhanden ist, die jedoch aus Gründen der Übersichtlichkeit nicht in den zuvor dargelegten Formeln dargelegt sind. Gemäß einem besonderen Aspekt können die zuvor dargelegten funktionalen Strukturelemente A über ein Kohlenstoffatom eines aromatischen oder heteroaromatischen Ringsystems mit einem oder mehreren löslichkeitsvermittelnden Strukturelementen B verbunden sein.

Neben mindestens einem funktionalen Strukturelement A umfasst eine erfindungsgemäße Verbindung mindestens ein löslichkeitsvermittelndes Strukturelement B gemäß Formel (L-II) 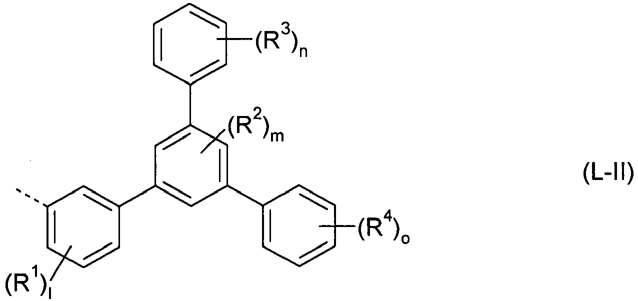 wobei
- R¹, R², R³, R⁴: jeweils unabhängig voneinander eine geradkettige Alkyl- oder Alkoxygruppe mit 1 bis 20 C-Atomen oder eine verzweigte Alkyl- oder Alkoxygruppe mit 3 bis 20 C-Atomen ist,
- l: 0, 1, 2, 3 oder 4 ist,
- m: 0, 1, 2 oder 3 ist und
- n, o: jeweils unabhängig voneinander 0, 1, 2, 3, 4 oder 5 ist;
wobei die gestrichelte Bindung die Bindung an das funktionale Strukturelement andeutet.

Zu den bevorzugten löslichkeitsvermittelnden Strukturelementen B gehören unter anderem die Strukturelemente der Formel (L-IIa): 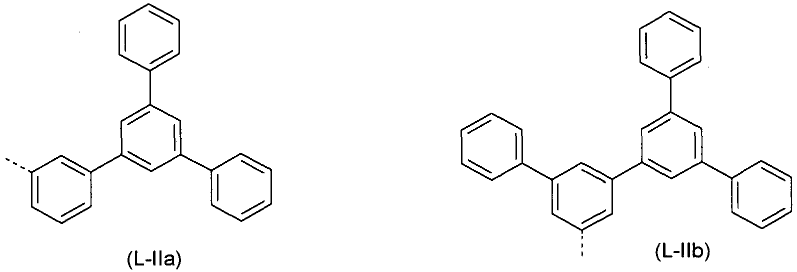 wobei die gestrichelte Bindung die Bindung an das funktionale Strukturelement A andeutet.

Besonders bevorzugt sind die löslichkeitsvermittelnden Strukturelemente B nach den Formeln (L-IIa) bis (L-IIa7): 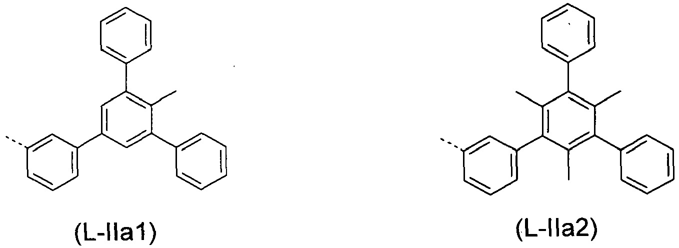 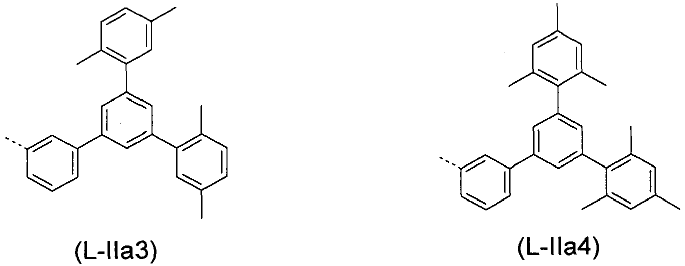 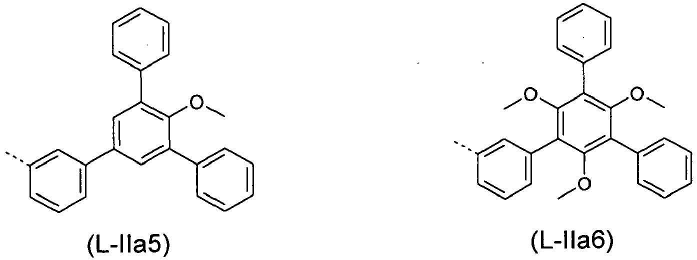 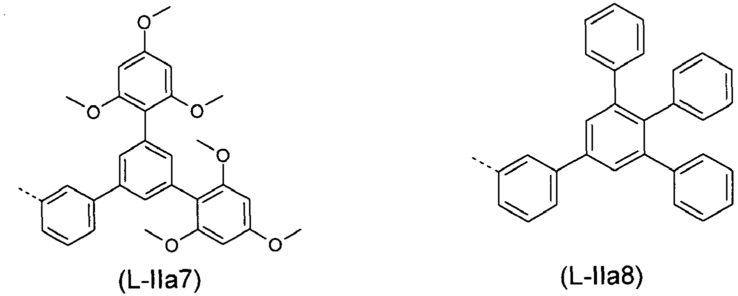 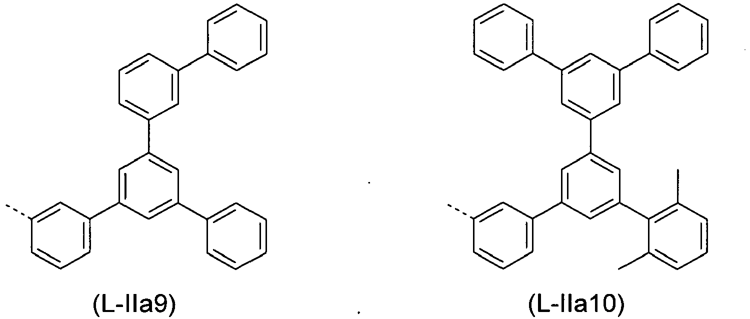 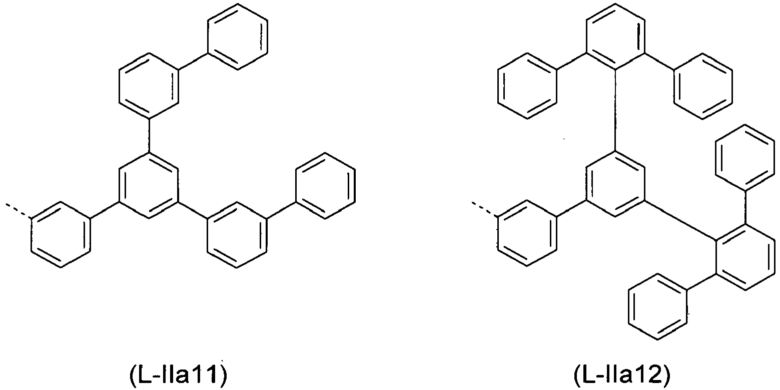 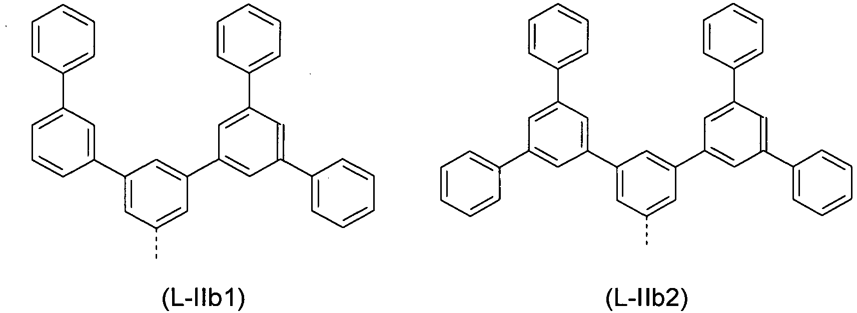 wobei die gestrichelte Bindung die Bindung an das funktionale A Strukturelement andeutet.

Als aromatisches oder heteroaromatisches Ringsystem mit 5 bis 60 Ringatomen, welches noch jeweils mit beliebigen Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin, Benzothiadiazol, Benzanthren, Benzanthracen, Rubicen und Triphenylen. Besonders bevorzugt im Sinne der vorliegenden Erfindung ist Fluoren, Spirobifluoren, Indenofluoren, Anthracen, Phenanthren, Dihydrophenanthren und Carbazol.

Eine Arylgruppe im Sinne der vorliegenden Offenbarung enthält 5 bis 60 C-Atome, eine Heteroarylgruppe im Sinne der vorliegenden Offenbarung enthält 2 bis 60 C-Atome und mindestens ein Heteroatom, mit der Maßgabe, dass die Summe aus C-Atomen und Heteroatomen mindestens 5 ergibt. Die Heteroatome sind vorzugsweise ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, oder eine kondensierte Aryl- oder Heteroarylgruppe, beispielsweise Naphthalin, Anthracen, Phenanthren, Chinolin, Isochinolin, Benzothiophen, Benzofuran und Indol, verstanden.

Im Rahmen der vorliegenden Erfindung werden unter einer Alkylgruppe mit 1 bis 20 C-Atomen, in der auch einzelne H-Atome oder CH₂-Gruppen durch die oben genannten Gruppen oder Reste R substituiert sein können, bevorzugt die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl und Octinyl verstanden. Unter einer Alkoxygruppe mit 1 bis 20 C-Atomen werden vorzugsweise Methoxy, Trifluormethoxy, Ethoxy, n-Propoxy, i-Propoxy, n-Butoxy, i-Butoxy, s-Butoxy, t-Butoxy und 2-Methylbutoxy verstanden.
Gemäß einem besonderen Aspekt der vorliegenden Erfindung liegt das Gewichtsverhältnis von Strukturelement A zu Strukturelement B in Formel (I) vorzugsweise im Bereich von 2:1 bis 1:20 und besonders bevorzugt im Bereich von 1:1 bis 1:3.
Die Herstellung der in den erfindungsgemäßen Formulierungen enthaltenen funktionalen Verbindungen gemäß Formel (I) kann unter Verwendung von bekannten Methoden erfolgen, wobei Edukte mit reaktiven Gruppen umgesetzt werden. Diese reaktiven Edukte enthalten die zuvor dargelegten Struktureinheiten der obigen Formeln sowie mindestens jeweils eine Abgangsgruppe, wie z.B. Brom, Iod, Boronsäure oder Boronsäureester.
Geeignete Reaktionen zur Bildung von C-C-Verknüpfungen sind dem Fachmann bekannt und in der Literatur beschrieben. Besonders geeignete und bevorzugte Kupplungsreaktionen, die alle zu C-C-Verknüpfungen führen, sind solche gemäß SUZUKI, YAMAMOTO, STILLE, HECK, NEGISHI, SONOGASHIRA und HIYAMA.

Zu den besonders bevorzugten funktionalen Verbindungen zählen Verbindungen der Formeln (V-Ia), (V-Ib), (V-Ic) und (V-Id) 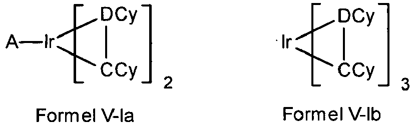 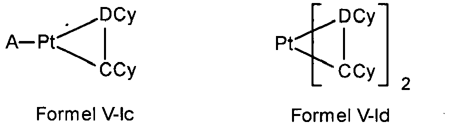 wobei für die verwendeten Symbole gilt:
- DCy: ist gleich oder verschieden bei jedem Auftreten eine cyclische Gruppe, die mindestens ein Donoratom, vorzugsweise Stickstoff, Kohlenstoff in Form eines Carbens oder Phosphor, enthält, über welches die cyclische Gruppe an das Metall gebunden ist, und die wiederum einen oder mehrere Substituenten R¹⁰ tragen kann; die Gruppen DCy und CCy sind über eine kovalente Bindung miteinander verbunden;
- CCy: ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹⁰ tragen kann;
- A: ist gleich oder verschieden bei jedem Auftreten ein mono-anionischer, zweizähnig chelatisierender Ligand, vorzugsweise ein Diketonatligand;
- R¹⁰: ist bei jedem Auftreten gleich oder verschieden H, D, F, Cl, Br, I, CHO, C(=O)Ar³, P(=O)(Ar³)₂, S(=O)Ar³, S(=O)₂Ar³, CR¹¹=CR¹¹Ar³, CN, NO₂, Si(R¹¹)₃, B(OR¹¹)₂, B(R¹¹)₂, B(N(R¹¹)₂)₂, OSO₂R¹¹, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R¹¹ substituiert sein kann, wobei eine oder mehrere nicht benachbarte CH₂-Gruppen durch R¹¹C=CR¹¹, C≡C, Si(R¹¹)₂, Ge(R¹¹)₂, Sn(R¹¹)₂, C=O, C=S, C=Se, C=NR¹¹, P(=O)(R¹¹), SO, SO₂, NR¹¹, O, S oder CONR¹¹ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R¹¹ substituiert sein kann, oder eine Aryloxy- oder Hetero-aryloxygruppe mit 5 bis 60 aromatischen Ringatomen, die durch einen oder mehrere Reste R¹¹ substituiert sein kann, oder eine Kombination dieser Systeme; dabei können zwei oder mehrere benachbarte Substituenten R¹⁰ auch miteinander ein mono- oder polycyclisches, aliphatisches oder aromatisches Ringsystem bilden;
- Ar³: ist bei jedem Auftreten gleich oder verschieden ein aromatisches oder heteroaromatisches Ringsystem mit 5 bis 40 aromatischen Ringatomen, das mit einem oder mehreren Resten R¹¹ substituiert sein kann;
- R¹¹: ist bei jedem Auftreten gleich oder verschieden H, D, CN oder ein aliphatischer, aromatischer und/oder heteroaromatischer Kohlen-wasserstoffrest mit 1 bis 20 C-Atomen, in dem auch H-Atome durch F ersetzt sein können; dabei können zwei oder mehrere benachbarte Substituenten R¹¹ auch miteinander ein mono- oder polycyclisches, aliphatisches oder aromatisches Ringsystem bilden,
wobei mindestens einer der genannten Reste DCy, CCy und/oder A mindestens eine Gruppe der Formel (L-II) umfasst.

Dabei kann durch Bildung von Ringsystemen zwischen mehreren Resten R¹⁰ auch eine Brücke zwischen den Gruppen DCy und CCy vorliegen. Weiterhin kann durch Bildung von Ringsystemen zwischen mehreren Resten R¹⁰ auch eine Brücke zwischen zwei oder drei Liganden CCy-DCy bzw. zwischen ein oder zwei Liganden CCy-DCy und dem Liganden A vorliegen, so dass es sich um ein polydentates bzw. polypodales Ligandensystem handelt.

Gemäß einer weiteren, besonderen Ausgestaltung der vorliegenden Erfindung werden lösliche funktionale Verbindungen, insbesondere Polyacene gemäß der Formel (V-II) bereitgestellt 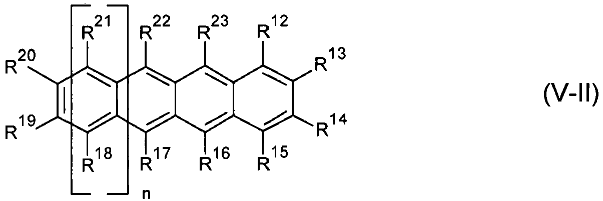 worin
die Reste R¹², R¹³, R¹⁴, R¹⁵, R¹⁶, R¹⁷, R¹⁸, R¹⁹, R²⁰, R²¹, R²² und R²³ jeweils unabhängig Wasserstoff, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen ist oder eine Silylgruppe oder eine substituierte Ketogruppe mit 1 bis 40 C-Atomen, eine Alkoxycarbonylgruppe mit 2 bis 40 C-Atomen, eine Aryloxycarbonylgruppe mit 7 bis 40 C-Atomen, eine Cyanogruppe (-CN), eine Carbamoylgruppe (-C(=O)NH₂), eine Haloformylgruppe (-C(=O)-X, worin X ein Halogenatom darstellt), eine Formylgruppe (-C(=O)-H), eine Isocyanogruppe, eine Isocyanatgruppe, eine Thiocyanatgruppe oder eine Thioisocyanatgruppe, eine Aminogruppe, eine Hydroxygruppe, eine Nitrogruppe, eine CF₃-Gruppe, Cl, Br, F, eine vernetzbare Gruppe oder ein substituiertes oder unsubstituiertes, aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme ist, wobei eine oder mehrere der Gruppen R¹², R¹³, R¹⁴, R¹⁵, R¹⁶, R¹⁷, R¹⁸, R¹⁹, R²⁰, R²¹, R²² und/oder R²³ ein mono- oder polycyclisches, aliphatisches oder aromatisches Ringsystem miteinander und/oder mit dem Ring, an dem die jeweilige Gruppe gebunden ist, bilden können; und worin jedes Paar R¹² und R¹³, R¹³ und R¹⁴, R¹⁴ und R¹⁵, R¹⁸ und R¹⁹, R¹⁹ und R²⁰, R²⁰ und R²¹ unabhängig optional gesättigten oder ungesättigten C₄-C₄₀ Ring bilden kann, der durch ein oder mehrere Sauerstoff- und/oder Schwefelatome oder eine Gruppe der Formel -N(R^{a})- unterbrochen sein kann, worin R^{a} ein Wasserstoffatom oder eine optional substituierte Kohlenwasserstoffgruppe darstellt, wobei der Ring gegebenenfalls substituiert sein kann; und
worin eine oder mehrere Kohlenstoffatome des Polyacengrundgerüsts optional durch ein oder mehrere Heteroatome ausgewählt aus N, P, As, O, S, Se and Te substituiert sein können; und wobei ein oder mehrere der Substituenten R¹² bis R²³, die an benachbarten Ringpositionen des Polyacens angeordnet sind zusammen einen weiteren gesättigten oder ungesättigten Ring bilden können, der durch ein oder mehrere Sauerstoff- und/oder Schwefelatome oder eine Gruppe der Formel -N(R^{a})-unterbrochen sein kann, worin R^{a} ein Wasserstoffatom oder eine optional substituierte Kohlenwasserstoffgruppe darstellt, oder ein aromatisches Ringsystem, welches an das Polyacen gebunden ist; und
worin n 0, 1, 2, 3 oder 4, vorzugsweise 0, 1 or 2, besonders bevorzugt 0 oder 2 ist;
wobei mindestens einer der Reste R¹², R¹³, R¹⁴, R¹⁵, R¹⁶, R¹⁷, R¹⁸, R¹⁹, R²⁰, R²¹, R²² und/oder R²³ mindestens eine Gruppe der Formel (L-II) umfasst.

Falls n in Formel (V-II) gleich 2 ist, stellt diese Verbindung eine Pentacen-Verbindung dar. Für n = 0 kann die Verbindung eine "Pseudopentacene-Verbindung " darstellen.

Gemäß einer weiteren Ausführungsform stellt die vorliegende Erfindung funktionale Verbindungen der allgemeinen Formel (V-IIIa) und/oder (V-IIIb) bereit 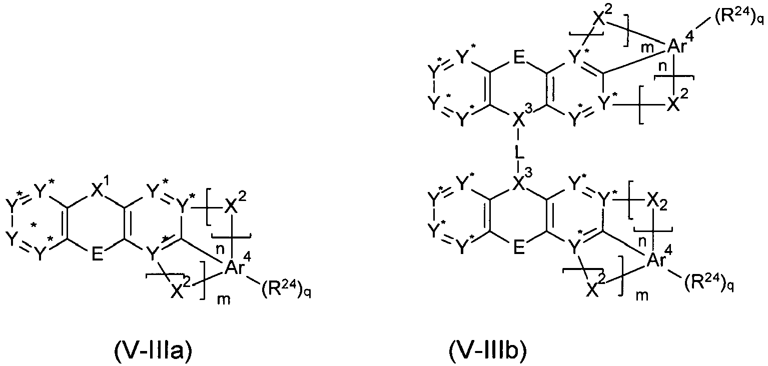 wobei für die Symbole und Indices gilt:
- Y*: ist C, wenn an die Gruppe Y eine Gruppe X² gebunden ist, oder ist bei jedem Auftreten gleich oder verschieden CR oder N, wenn an die Gruppe Y keine Gruppe X² gebunden ist;
- E: ist bei jedem Auftreten gleich oder verschieden eine kovalente Einfachbindung oder eine bivalente Brücke, ausgewählt aus N(R²⁵), B(R²⁵), C(R²⁵)₂, O, Si(R²⁵)₂, C=NR²⁵, C=C(R²⁵)₂, S, S=O, SO₂, P(R²⁵) und P(=O)R¹;
- X¹: ist bei jedem Auftreten gleich oder verschieden eine bivalente Brücke, ausgewählt aus N(R²⁵), B(R²⁵), O, C(R²⁵)₂, Si(R²⁵)₂, C=NR²⁵, C=C(R²⁵)₂, S, S=O, SO₂, P(R²⁵) und P(=O)R²⁵;
- X²: ist bei jedem Auftreten gleich oder verschieden eine bivalente Brücke, ausgewählt aus N(R²⁵), B(R²⁵), C(R²⁵)₂, Si(R²⁵)₂, C=O, C=NR²⁵, C=C(R²⁵)₂, S, S=O, SO₂, CR²⁵-CR²⁵, P(R²⁵) und P(=O)R²⁵;
- X³: ist bei jedem Auftreten gleich oder verschieden eine bivalente Brücke, ausgewählt aus N, B, C(R¹), Si(R¹), P und P(=O);
- L: ist ein bivalentes, aromatisches oder heteroaromatisches Ring-system mit 5 bis 40 aromatischen Ringatomen, welches durch einen oder mehrere Reste R¹ substituiert sein kann;
- n, m: sind 0 oder 1, mit der Maßgabe, dass n+m = 1 oder 2 ist;
- q: ist 1, 2, 3, 4, 5 oder 6;
- R²⁴: ist bei jedem Auftreten gleich oder verschieden H, D, F, Cl, Br, I, N(Ar)₂, C(=O)Ar, P(=O)Ar₂, S(=O)Ar, S(=O)₂Ar, CR²⁶=CR²⁶Ar, CN, NO₂, Si(R²⁶)₃, B(OR²⁶)₂, OSO₂R²⁶, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R²⁶ substituiert sein kann, wobei eine oder mehrere nicht benachbarte CH2-Gruppen durch R²⁶C=CR²⁶, C≡C, Si(R²⁶)₂, Ge(R²⁶)₂, Sn(R²⁶)₂, C=O, C=S, C=Se, C=NR²⁶, P(=O)(R²⁶), SO, SO₂, NR²⁶, O, S oder CONR²⁶ ersetzt sein können und wobei ein oder mehrere H-Atome durch D, F, Cl, Br, I, CN oder NO₂ ersetzt sein können, eine vernetzbare Gruppe oder eine aromatische oder hetero-aromatische Gruppe mit 5 bis 40 Ringatomen, die jeweils durch einen oder mehrere Reste R²⁶ substituiert sein kann, oder ein aromatisches oder heteroaromatisches Ringsystem mit 5 bis 40 aromatischen Ringatomen, das jeweils durch einen oder mehrere Reste R²⁶ substituiert sein kann, oder eine Aryloxy- oder Heteroaryloxygruppe mit 5 bis 40 aromatischen Ringatomen, die durch einen oder mehrere Reste R²⁶ substituiert sein kann, oder eine Kombination dieser Systeme; dabei können zwei oder mehrere Substituenten R²⁴ auch miteinander, zusammen mit den Atomen, an die sie gebunden sind oder, wenn sie an Ar gebunden sind, mit Ar ein mono- oder polycyclisches, aliphatisches, aromatisches oder heteroaromatisches Ringsystem bilden;
- R²⁵: ist bei jedem Auftreten gleich oder verschieden H, D, F, Cl, Br, I, CN, NO₂, CF₃, B(OR²⁶)₂, Si(R²⁶)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R²⁶ substituiert sein kann, wobei eine oder mehrere nicht benachbarte CH2-Gruppen durch -R²⁶C=CR²⁶-, -C≡C-, Si(R²⁶)₂, Ge(R²⁶)₂, Sn(R²⁶)₂, C=O, C=S, C=Se, C=N R²⁶, -O-, -S-, -COO- oder -CON R²⁶- ersetzt sein können und wobei ein oder mehrere H-Atome durch D, F, Cl, Br, I, CN oder NO₂ ersetzt sein können, oder Arylamine, oder substituierte oder unsubstituierte Carbazole, die jeweils mit einem oder mehreren Resten R²⁶ substituiert sein können, oder eine Aryl- oder Heteroarylgruppe mit 5 bis 40 Ring-atomen, die durch einen oder mehrere aromatische oder heteroaromatische oder durch nicht-aromatische Reste R²⁷ substituiert sein kann oder ein aromatisches oder hetero-aromatisches Ringsystem mit 5 bis 40 aromatischen Ringatomen, das durch einen oder mehrere nicht-aromatische Reste R²⁶ substituiert sein kann, oder eine Aryloxy- oder Heteroaryloxygruppe mit 5 bis 40 aromatischen Ringatomen, die durch einen oder mehrere nicht-aromatische Reste R²⁷ substituiert sein kann, oder eine Kombination dieser Systeme; dabei können zwei oder mehrere Substituenten R²⁵, zusammen mit den Atomen, an die sie gebunden sind, auch miteinander ein mono- oder polycyclisches, aliphatisches, aromatisches oder heteroaromatisches Ringsystem bilden;
- R²⁶: ist bei jedem Auftreten gleich oder verschieden H, D oder ein aliphatischer oder aromatischer Kohlenwasserstoffrest mit 1 bis 20 C-Atomen;
- Ar⁴: ist bei jedem Auftreten gleich oder verschieden ein aromatisches oder heteroaromatisches Ringsystem mit 5 bis 40 Ringatomen, das mit einem oder mehreren Resten R²⁵ substituiert sein kann;
wobei mindestens einer der zuvor dargelegten Reste eine Gruppe der Formel (L-II) umfasst.

Darüber hinaus sind lösliche funktionale Verbindungen der Formel (V-V) bevorzugt, 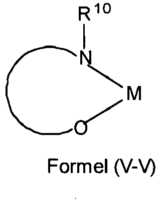 wobei R¹⁰ dieselbe Bedeutung hat, wie zuvor für die Formeln (V-Ia), (V-Ib), (V-Ic) und (V-Id) beschrieben, die gebogene Linie zwei oder drei Atome und Bindungen darstellt, welche erforderlich sind, um mit M einen 5- oder 6-Ring zu ergänzen, wobei diese Atome auch durch einen oder mehrere Reste R¹⁰ substituiert sein können, wobei mindestens einer der zuvor dargelegten Reste eine Gruppe der Formel (L-II) umfasst, und M ein Alkalimetall, ausgewählt aus Lithium, Natrium, Kalium, Rubidium und Caesium, darstellt.

Dabei ist es möglich, dass der Komplex der Formel (V-V) in monomerer Form vorliegt, wie oben abgebildet, oder dass er in Form von Aggregaten vorliegt, beispielsweise aus zwei Alkalimetallionen und zwei Liganden, vier Alkalimetallionen und vier Liganden, sechs Alkalimetallionen und sechs Liganden oder in Form anderer Aggregate.

Bevorzugte Verbindungen der Formel (V-V) sind die Verbindungen der folgenden Formeln (V-V1) und (V-V2), 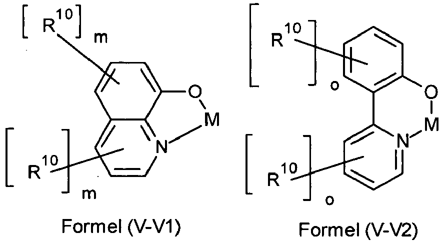 wobei die verwendeten Symbole dieselbe Bedeutung haben, wie zuvor für die Formeln (V-Ia), (V-Ib), (V-Ic) und (V-Id) und oben für die Formel (V-V) beschrieben, und m gleich oder verschieden bei jedem Auftreten für 0, 1, 2 oder 3 steht und o gleich oder verschieden bei jedem Auftreten für 0, 1, 2, 3 oder 4 steht.

Weitere bevorzugte organische Alkalimetallverbindungen sind die Verbindungen der folgenden Formel (V-V3), 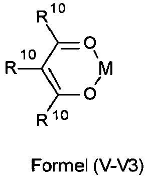 wobei die verwendeten Symbole dieselbe Bedeutung haben, wie zuvor für die Formeln (V-Ia), (V-Ib), (V-Ic) und (V-Id) und oben für die Formel (V-V) beschrieben, wobei mindestens einer der zuvor dargelegten Reste eine Gruppe der Formel (L-II) umfasst.

Bevorzugt ist das Alkalimetall ausgewählt aus Lithium, Natrium und Kalium, besonders bevorzugt aus Lithium und Natrium und ist ganz besonders bevorzugt Lithium.

Besonders bevorzugt ist eine Verbindung der Formel (V-V1), insbesondere mit M = Lithium. Ganz besonders bevorzugt sind weiterhin die Indices m = 0. Ganz besonders bevorzugt handelt es sich also um unsubstituiertes Lithiumchinolinat.

Zu den funktionalen Verbindungen der Formel (I) zählen unter anderem (wobei nicht alle diese Verbindungen durch Formel (L-II) beschrieben werden)

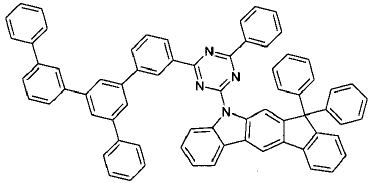

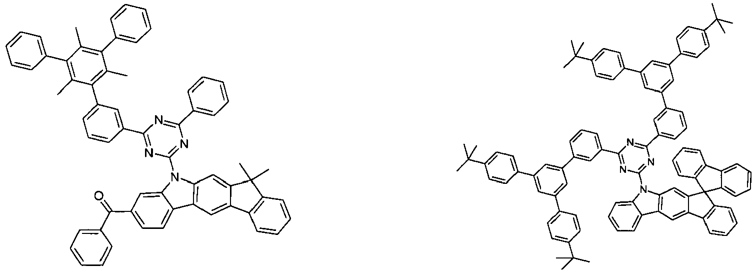

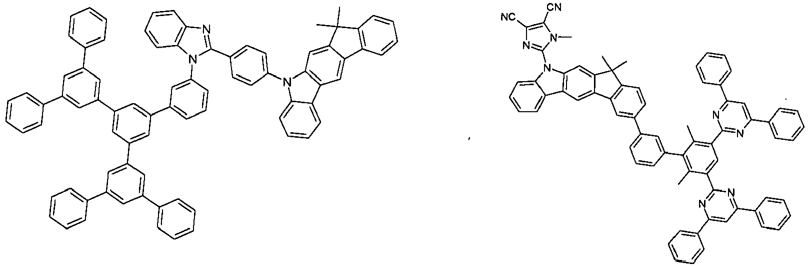

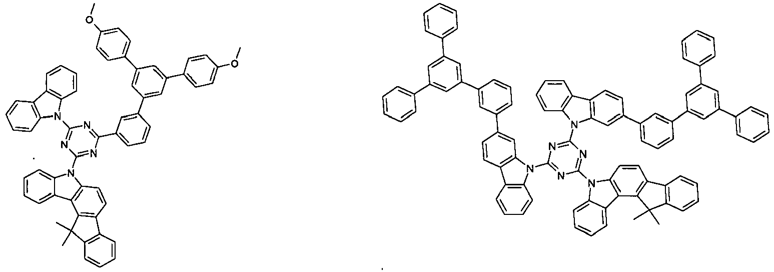

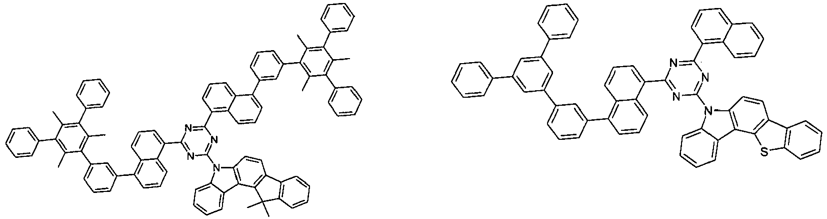

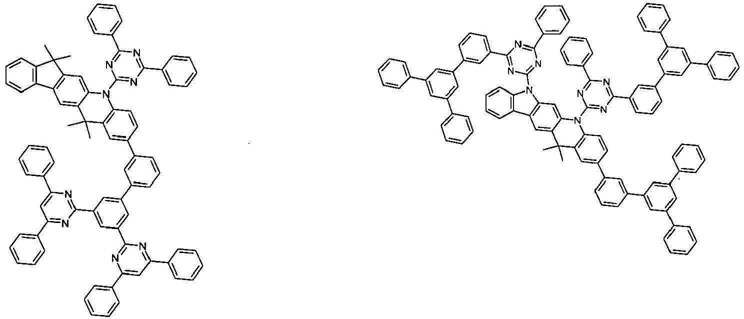

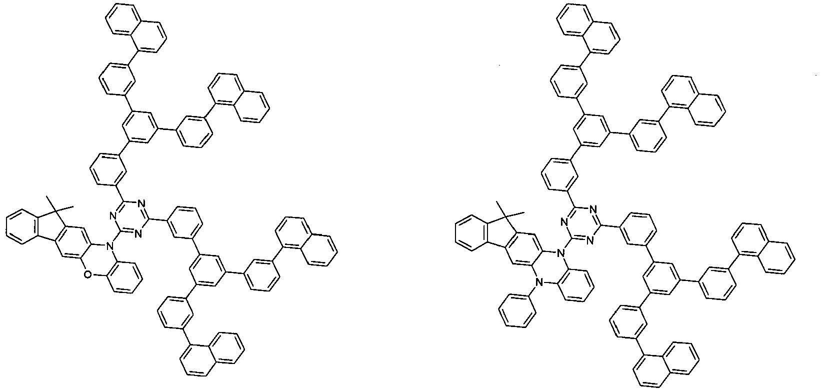

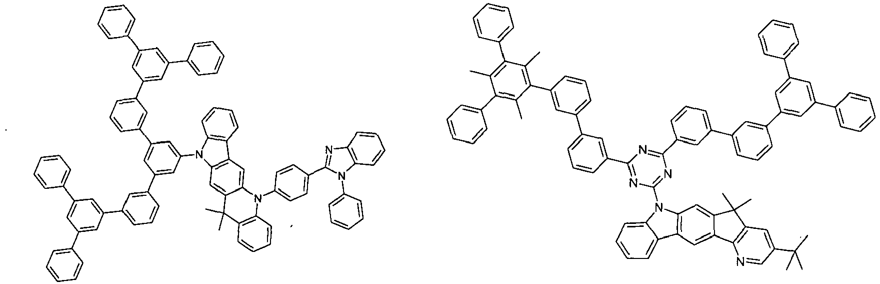

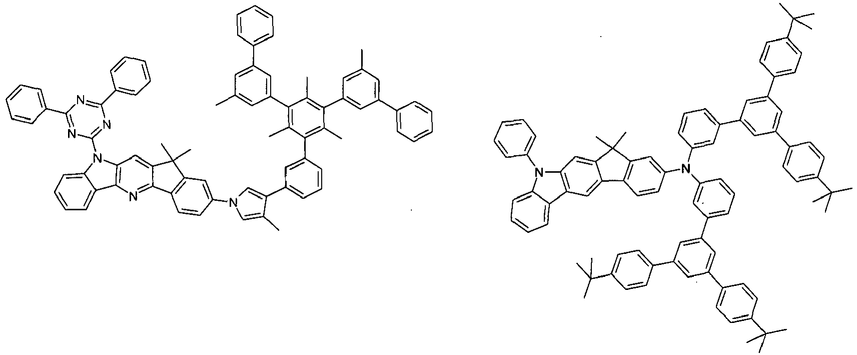

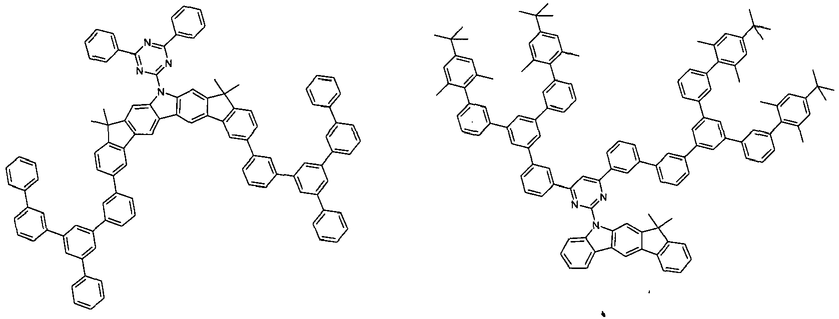

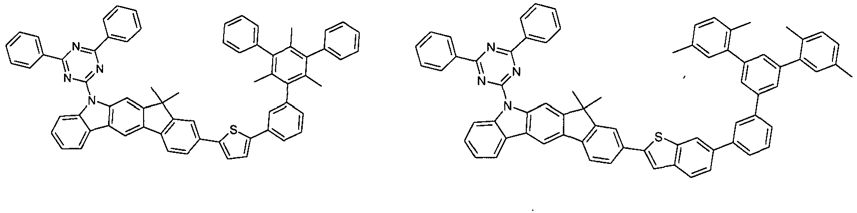

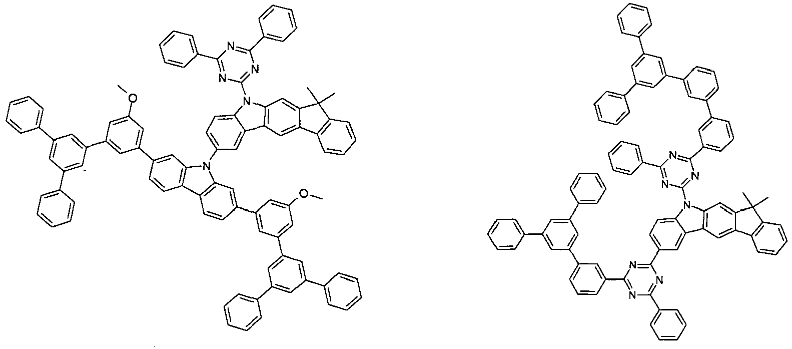

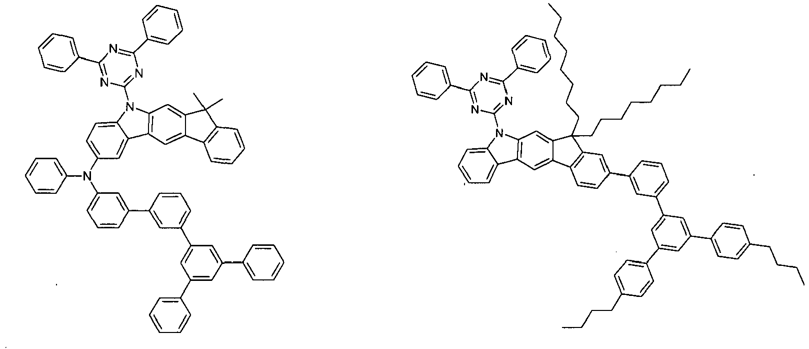

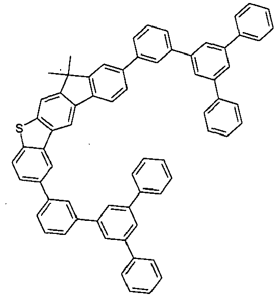

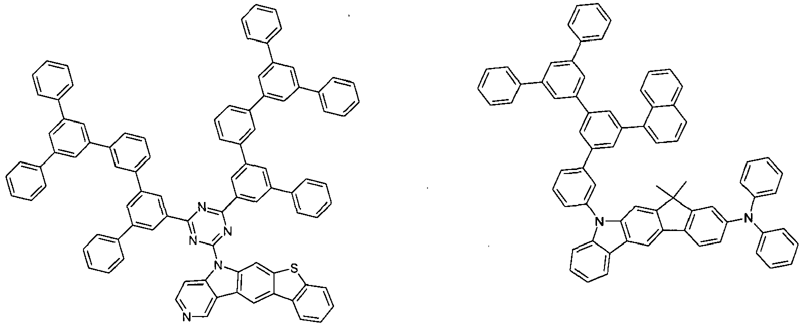

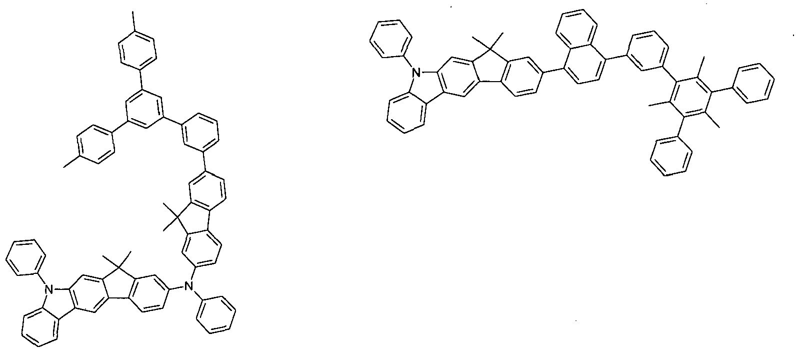

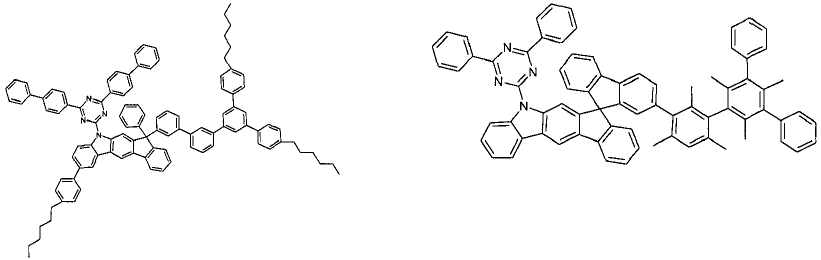

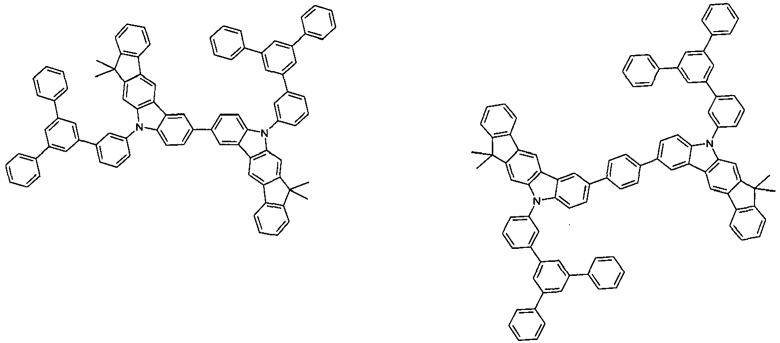

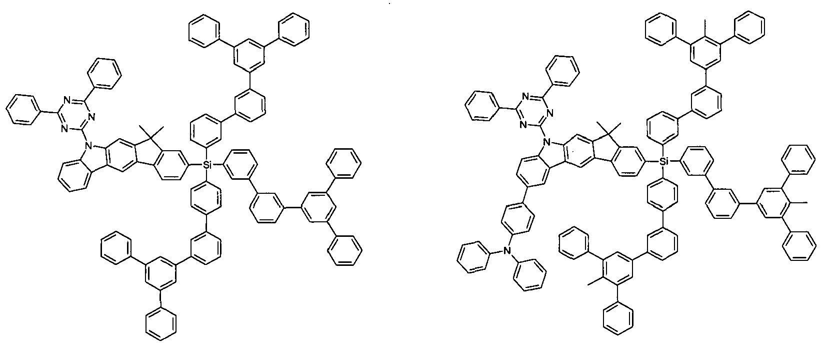

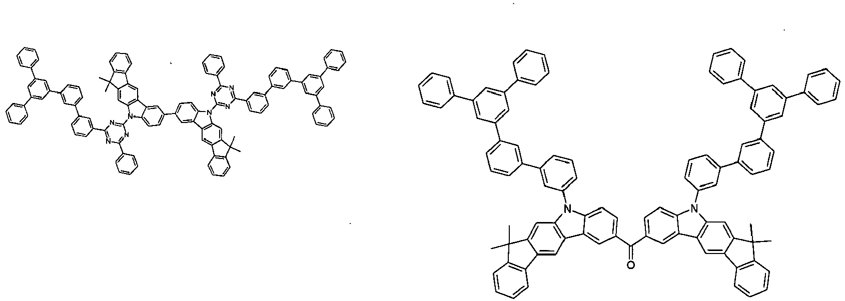

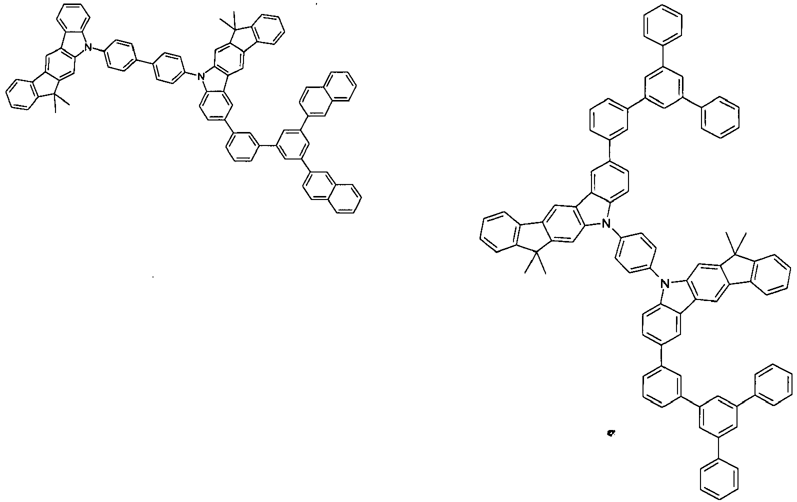

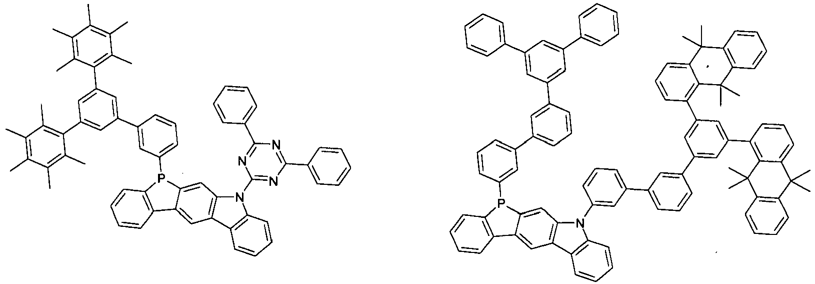

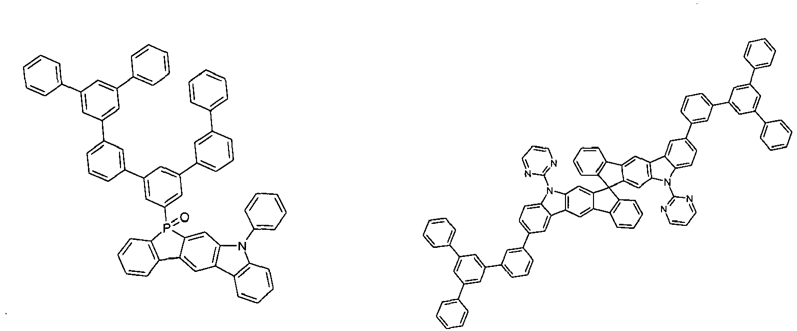

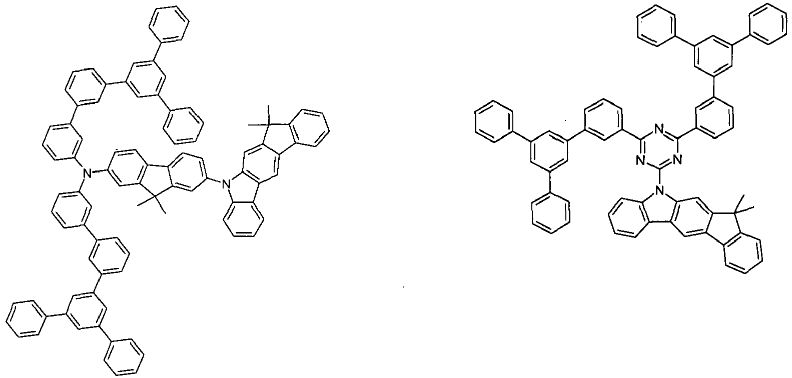

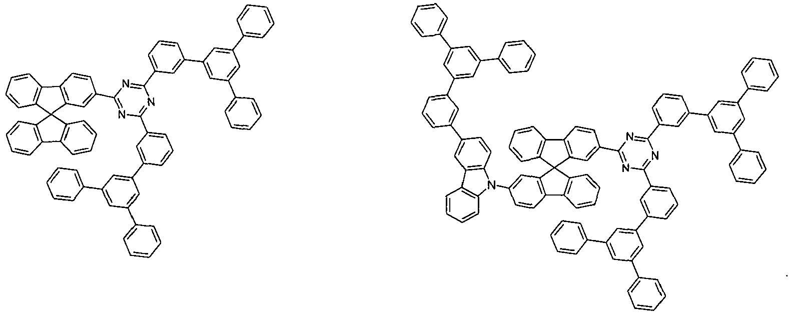

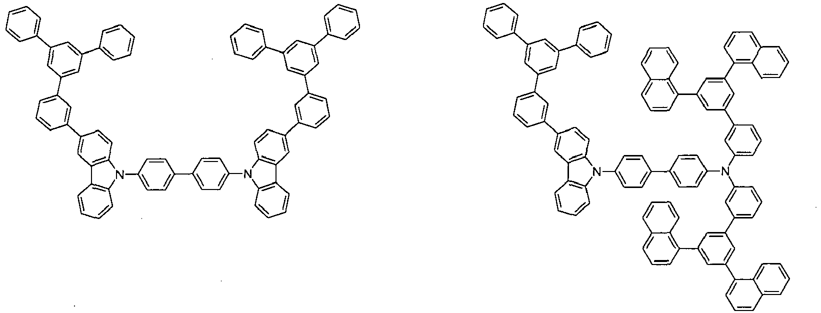

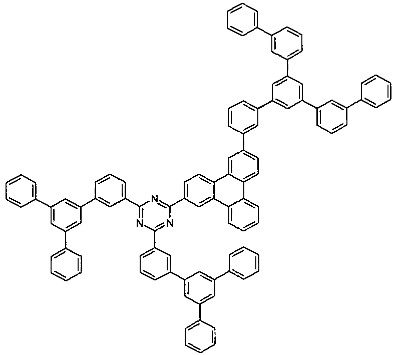

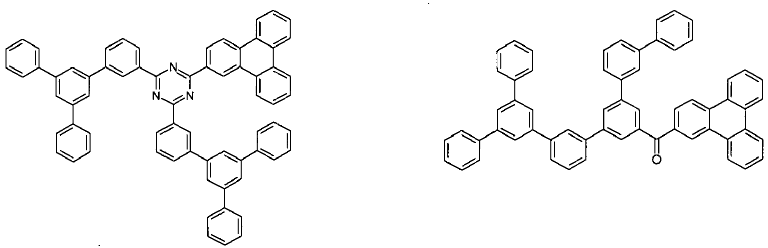

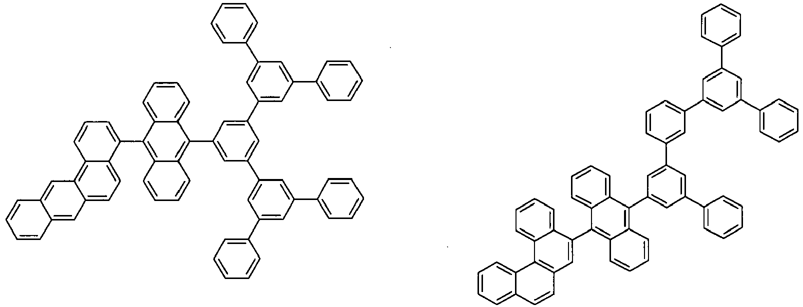

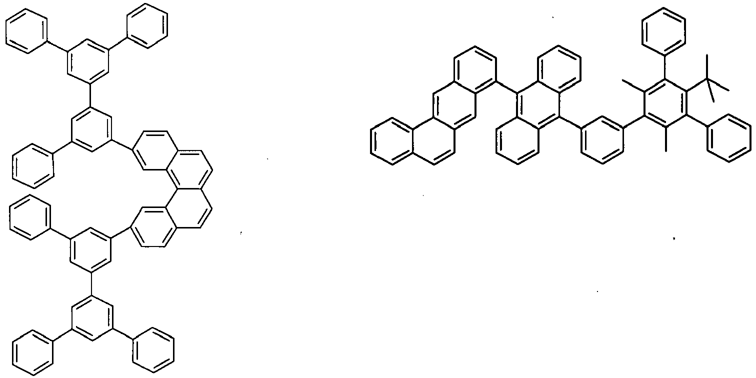

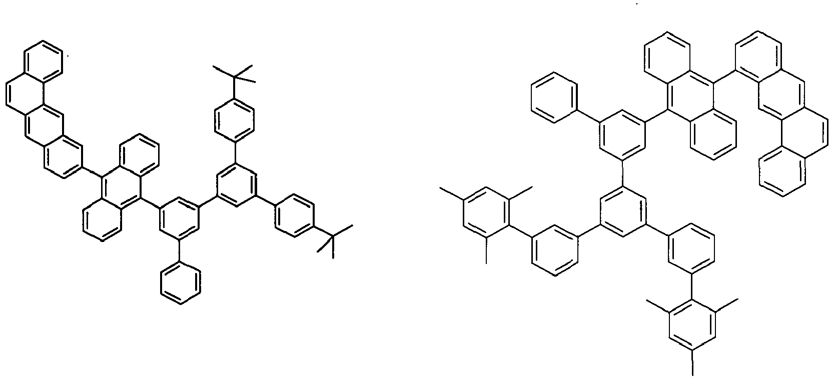

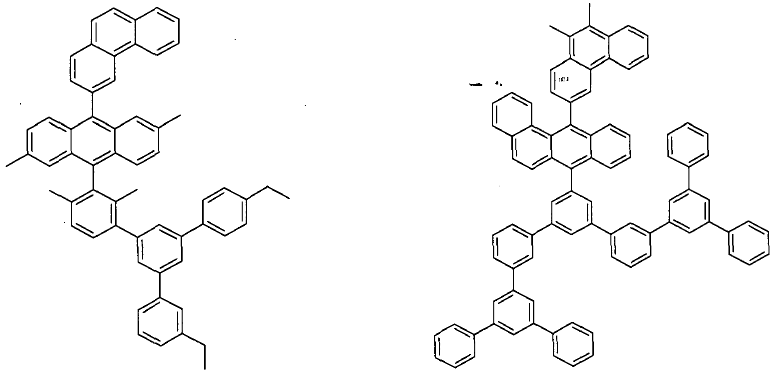

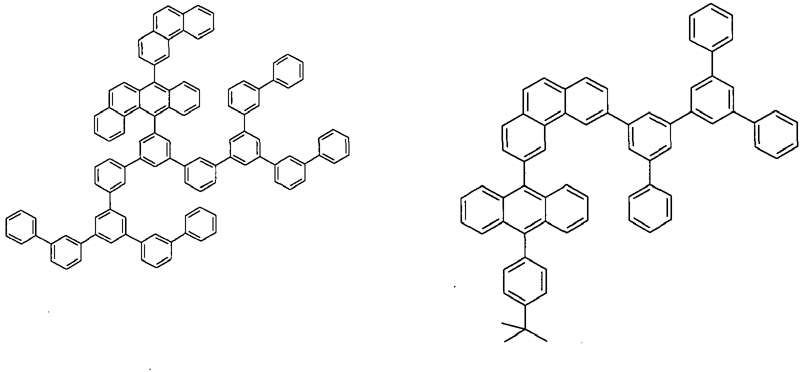

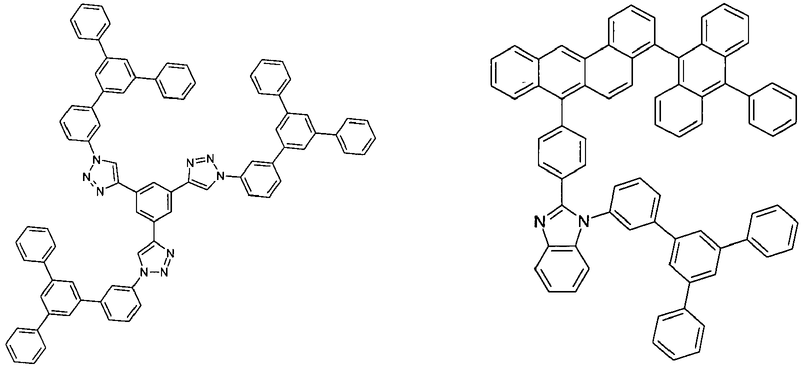

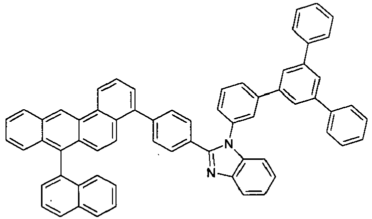

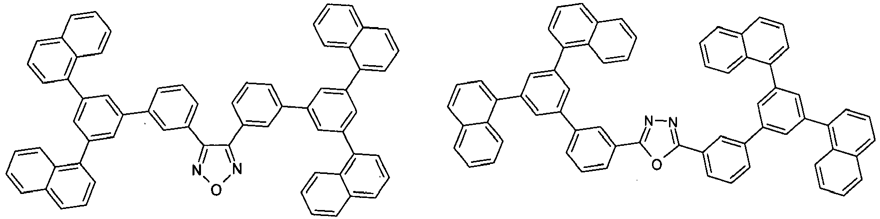

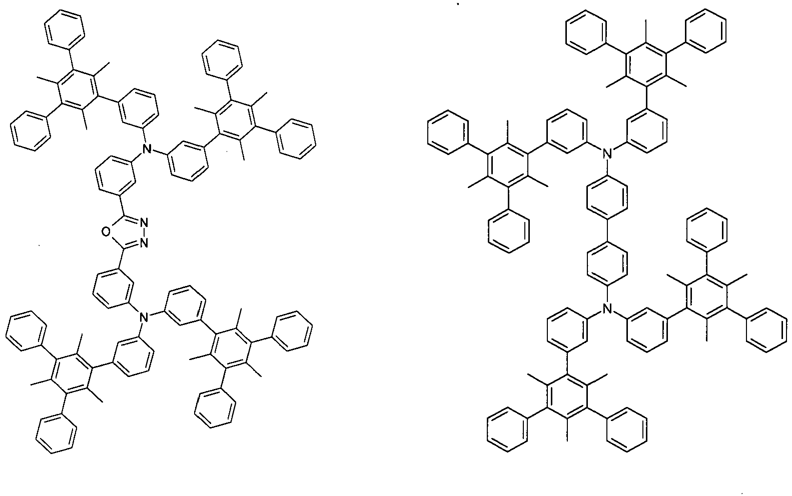

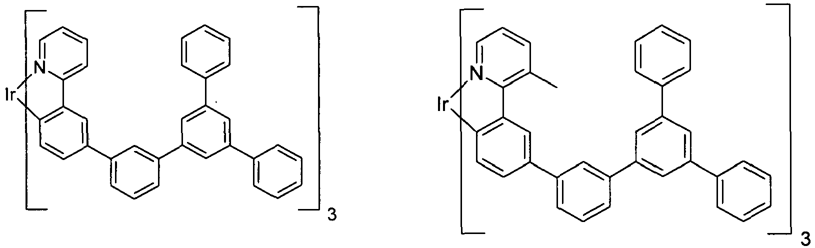

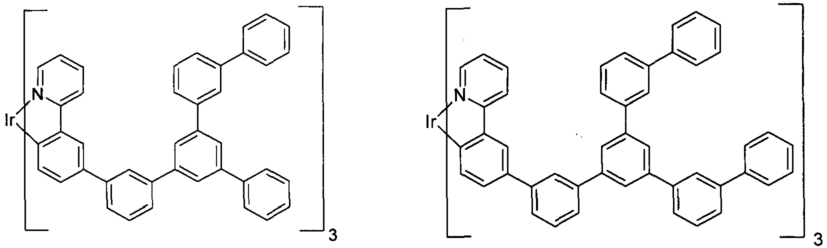

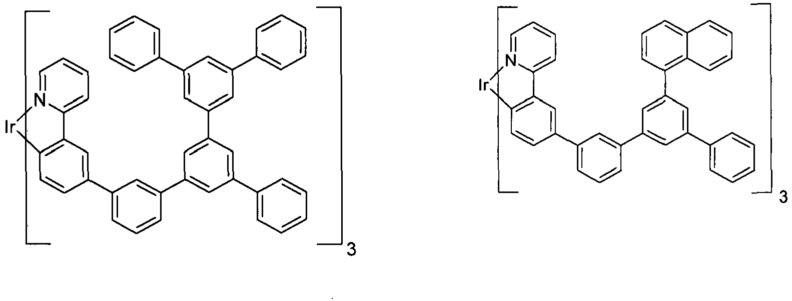

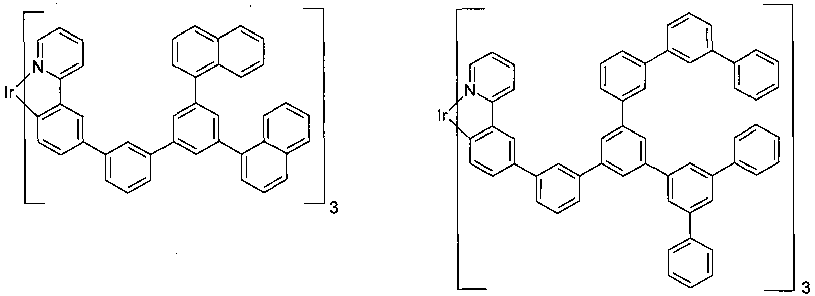

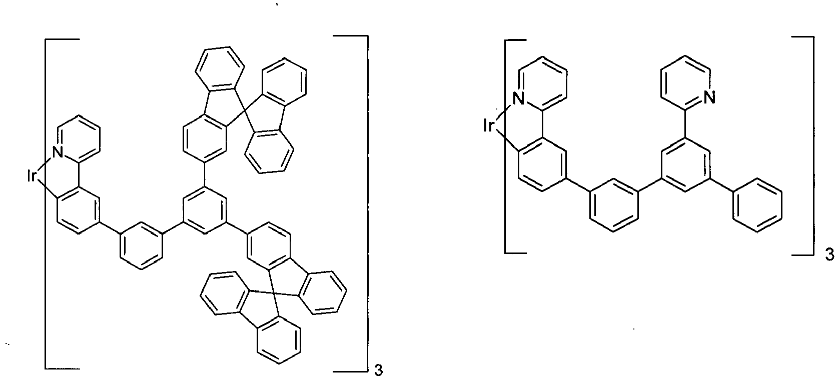

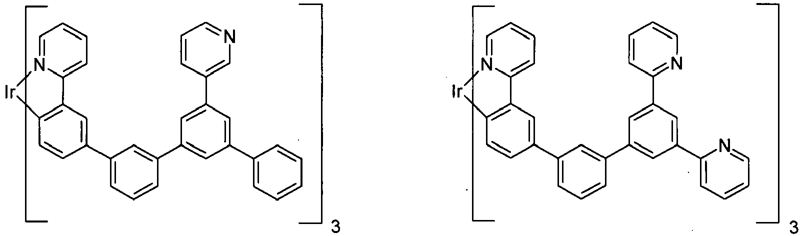

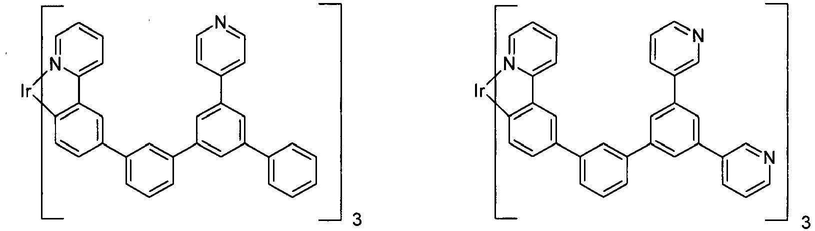

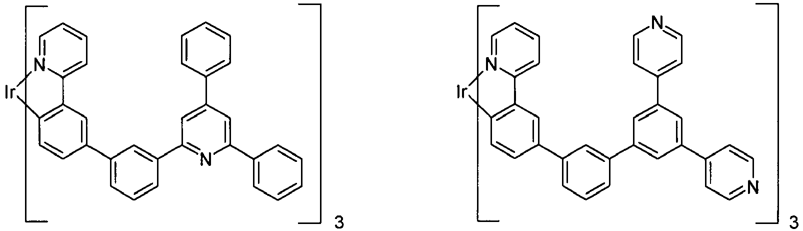

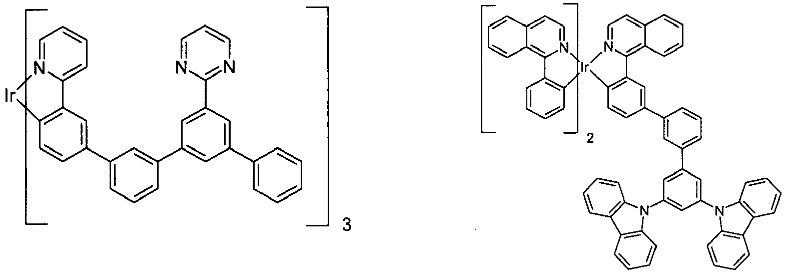

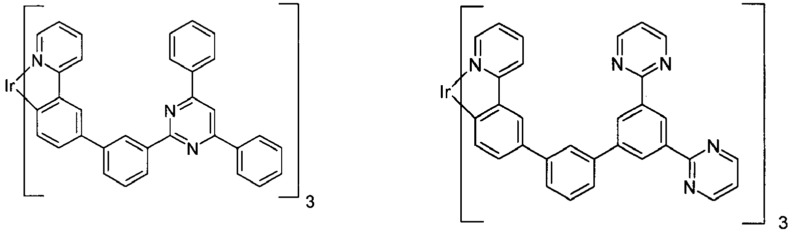

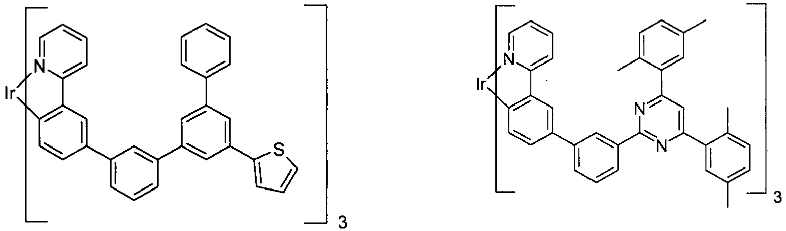

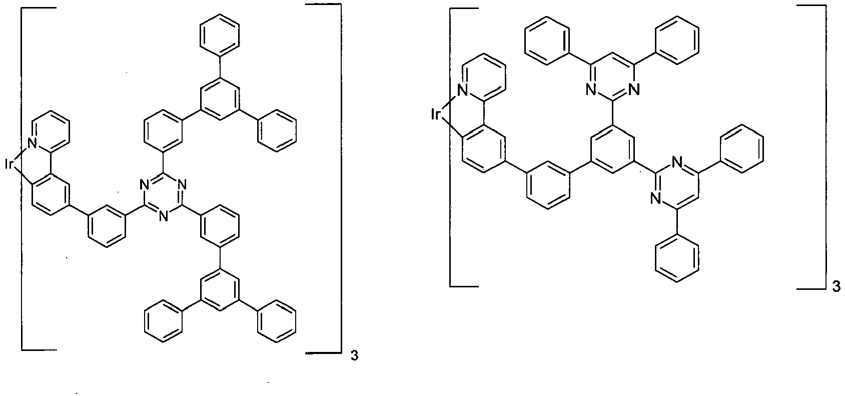

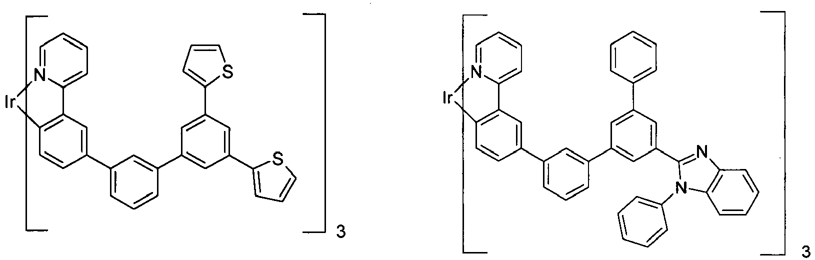

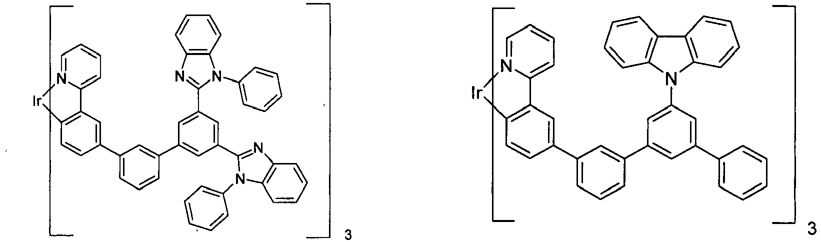

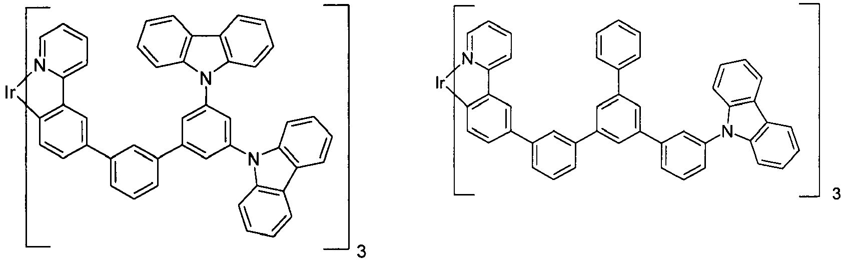

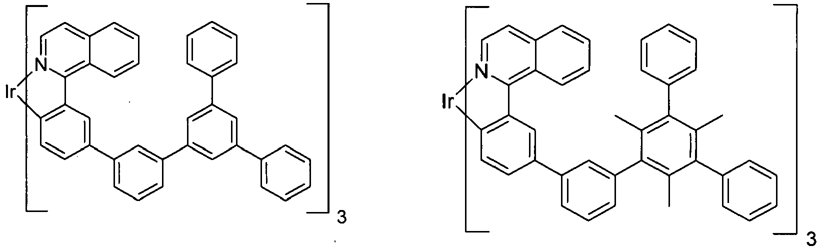

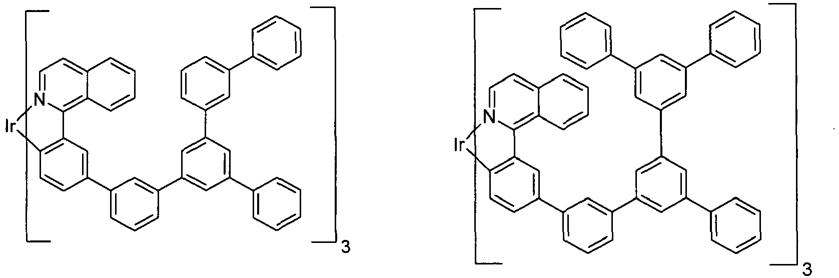

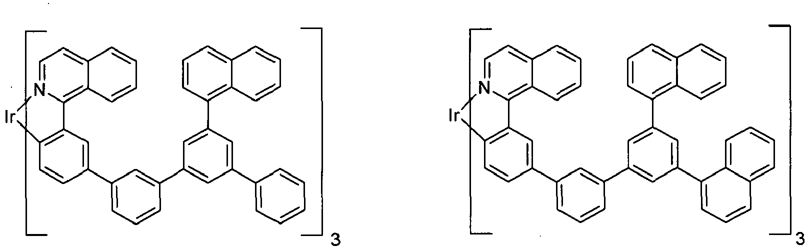

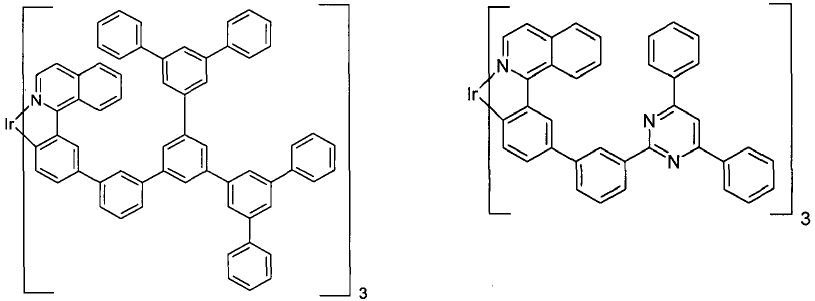

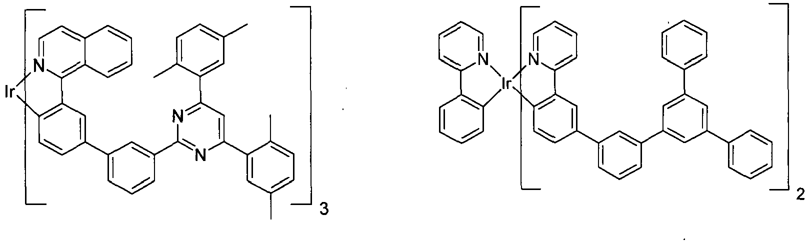

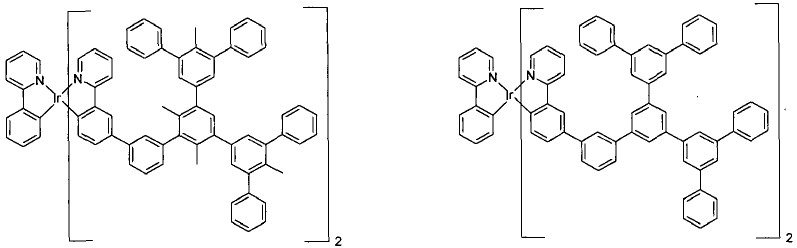

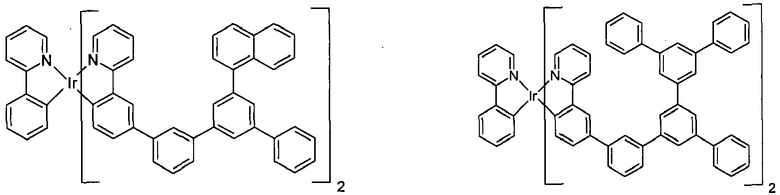

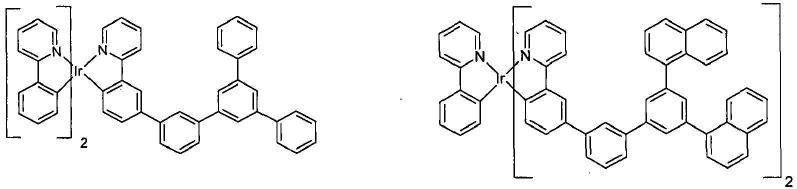

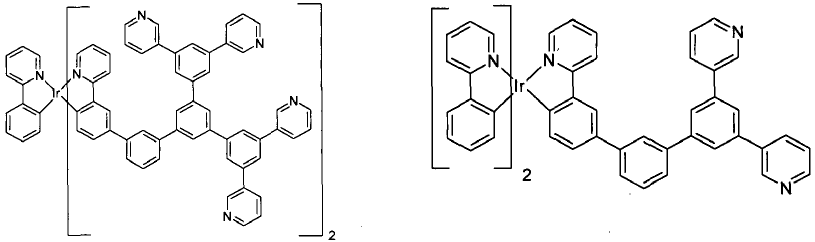

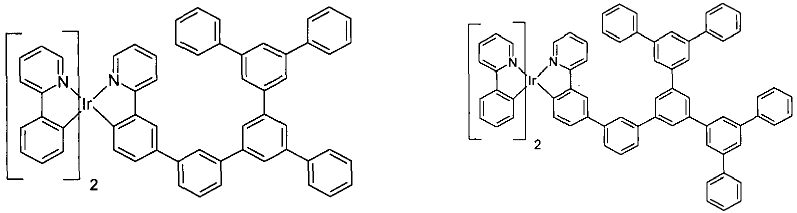

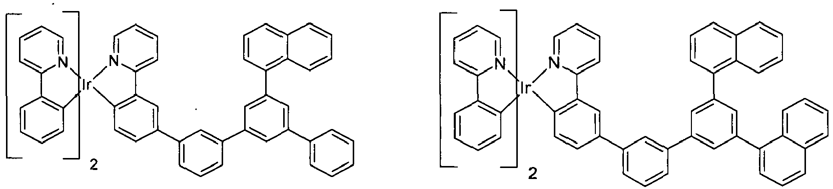

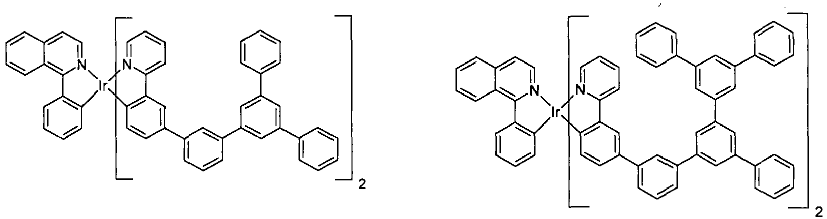

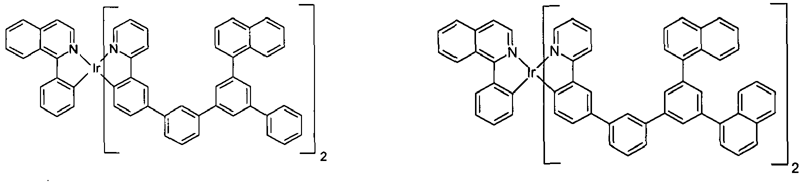

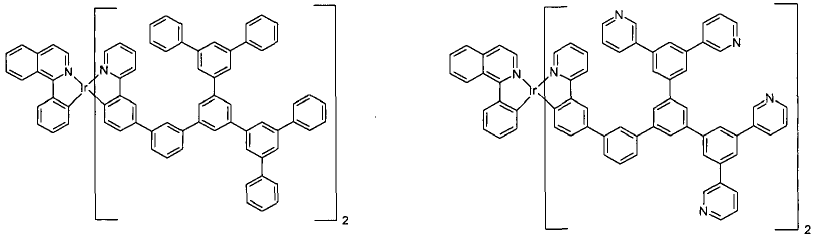

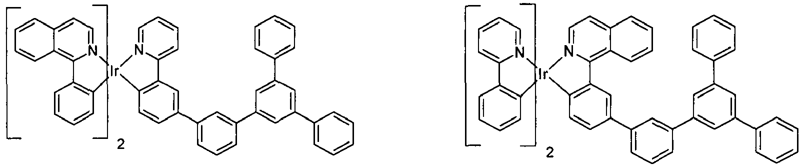

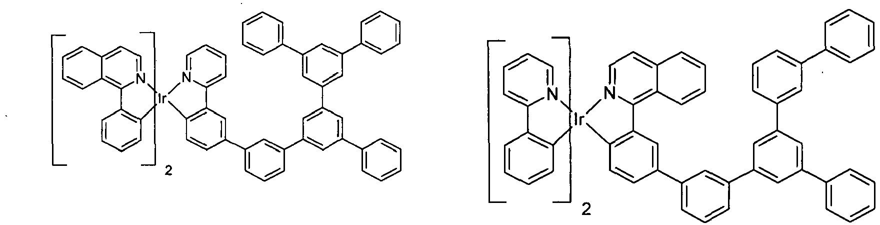

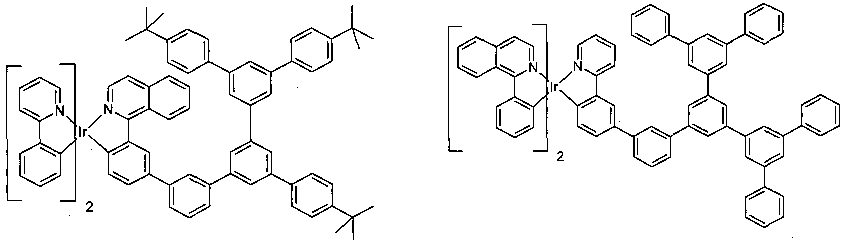

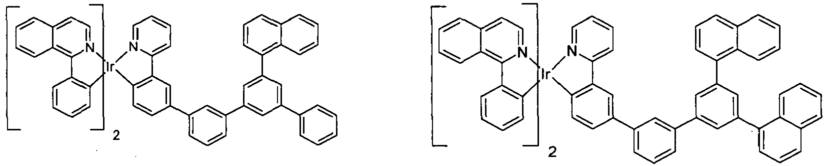

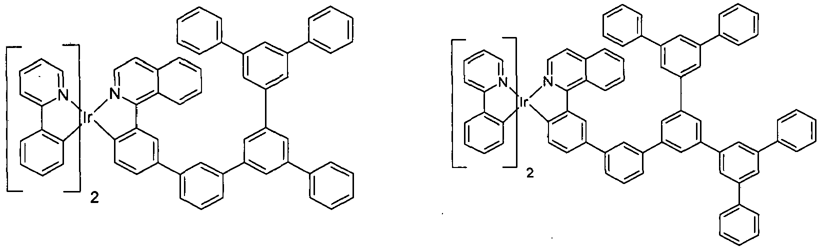

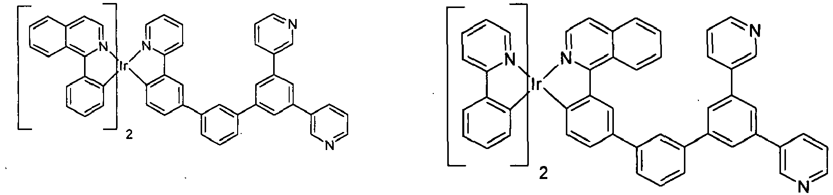

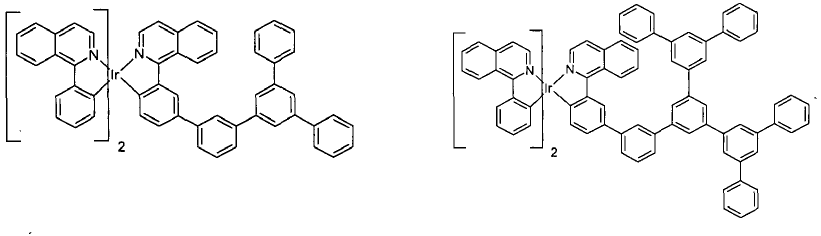

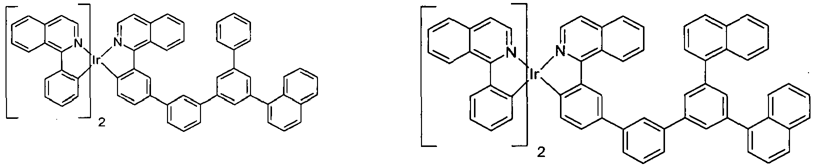

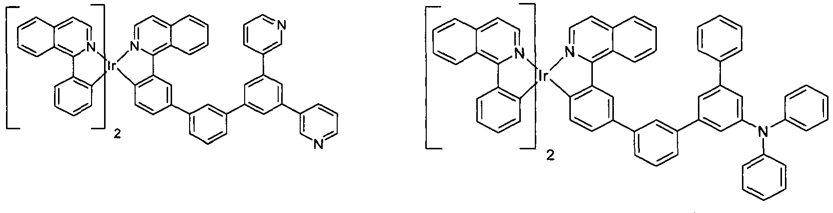

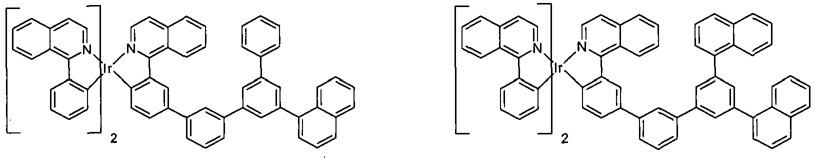

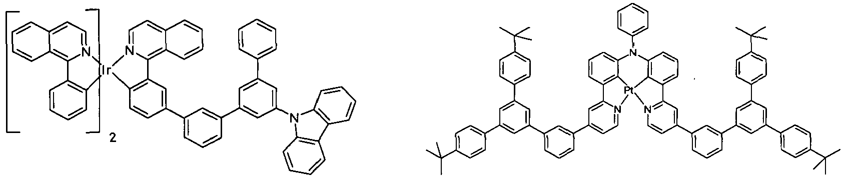

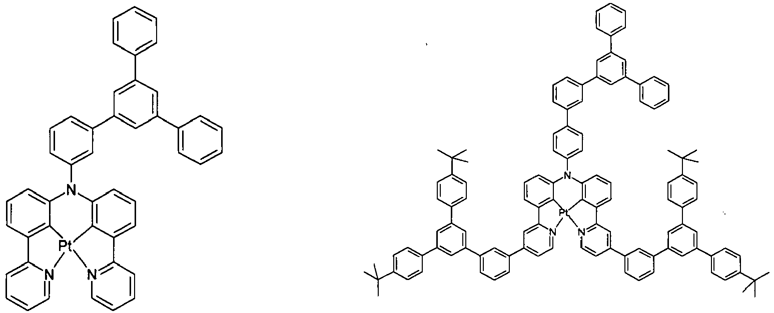

Neben den genannten Komponenten kann die erfindungsgemäße Formulierung weitere Additive und Verarbeitungshilfsmittel umfassen. Hierzu gehören unter anderem oberflächenaktive Stoffe, Tenside, Gleit- und Schmiermittel, Additive, die die Leitfähigkeit erhöhen, Dispergiermittel, Hydrophobisierungsmittel, Haftvermittler, Fließverbesserer, Entschäumer, Entlüfter, Verdünnungsmittel, die reaktiv oder unreaktiv sein können, Füllstoffe, Hilfsstoffe, Verarbeitungshilfsmittel, Farbstoffe, Pigmente, Stabilisatoren, Sensibilisatoren, Nanopartikel und Inhibitoren.

Verbindungen, welche die in der vorliegenden Anmeldung beschriebenen Struktureinheiten enthalten, finden beispielsweise zur Herstellung von OLEDs oder anderer elektro-nischer Vorrichtungen Verwendung, vorzugsweise als Lochtransport-, Lochinjektions-, Emitter-, Elektronentransport-, Elektroneninjektions-, Ladungsblockier- und/oder Ladungserzeugungsschicht.

Die Erzeugung der funktionalen Schichten kann beispielsweise durch Beschichtung aus Lösung, vorzugsweise Spincoating oder mit einem beliebigen Druckverfahren, wie z.B. Siebdruck, Flexodruck oder Offsetdruck, besonders bevorzugt aber LITI (Light Induced Thermal Imaging, Thermotransferdruck) oder Ink-Jet Druck (Tintenstrahldruck), erfolgen.

Die organische, elektronische Vorrichtung ist vorzugsweise eine organische elektrolumineszierende Vorrichtung (OLED), eine polymere elektrolumineszierende Vorrichtung (PLED), eine organische integrierte Schaltung (O-IC), ein organischer Feld-Effekt-Transistor (O-FET), ein organischer Dünnfilmtransistor (O-TFT), ein organischer, lichtemittierender Transistor (O-LET), eine organische Solarzelle (O-SC), ein organischer, optischer Detektor, ein organischer Fotorezeptor, ein organisches Feld-Quench-Device (O-FQD), eine lichtemittierende elektrochemische Zelle (LEC) oder eine organische Laserdiode (O-Laser).

Im Sinne der vorliegenden Erfindung ist es bevorzugt, dass die funktionale Verbindung als Schicht (oder in einer Schicht) in der elektronischen Vorrichtung vorliegt.

In einer weiteren Ausführungsform umfasst die Vorrichtung mehrere Schichten. Die funktionale Verbindung kann dabei vorzugsweise in einer Lochtransport-, Lochinjektions-, Elektronentransport-, Elektroneninjektions- und/oder Emissionsschicht enthalten sein. Besonders bevorzugt ist die Verwendung der funktionalen Verbindungen in der Lochtransport- und/oder Emissionsschicht.

Offenbart ist dementsprechend auch eine elektronische Vorrichtung, die mindestens drei, in einer bevorzugten Ausführungsform aber alle genannten Schichten aus Lochinjektions-, Lochtransport-, Emissions-, Elektronentransport-, Elektroneninjektions-, Ladungsblockier- und/oder Ladungserzeugungsschicht enthält und in der mindestens eine Schicht eine erfindungsgemäß einzusetzende Verbindung umfasst. Die Dicke der Schichten, beispielsweise der Lochtransport- und/oder Lochinjektionsschicht kann vorzugsweise im Bereich von 1 bis 500 nm, besonders bevorzugt im Bereich von 2 bis 200 nm liegen.

Die Vorrichtung kann ferner Schichten enthalten, welche aus weiteren niedermolekularen Verbindungen oder Polymeren aufgebaut sind. Diese können auch durch Verdampfen von niedermolekularen Verbindungen im Hochvakuum erzeugt werden.

Es kann außerdem bevorzugt sein, die einzusetzenden funktionalen Verbindungen nicht als Reinsubstanz, sondern als Mischung (Blend) zusammen mit weiteren beliebigen polymeren, oligomeren, dendritischen oder niedermolekularen Substanzen zu verwenden. Diese können beispielsweise die elektronischen Eigenschaften verbessern oder selber emittieren. Solche Mischungen sind daher ebenfalls Gegenstand der vorliegenden Anmeldung.

In einer bevorzugten Ausführungsform werden die funktionalen Verbindungen als Hostmaterialien oder Matrixmaterialien in einer emittierenden Schicht eingesetzt. Dabei kann die organische Elektrolumineszenzvorrichtung eine oder mehrere emittierende Schichten enthalten, wobei mindestens eine emittierende Schicht mindestens eine erfindungsgemäße Verbindung, wie oben definiert, enthält. Wenn mehrere Emissionsschichten vorhanden sind, weisen diese vorzugsweise mehrere Emissionsmaxima zwischen 380 nm und 750 nm auf, so dass insgesamt weiße Emission resultiert, d.h. in den emittierenden Schichten werden verschiedene emittierende Verbindungen verwendet, die fluoreszieren oder phosphoreszieren können. Ganz besonders bevorzugt sind Dreischichtsysteme, wobei die drei Schichten blaue, grüne und orange oder rote Emission zeigen (für den prinzipiellen Aufbau siehe z.B. WO 05/011013). Weiß emittierende Vorrichtungen eignen sich z.B. als Hintergrundbeleuchtung von LCD-Displays oder für generelle Beleuchtungsanwendungen.

Außer diesen Schichten kann die organische Elektrolumineszenzvorrichtung noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Excitonenblockierschichten und/oder Ladungserzeugungsschichten (Charge-Generation Layers, IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Ebenso können zwischen zwei emittierende Schichten Interlayer eingebracht sein, welche beispielsweise eine Excitonen-blockierende Funktion aufweisen. Es sei aber darauf hingewiesen, dass nicht notwendigerweise jede dieser Schichten vorhanden sein muss. Diese Schichten können ebenfalls die erfindungsgemäßen Verbindungen, wie oben definiert, enthalten. Möglich ist auch, dass mehrere OLEDs übereinander angeordnet werden, wodurch eine weitere Effizienzsteigerung hinsichtlich der Lichtausbeute erreicht werden kann. Zur Verbesserung der Lichtauskopplung kann die letzte organische Schicht auf der Lichtaustrittseite bei OLEDs beispielsweise auch als Nanoschaum ausgeführt sein, wodurch der Anteil der Totalreflexion verringert wird.

Weiterhin bevorzugt ist eine organische Elektrolumineszenzvorrichtung, wobei eine oder mehrere Schichten mit einem Sublimationsverfahren beschichtet werden. Dabei werden die Materialien in Vakuum-Sublimationsanlagen bei einem Druck kleiner 10⁻⁵ mbar, vorzugsweise kleiner 10⁻⁶ mbar, besonders bevorzugt kleiner 10⁻⁷ mbar aufgedampft.

Bevorzugt ist ebenfalls eine organische Elektrolumineszenzvorrichtung, die dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, die dadurch gekennzeichnet ist, dass eine oder mehrere Schichten aus Lösung, wie z.B. durch Spincoating, oder mit einem beliebigen Druckverfahren, wie z.B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden.

Die Vorrichtung enthält gewöhnlich eine Kathode und eine Anode (Elektroden). Die Elektroden (Kathode, Anode) werden im Sinne der vorliegenden Erfindung so gewählt, dass ihre Bandenergien möglichst gut mit denen der angrenzenden, organischen Schichten übereinstimmen, um eine möglichst effiziente Elektronen- bzw. Lochinjektion zu gewährleisten.

Als Kathode sind Metallkomplexe, Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z.B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z.B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide in Frage (z.B. LiF, Li₂O, BaF₂, MgO, NaF, etc.). Die Schichtdicke dieser Schicht liegt vorzugsweise zwischen 1 und 10 nm, besonders bevorzugt zwischen 2 und 8 nm.

Als Anode sind Materialien mit hoher Austrittsarbeit bevorzugt. Vorzugsweise weist die Anode ein Potential größer 4,5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z.B. Al/Ni/NiOₓ, Al/PtOx) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige, gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere, wie z.B Poly(ethylendioxythiophen) (PEDOT) und Polyanilin (PANI).

Offenbart ist ein Verfahren zur Herstellung einer elektronischen Vorrichtung, bei dem eine erfindungsgemäße Formulierung auf ein Substrat aufgetragen und getrocknet wird.

Vorzugsweise kann das Lösungsmittel bei einer Temperatur im Bereich von -50°C bis 300°C, besonders bevorzugt im Bereich von 20°C bis 250°C entfernt werden. Hierbei kann die Trocknung bei einem Druck im Bereich von 10⁻³ mbar bis 1 bar, besonders bevorzugt im Bereich von 10⁻² mbar bis 100 mbar erfolgen.

Die Vorrichtung wird in an sich bekannter Weise je nach Anwendung entsprechend strukturiert, kontaktiert und schließlich hermetisch versiegelt, da sich die Lebensdauer derartiger Vorrichtungen bei Anwesenheit von Wasser und/oder Luft drastisch verkürzt.

Die Erfindung wird im Folgenden anhand von Ausführungsbeispielen näher erläutert, ohne dadurch jedoch beschränkt zu werden.

### Ausführungsbeispiele

### Beispiel 1: Synthese der Verbindungen 3 und 4

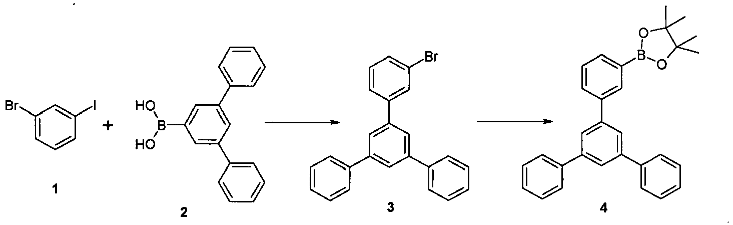

### Synthese der Verbindung 3

40.0 g (146 mmol) 3-Boronsäure-[3,1';5,1"]terphenyl 2, 18,8 g (146 mmol) 1-lod-3-bromphenyl (1) und 109,3 g (730 mmol) Kaliumcarbonat werden in 1350 ml Toluol und 1150 ml Wasser suspendiert. Zu dieser Suspension werden 844 mg (0,73 mmol) Palladium(0)tetrakis(triphenylphosphin) gegeben, und die Reaktionsmischung wird 16 Stunden unter Rückfluss erhitzt. Nach Erkalten wird die organische Phase abgetrennt, dreimal mit 200 ml Wasser gewaschen, mit Natriumsulfat getrocknet und anschließend zur Trockene eingeengt. Der Rückstand wird mit Ethanol gewaschen und aus Ethylacetat umkristallisiert und abschließend bei vermindertem Druck getrocknet. Die Ausbeute beträgt 47,6 g (123 mmol), entsprechend 84,5 % der Theorie.

### Synthese der Verbindung 4

40,0 g (104 mmol) 1-Brom-3-([3,1';5,1"]terphen-1-yl)-phenyl 3, 29,0 g (114 mmol) Bispinakolatodiboron, 29,5 g (301 mmol) Kaliumacetat werden in 800 ml Dimethylsulfoxid suspendiert. Zu dieser Suspension werden 4,24 g (5,2 mmol) 1,1-Bis(diphenylphosphino)ferrocen-dichloro-palladium(II)*DCM gegeben, und die Reaktionsmischung wird 16 Stunden unter Rückfluss erhitzt. Nach Erkalten werden 600 ml Ethylacetat und 400 ml Wasser hinzugegeben, und die organische Phase wird abgetrennt, dreimal mit 200 ml Wasser gewaschen, mit Natriumsulfat getrocknet und anschließend zur Trockene eingeengt. Das Rohprodukt wird aus Heptan umkristallisiert und abschließend bei vermindertem Druck getrocknet. Die Ausbeute beträgt 34,5 g (80 mmol), entsprechend 46,1 % der Theorie.

### Beispiel 2: Synthese der Verbindungen 5 bis 8

### Synthese der Verbindung 5

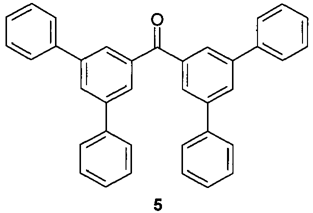

74,7 g (150 mmol) Bis(3,5-dibromphenyl)keton, 109,7 g (900 mmol) Phenylboronsäure, 267,5 g (1162 mmol) Trikaliumphosphat-Monohydrat, 5,5 g (18 mmol) Tri-*o*-tolylphosphin und 673,5 mg (3 mmol) Palladium(II)-acetat werden in einem Gemisch von 600 ml Toluol, 300 ml Dioxan und 750 ml Wasser suspendiert und für 72 Stunden zum Rückfluß erhitzt. Nach Abkühlen wird die organische Phase abgetrennt, dreimal mit Wasser gewaschen und über Natriumsulfat getrocknet. Anschließend wird über Aluminiumoxid filtriert, auf etwa 200 ml eingeengt und mit 500 ml Ethanol versetzt, wobei das Rohprodukt ausfällt. Der Feststoff wird abgesaugt und mit 100 ml Ethanol gewaschen, dann wird in siedendem Toluol gelöst und durch Zugabe von Ethanol in der Hitze wieder ausgefällt. Die Ausbeute beträgt 44,0 g (90 mmol), entsprechend 60,2 % der Theorie.

### Synthese der Verbindung 6

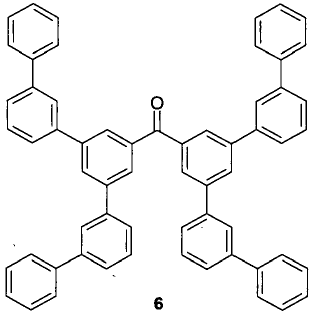

Die Synthese wird analog Verbindung **5** durchgeführt, wobei Phenylboronsaüre durch 3-Brombiphenyl ersetzt wird. Die Ausbeute beträgt 84,3 g (89 mmol), entsprechend 59,3 % der Theorie.

### Synthese der Verbindung 7

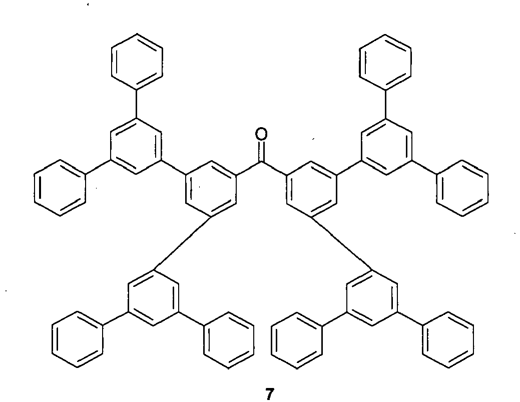

Die Synthese wird analog Verbindung **5** durchgeführt, wobei Phenylboronsaüre durch 1-Brom-[3,1';5,1"]-terphen-1-yl ersetzt wird. Die Ausbeute beträgt 105,3 g (96 mmol), entsprechend 64,0 % der Theorie.

### Synthese der Verbindung 8

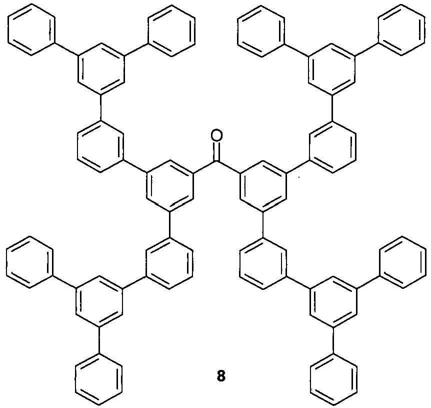

Die Synthese wird analog Verbindung **5** durchgeführt, wobei Phenylboronsaüre durch 1-Brom-3-([3,1';5,1"]-terphen-1-yl)-phenyl ersetzt wird. Die Ausbeute beträgt 123,2 g (88 mmol), entsprechend 58,7 % der Theorie.

**Vergleich der Eigenschaften**

| | Verbindung 5 | Verbindung 6 | Verbindung 7 | Verbindung 8 |
|---|---|---|---|---|
| ΔT_{g} (°C) | 0 | 8,2 | 27,0 | 39,2 |
| ΔHOMO (eV) | 0 | 0,05 | 0,02 | 0,01 |
| ΔLUMO (ev) | 0 | 0,01 | 0,00 | 0,01 |

Wie man aus den Ergebnissen erkennen kann, weist die erfindungsgemäße Verbindung 8 die gleichen Energieniveaus auf, wie vergleichbare Verbindungen gemäß dem Stand der Technik, allerdings mit einer deutlichen höheren T_{g}.

### Beispiel 2: Synthese der Verbindungen 9 bis 12

### Synthese der Verbindung 9

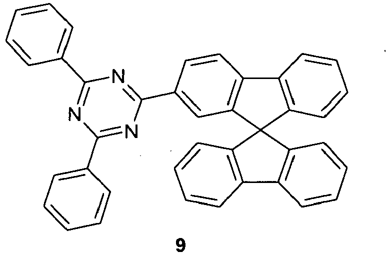

28,0 g (50,0 mmol) Spiro-9,9'-bifluoren-2-boronsäure, 14,7 g (55,0 mmol) 2-Chlor-4,6-diphenyl-1,3,5-triazin und 44,6 g (210,0 mmol) Trikaliumphosphat werden in 500 ml Toluol, 500 ml Dioxan und 500 ml Wasser suspendiert. Zu dieser Suspension werden 913 mg (3,0 mmol) Tri-o-tolylphosphin und dann 112 mg (0,5 mmol) Palladium(II)acetat gegeben, und die Reaktionsmischung wird 16 Stunden unter Rückfluss erhitzt. Nach Erkalten wird die organische Phase abgetrennt, über Kieselgel filtriert, dreimal mit 200 ml Wasser gewaschen und anschließend zur Trockene eingeengt. Der Rückstand wird aus Toluol umkristallisiert und abschließend im Hochvakuum sublimiert. Die Ausbeute beträgt 38 g (41,5 mmol), entsprechend 95,0 % der Theorie.

### Synthese der Verbindung 10

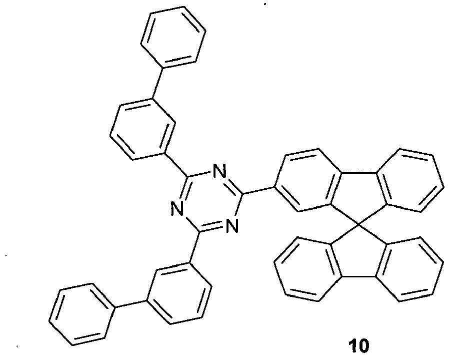

### a) Synthese von 2-Chlor-(4,6-bis-biphen-3-yl)-1,3,5-triazin

Zu einer auf -78°C gekühlten Lösung von 37,3 g (160 mmol) 3-Brombiphenyl in 250 ml abs. Tetrahydrofuran wurden 80,2 ml einer 2,0 molaren Lösung von n-Butyllithium in Hexan langsam hinzugetropft und 15 Minuten lang gerührt. Die Reaktionslösung wird langsam zu einer auf -78°C gekühlten Lösung von 10,0 g (45 mmol) Cyanurchlorid in 400 ml abs. Tetrahydrofuran getropft und die Kühlung wird entfernt. Nach Erreichen der Raumtemperatur wird das ausgefallene Produkt abfiltriert. Die Ausbeute beträgt 14,7 g (35 mmol), entsprechend 77,8 % der Theorie.

### b) Synthese von 2-(4,6-Bis-biphen-3-yl)-1,3,5-triazin-2-yl)-spiro-9,9'-bifluoren

Die Synthese wird analog Verbindung **9** mit 10,2 g (28,3 mmol) Spiro-9,9'-bifluoren-2-boronsäure durchgeführt, wobei 2-Chlor-4,6-diphenyl-1,3,5-triazin durch 10,5 g (25,0 mmol) 2-Chlor-4,6-bis-(biphen-3-yl)-1,3,5-triazin ersetzt wird. Die Ausbeute beträgt 12,7 g (17,8 mmol), entsprechend 71,3 % der Theorie.

### Synthese der Verbindung 11

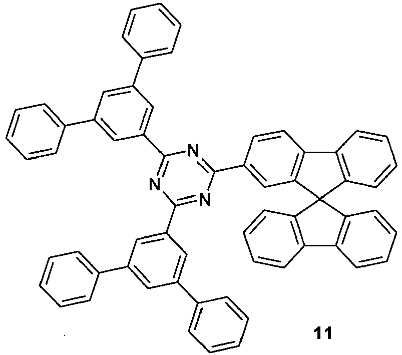

### a) Synthese von 2-Chlor-(4,6-bis-[3,1';5,1"]-terphen-1-yl)-1,3,5-triazin

Die Synthese wird analog Verbindung **10** (Stufe 1) durchgeführt, wobei 3-Brombiphenyl durch 50,3 g (163 mmol) 1-Brom-[3,1';5,1"]-terphen-1-yl ersetzt wird. Die Ausbeute beträgt 21,1 g (37 mmol), entsprechend 67,9 % der Theorie.

### b) Synthese von 2-(4,6-Bis-[3,1';5,1"]-terphen-1-yl)-1,3,5-triazin-2-yl)-spiro-9,9'-bifluoren

Die Synthese wird analog Verbindung **9** mit 17,5 g (48 mmol) Spiro-9,9'-bifluoren-2-boronsäure durchgeführt, wobei 2-Chlor-4,6-diphenyl-1,3,5-triazin durch 21,06 g (37 mmol) 2-Chlor-(4,6-Bis-[3,1';5,1"]-terphen-1-yl)-1,3,5-triazin ersetzt wird. Die Ausbeute beträgt 20,2 g (24 mmol), entsprechend 64,4 % der Theorie.

### Synthese der Verbindung 12

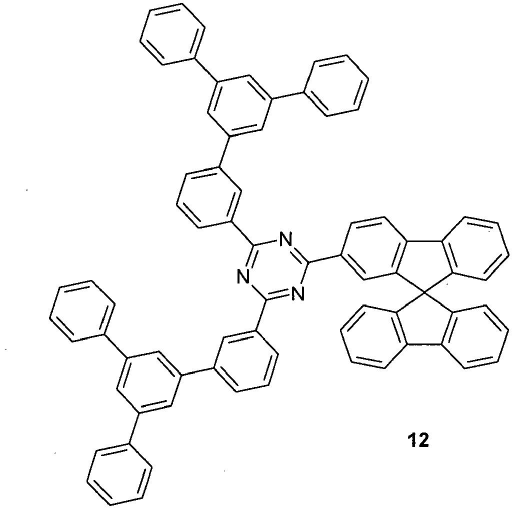

### a) Synthese von 2-Chlor-4,6-bis-(3-([3,1';5,1"]-terphen-1-yl)-phen-1-yl)-1,3,5-triazin

Die Synthese wird analog Verbindung **10** (Stufe 1) durchgeführt, wobei 3-Brombiphenyl durch 43,88 g (143 mmol) 1-Brom-3-([3,1';5,1"]-terphen-1-yl)-phenyl ersetzt wird. Die Ausbeute beträgt 6,3 g (9,0 mmol), entsprechend 23,3 % der Theorie.

### b) Synthese von 2-(4,6-Bis-(3-([3,1';5,1"]-terphen-1-yl)-phen-1-yl)-1,3,5-triazin-2-yl)-spiro-9,9'-bifluoren

Die Synthese wird analog Verbindung **9** mit 4,07 g (11,3 mmol) Spiro-9,9'-bifluoren-2-boronsäure durchgeführt, wobei 2-Chlor-4,6-diphenyl-1,3,5-triazin durch 6,3 g (9,0 mmol) 2-Chlor-4,6-bis-(3-([3,1';5,1"]-terphen-1-yl)-phen-1-yl)-1,3,5-triazin ersetzt wird. Die Ausbeute beträgt 4,9 g (4,8 mmol), entsprechend 56,3 % der Theorie.

**Vergleich der Eigenschaften**

| | Verbindung 9 | Verbindung 10 | Verbindung 11 | Verbindung 12 |
|---|---|---|---|---|
| ΔT_{g} (°C) | 0,00 | 4,20 | 24,7 | 37,3 |
| ΔHOMO (eV) | 0,00 | 0,02 | 0,02 | 0,01 |
| ΔLUMO (ev) | 0,00 | 0,01 | 0,03 | 0,02 |

Wie man aus den Ergebnissen erkennen kann, weist die erfindungsgemäße Verbindung 12 die gleichen Energieniveaus auf, wie vergleichbare Verbindungen gemäß dem Stand der Technik, allerdings mit einer deutlichen höheren T_{g}.

### Beispiel 4: Synthese der Verbindungen 13 bis 17

### Synthese der Verbindung 13

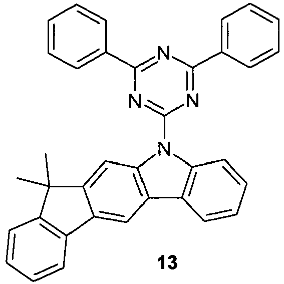

8 g (28,2 mmol) 12,12-Dimethyl-10,12-dihydro-10-aza-indeno[2,1-b]fluoren werden in 225 ml Dimethylformamid unter Schutzgasatmosphäre gelöst und mit 1,5 g NaH, 60%ig in Mineralöl, (37,5 mmol) versetzt. Nach 1 Stunde bei Raumtemperatur wird eine Lösung von 2-Chlor-4,6-diphenyl-[1,3,5]-triazin (8,5 g, 31,75 mmol) in 75 ml Dimethylformamid zugetropft. Das Reaktionsgemisch wird dann 12 Stunden bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert. Die Ausbeute beträgt 12 g (23 mmol), entsprechend 83 % der Theorie.

### Synthese der Verbindung 14

18,6 g (64,6 mmol) 12,12-Dimethyl-10,12-dihydro-10-aza-indeno[2,1-b]-fluoren werden in 400 ml Dimethylformamid unter Schutzgasatmosphäre gelöst und mit 3,1 g NaH 60%ig in Mineralöl (77,5 mmol) versetzt. Nach 1 Stunde bei Raumtemperatur wird eine Lösung von 2,4-Bis-biphenyl-3-yl-6-chlor-[1,3,5]triazine (32,6 g, 64,6 mmol) in 100 ml THF zugetropft. Das Reaktionsgemisch wird 12 Stunden bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert, aus Toluol umkristallisiert und abschließend im Hochvakuum sublimiert. Die Ausbeute beträgt 41,5 g (61 mmol), entsprechend 80 % der Theorie.

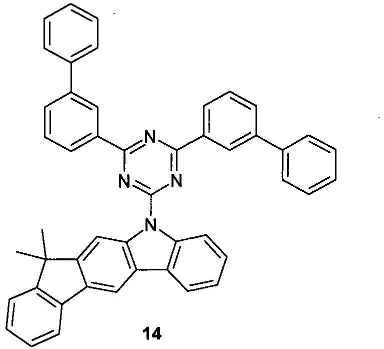

### Synthese der Verbindung 15

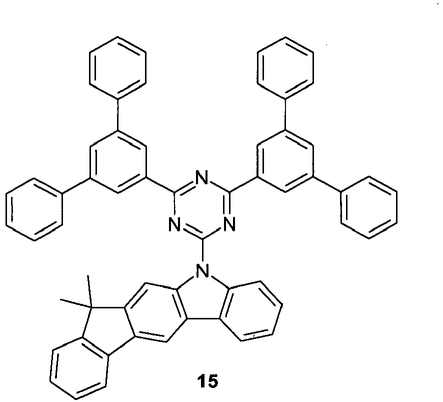

11,3 g (40 mmol) 12,12-Dimethyl-10,12-dihydro-10-aza-indeno[2,1-b]-fluoren werden in 285 ml Dimethylformamid unter Schutzgasatmosphäre gelöst und mit 1,9 g NaH 60%ig in Mineralöl (19 mmol) versetzt. Nach 1 Stunde bei Raumtemperatur wird eine Lösung von 2-Chlor-4,6-bis-[1,1';3',1"]terphenyl-5'-yl-[1,3,5]triazin (25,1 g, 44 mmol) in 315 ml Dimethylformamid zugetropft. Das Reaktionsgemisch wird 12 Stunden bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert. Die Ausbeute beträgt 23 g (28 mmol), entsprechend 70 % der Theorie.

### Synthese der Verbindung 16

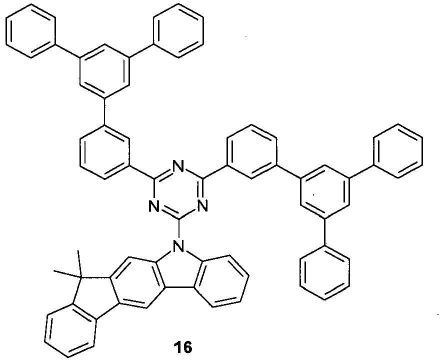

8,0 g (28 mmol) 12,12-Dimethyl-10,12-dihydro-10-aza-indeno[2,1-b]fluoren werden in 210 ml Dimethylformamid unter Schutzgasatmosphäre gelöst und mit 1,4 g NaH 60 %ig in Mineralöl (35 mmol) versetzt. Nach 1 Stunde bei Raumtemperatur wird eine Lösung von 2-Chlor-[4,6-bis-5'-(3-bromphenyl)-[1,1';3',1"]terphenyl-5'-yl]-[1,3,5]triazin (22,5 g, 31 mmol) in 250 ml Dimethylformamid zugetropft. Das Reaktionsgemisch wird 12 Stunden bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird aus Heptan/Toluol umkristallisiert. Die Ausbeute beträgt 12,2 g (13 mmol), entsprechend 44 % der Theorie.

### Synthese der Verbindung 17

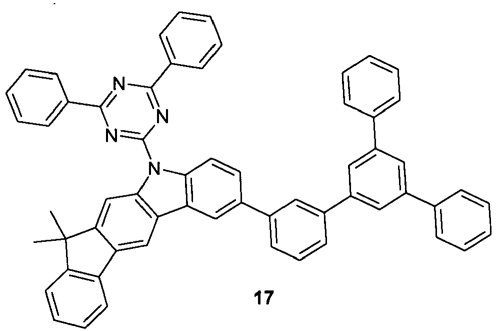

25,0 g (42,1 mmol) 7-Brom-10-(4,6-diphenyl-[1,3,5]triazin-2-yl)-12,12-dimethyl-10,12-dihydro-10-aza-indeno[2,1-b]fluoren und 19,9 g 1-Pinacolyl-boronsäureester-3-([3,1';5,1"]terphen-1-yl)-phenyl (46,3 mmol) werden in 80 ml Toluol gelöst und entgast. Es wird mit 281 ml einer entgasten 2M K₂CO₃ und mit 2,4 g (2,1 mmol) Pd(PPh₃)₄ versetzt. Die Reaktionsmischung wird anschließend bei 80°C für 48 Stunden unter Schutzgasatmosphäre gerührt. Die abgekühlte Lösung wird mit Toluol aufgestockt und mehrmals mit Wasser gewaschen, getrocknet und eingeengt. Der Rückstand wird aus Heptan/Toluol umkristallisiert. Die Ausbeute beträgt 21,8 g (26,6 mmol), entsprechend 63,2 % der Theorie.

| | Verbindung 13 | Verbindung 14 | Verbindung 15 | Verbindung 16 | Verbindung 17 |
|---|---|---|---|---|---|
| ΔT_{g} (°C) | 0,0 | 6,9 | 26,5 | 57,1 | 59,3 |
| ΔHOMO (eV) | 0,00 | 0,01 | 0,00 | 0,01 | 0,01 |
| ΔLUMO (ev) | 0,00 | 0,00 | 0,01 | 0,01 | 0,01 |

Wie man aus den Ergebnissen erkennen kann, weisen die erfindungsgemäßen Verbindungen 16 und 17 die gleichen Energieniveaus auf, wie vergleichbare Verbindungen gemäß dem Stand der Technik, allerdings mit einer deutlichen höheren T_{g}.

### Beispiel 5: Synthese der Verbindung 18

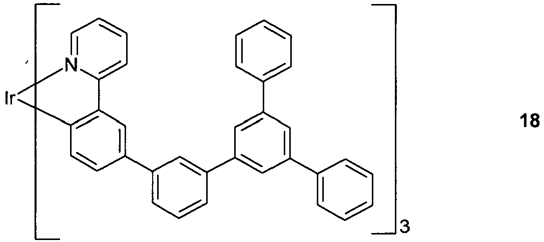

1,7 g (2,0 mmol) fac-Tris[2-(2-pyridinyl-κN)(5-bromphenyl)-κC]-iridium(III), 7,42 g (17 mmol) 1-Pinakolylboronsäureester-3-([3,1';5,1"]terphen-1-yl)-phenyl, 2,51 g (12 mmol) Kaliumphosphat werden in 100 ml Toluol, 100 ml Dioxan und 111 ml Wasser suspendiert. Zu dieser Suspension werden 4 mg (0,1 mmol) Palladium(II)acetat und 35 mg (0,2 mmol) Tri-o-tolylphosphin gegeben, und die Reaktionsmischung wird 24 Stunden unter Rückfluss erhitzt. Nach Erkalten wird die organische Phase abgetrennt, dreimal mit 200 ml Wasser gewaschen, über Kieselgel filtriert, mit Natriumsulfat getrocknet und anschließend zur Trockene eingeengt. Der Rückstand wird aus Dioxan/Ethanol umkristallisiert und abschließend bei vermindertem Druck getrocknet. Die Ausbeute beträgt 2,42 g (1,6 mmol), entsprechend 80,9 % der Theorie.

| | T-1 Ir(ppy)₃ | Verbindung 18 |
|---|---|---|
| ΔTm (°C) | 0,0 | 32,8 |
| ΔHOMO (eV) | 0,00 | 0,06 |
| ΔLUMO (ev) | 0,00 | 0,06 |

Wie man aus den Ergebnissen erkennen kann, weist die erfindungsgemäße Verbindung 18 die gleichen Energieniveaus auf, wie die vergleichbare Verbindung (Ir(ppy)₃) gemäß dem Stand der Technik, allerdings mit einer deutlichen höheren T_{g}.

### Beispiel 6: Synthese der Verbindungen 20 bis 29

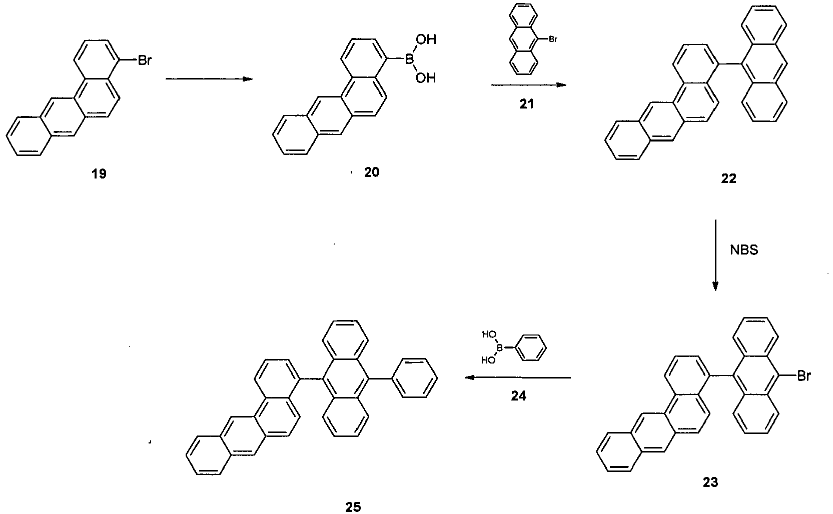

### Synthese der Verbindung 20

Eine Suspension von 30,7 g (100 mmol) 4-Brombenz[a]anthracen (**19**) in 1000 ml THF wird bei -78°C unter gutem Rühren tropfenweise mit 52 ml (130 mmol) n-Butyllithium (2,5 M in n-Hexan) versetzt und 2 Stunden nachgerührt. Die rote Lösung wird unter gutem Rühren auf ein Mal mit 16,7 ml (150 mmol) Trimethylborat versetzt, 30 Minuten bei -78°C nachgerührt, dann während 3 Stunden auf Raumtemperatur erwärmt, mit 300 ml Wasser versetzt und 30 Minuten gerührt. Die organische Phase wird abgetrennt und im Vakuum zur Trockene eingeengt. Der Feststoff wird in 100 ml n-Hexan aufgenommen, abgesaugt, einmal mit 100 ml n-Hexan gewaschen und im Vakuum getrocknet. Ausbeute: 23,7 g (87,0 mmol), entsprechend 87,0 % der Theorie, Reinheit ca. 90,0 %ig (NMR) an Boronsäure, mit wechselnden Mengen an Boronsäureanhydrid und Borinsäure. Die Boronsäure kann ohne weitere Reinigung in dieser Form verwendet werden.

### Synthese der Verbindung 22

25,0 g (97,2 mmol) 9-Bromanthracen (**21**), 27,0 g (99,2 mmol) Benz[a]-anthracen-4-boronsäure (**20**) und 44,5 g (210 mmol) Trikaliumphosphat werden in 500 ml Toluol, 600 ml Wasser und 100 ml Dioxan suspendiert. Zu dieser Suspension werden 1,83 g (6,01 mmol) Tri-o-tolylphosphin und dann 225 mg (1,0 mmol) Palladium(II)acetat gegeben und anschließend 16 Stunden unter Rückfluss erhitzt. Nach Erkalten wird die organische Phase abgetrennt, dreimal mit 500 ml Wasser gewaschen, mit Natriumsulfat getrocknet und anschließend zur Trockene eingeengt. Der Feststoff wird aus 300 ml Toluol umkristallisiert und abschließend bei vermindertem Druck getrocknet. Die Ausbeute beträgt 26,2 g (64,8 mmol), entsprechend 64,8 % der Theorie.

### Synthese der Verbindung 23

Zu einer auf 0°C gekühlten Suspension von 26,0 g (64,3 mmol) **22** in 600 ml Chloroform werden 1,3 g (8,02 mmol) Eisen(III)chlorid und dann 13,3 g (74,7 mmol) N-Bromsuccinimid gegeben und 4 Stunden bei 0°C gerührt. Nach Erwärmen auf Raumtemperatur werden 400 ml Wasser zugegeben, die organische Phase abgetrennt, dreimal mit 300 ml Wasser gewaschen, mit Natriumsulfat getrocknet und anschließend zur Trockene eingeengt. Der erhaltene orangefarbene Feststoff wird aus Toluol umkristallisiert und abschließend bei vermindertem Druck getrocknet. Die Ausbeute beträgt 23,7 g (49,0 mmol), entsprechend 76,6 % der Theorie.

### Synthese der Verbindung 25

10,0 g (20,7 mmol) **23**, 2,80 g (23,0 mmol) Phenylboronsäure (**24**) und 8,5 g (80,2 mmol) Natriumcarbonat werden in 70 ml Toluol, 56 ml Wasser und 21 ml Ethanol suspendiert. Zu dieser Suspension werden 240 mg (0,208 mmol) Tetrakis(triphenylphosphin)-palladium(0) gegeben und anschließend 16 Stunden unter Rückfluss erhitzt. Nach Erkalten wird der ausgefallene Feststoff abgesaugt, dreimal mit 50 ml Toluol, dreimal mit 50 ml Ethanol : Wasser (1:1, v:v) und dreimal mit 100 ml Ethanol gewaschen, dreimal aus DMF (ca. 10 ml/g) umkristallisiert und anschließend zweimal sublimiert. Die Ausbeute beträgt 6,07 g (12,6 mmol), entsprechend 61,3 % der Theorie.

### Synthese der Verbindung 27

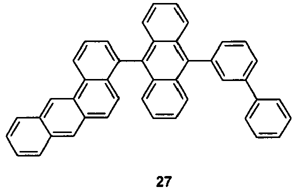

Die Synthese wird analog Verbindung **25** mit 6,0 g (12,4 mmol) Verbindung **23** durchgeführt, wobei Verbindung **24** durch 2,8 g (14,1 mmol) Biphenyl-3-boronsäure (**26**) ersetzt wird. Die Ausbeute beträgt 5,04 g (9,05 mmol), entsprechend 73,0 % der Theorie.

### Synthese der Verbindung 28

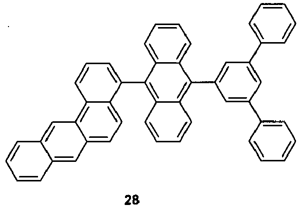

Die Synthese wird analog Verbindung **25** mit 3,0 g (6,21 mmol) Verbindung **23** durchgeführt, wobei Verbindung **24** durch 1,87 g (6,82 mmol) 3-Boronsäure-[3,1';5,1"]terphenyl (**2**) ersetzt wird. Die Ausbeute beträgt 3,41 g (5,39 mmol), entsprechend 86,8 % der Theorie.

### Synthese der Verbindung 29

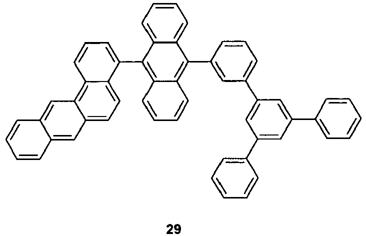

Die Synthese wird analog Verbindung **25** mit 2,0 g (4,14 mmol) Verbindung **23** durchgeführt, wobei Verbindung **24** durch 2,0 g (4,63 mmol) 1-Boronsäure-3-([3,1';5,1"]-terphen-1-yl)-phenyl (**4**) ersetzt wird. Die Ausbeute beträgt 2,72 g (3,84 mmol), entsprechend 93,8 % der Theorie.

**Vergleich der Eigenschaften**

| | Verbindung 25 | Verbindung 27 | Verbindung 28 | Verbindung 29 |
|---|---|---|---|---|
| ΔT_{g} (°C) | 0,00 | 1,9 | 23,2 | 29,0 |
| ΔHOMO (eV) | 0,00 | 0,01 | 0,00 | 0,00 |
| ΔLUMO (ev) | 0,00 | 0,00 | 0,00 | 0,00 |

Wie man aus den Ergebnissen erkennen kann, weist die erfindungsgemäße Verbindung **29** die gleichen Energieniveaus auf, wie vergleichbare Verbindungen gemäß dem Stand der Technik, allerdings mit einer deutlichen höheren T_{g}.

### Beispiel 7: Herstellung und Charakterisierung organischer Elektrolumineszenzvorrichtungen

Erfindungsgemäße Materialien werden aus Lösung verwendet und führen dort zu einfachen Devices mit dennoch guten Eigenschaften. Die Herstellung solcher Bauteile lehnt sich an die Herstellung polymerer Leuchtdioden (PLEDs) an, die in der Literatur bereits vielfach beschrieben ist (z.B. in der WO 2004/037887 A2). Im vorliegenden Fall werden die erfindungsgemäßen Verbindungen in Toluol bzw. Chlorbenzol gelöst. Die in den hier genannten Beispielen eingesetzte Konzentration beträgt 20 Gew.-% des Emitters (T-1 oder Verbindung 18) und 80 Gew.-% der Matrixmaterialien (Verbindungen 5 bis 17). Der typische Feststoffgehalt solcher Lösungen liegt zwischen 16 und 25 g/l, wenn, wie hier, die für eine Device typische Schichtdicke von 80 nm mittels Spincoating erzielt werden soll.

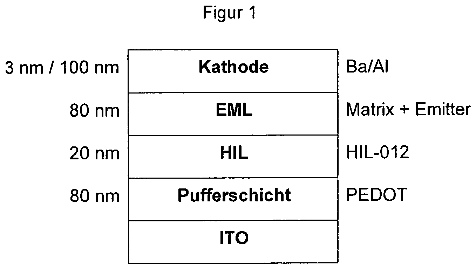

Fig. 1 zeigt den typischen Aufbau einer solchen Device. In der EML liegen die gemeinsam gelösten Matrixmaterialien sowie der Emitter in Form einer amorphen Schicht vor. Strukturierte ITO-Substrate und das Material für die sogenannte Pufferschicht (PEDOT, eigentlich PEDOT:PSS) sind käuflich erhältlich (ITO von Technoprint und anderen, PEDOT:PPS als wässrige Dispersion Clevios P von H.C. Starck). Die verwendete Interlayer (HIL-012 von Merck) dient der Lochinjektion. Die Emissionsschicht wird in einer Inertgasatmosphäre, im vorliegenden Fall Argon, aufgeschleudert und 10 Minuten bei 160°C bzw. 180°C ausgeheizt. Zuletzt wird eine Kathode aus Barium und Aluminium im Vakuum aufgedampft. Zwischen EML und Kathode können auch die in den vorgenannten Beispielen verwendeten Schichten HBL und ETL durch Bedampfung aufgebracht werden, auch kann die Interlayer durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden.

Die lösungsprozessierten Devices wurden standardmäßig charakterisiert, die genannten OLED-Beispiele wurden nicht optimiert. In Tabelle 1 sind die Ergebnisse dargestellt.

**Tabelle 1: Ergebnisse der Devicekonfiguration gemäß Figur 1**

| Bsp. | EML 80 nm | Max. Eff. [cd/A] | Spannung [V] bei 1000 cd/m² | CIE (x,y) | Lebensdauer [h], Anfangshelligkeit 1000 cd/m² |
|---|---|---|---|---|---|
| Vergl. | Verbindung 05 : T-1 | 27 | 4,2 | 0,36/0,62 | 2000 |
| Vergl. | Verbindung 06 : T-1 | 28 | 4,3 | 0,35/0,61 | 3500 |
| Vergl. | Verbindung 07 : T-1 | 30 | 4,1 | 0,35/0,62 | 4000 |
| | Verbindung 08 : T-1 | 37 | 4,2 | 0,35/0,62 | 17000 |
| Vergl. | Verbindung 09 : T-1 | 11 | 5,7 | 0,36/0,61 | 9500 |
| Vergl. | Verbindung 10 : T-1 | 14 | 5,6 | 0,36/0,61 | 10000 |
| Vergl. | Verbindung 11 : T-1 | 15 | 5,5 | 0,36/0,61 | 9000 |
| | Verbindung 12 : T-1 | 24 | 5,5 | 0,36/0,61 | 20500 |
| Vergl. | Verbindung 13 : T-1 | 14 | 4,3 | 0,33/0,62 | 3800 |
| Vergl. | Verbindung 14 : T-1 | 13 | 4,5 | 0,33/0,62 | 5200 |
| Vergl. | Verbindung 15 : T-1 | 15 | 4,4 | 0,33/0,62 | 4600 |
| | Verbindung 16 : T-1 | 26 | 4,1 | 0,34/0,62 | 18400 |
| | Verbindung 17 : T-1 | 28 | 4,2 | 0,34/0,62 | 23000 |
| Vergl. | Verbindung 09 : T-1 | 11 | 5,7 | 0,36/0,61 | 9500 |
| | Verbindungen 9 : 18 | 28 | 5,0 | 0,35/0,62 | 20500 |

Wie man aus den Ergebnissen erkennen kann, stellen die erfindungsgemäßen Verbindungen 8, 12, 16, 17 und 18 in Bezug auf Betriebsspannung, Lebensdauer und Effizienz eine deutliche Verbesserung gegenüber den vergleichbaren Verbindungen gemäß dem Stand der Technik dar.

### Beispiel 8: Herstellung und Charakterisierung organischer Elektrolumineszenzvorrichtungen - (Verbindungen 25, 27, 28, 29)

Die in den hier genannten Beispielen eingesetzte Konzentration beträgt 5 Gew.-% des Emitters (SEB-095, Merck) und 95 Gew.-% der Matrixmaterialien (Verbindungen **28** und **29**). Der typische Feststoffgehalt solcher Lösungen liegt zwischen 15 g/l, wenn, wie hier, die für eine Device typische Schichtdicke von 50 nm mittels Spincoating erzielt werden soll. In Tabelle 2 sind die Deviceergebnisse dargestellt.

**Tabelle 2: Ergebnisse der Devicekonfiguration**

| Bsp. | EML 50 nm | Max. Eff. [cd/A] | Spannung [V] bei 1000 cd/m² | CIE (x,y) | Lebensdauer [h], Anfangshelligkeit 1000 cd/m² |
|---|---|---|---|---|---|
| Vergl. | Verbindung 25 : Emitter | n.a. | n.a. | n.a. | n.a. |
| Vergl. | Verbindung 27 : Emitter | n.a. | n.a. | n.a. | n.a. |
| Vergl. | Verbindung 28 : Emitter | 5,0 | 4,8 | 0,14, 0,17 | 300 |
| | Verbindung 29 : Emitter | 6,1 | 4,9 | 0,14, 0,17 | 500 |

Aufgrund der schlechten Löslichkeit der Verbindungen **25** und **27** konnten mit diesen Matrices keine Devices hergestellt werden. Die Verbindungen **28** und **29** lassen sich jedoch durch die verbesserte Löslichkeit gut prozessieren. Wie aus der Tabelle 2 ersichtlich ist, stellt die erfindungsgemäße Verbindung **29** in Bezug auf Lebensdauer und Effizienz eine deutliche Verbesserung gegenüber der vergleichbaren Verbindung gemäß dem Stand der Technik dar.

## Patentansprüche

1. Formulierung umfassend mindestens ein Lösungsmittel und mindestens eine funktionale Verbindung der allgemeinen Formel (I)
A⁅B]ₖ (I)
wobei
A ein funktionales Strukturelement,
B ein löslichkeitsvermittelndes Strukturelement und
k eine ganze Zahl im Bereich von 1 bis 20 ist,
das löslichkeitsvermittelnde Strukturelement B der allgemeinen Formel (L-II) entspricht 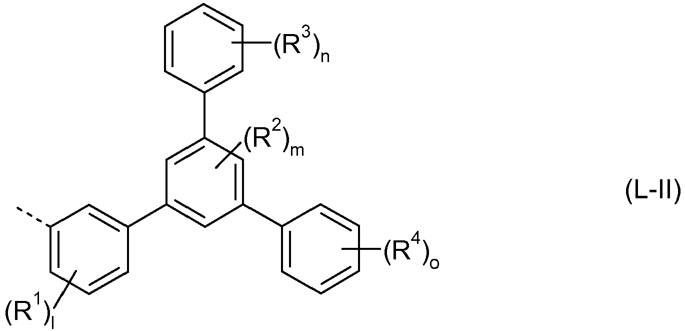 wobei
R¹, R², R³, R⁴ jeweils unabhängig voneinander eine geradkettige Alkyl- oder Alkoxygruppe mit 1 bis 20 C-Atomen oder eine verzweigte oder cyclische Alkyl- oder Alkoxygruppe mit 3 bis 20 C-Atomen ist;
l 0, 1, 2, 3 oder 4 ist;
m 0, 1, 2 oder 3 ist; und
n, o jeweils unabhängig voneinander 0, 1, 2, 3, 4 oder 5 ist;
wobei die gestrichelte Bindung die Bindung an das funktionale Strukturelement A andeutet;
wobei Verbindungen der Formel (A-III) 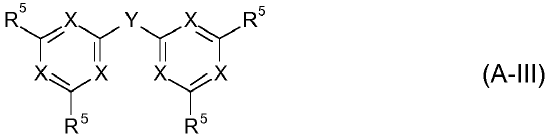 wobei für die verwendeten Symbole gilt:
Y ist C=O oder C(R⁷)₂;
X ist bei jedem Auftreten gleich oder verschieden CR⁹ oder N;
R⁵ ist bei jedem Auftreten gleich oder verschieden Wasserstoff oder ein aromatisches oder heteroaromatisches Ringsystem mit 5 bis 60 aromatischen Ringatomen, welches durch einen oder mehrere Reste R⁶ substituiert sein kann, oder eine Gruppe N(Ar)₂, Si(Ar)₃, C(=O)Ar, OAr, ArSO, ArSO₂, P(Ar)₂, P(O)(Ar)₂ oder B(Ar)₂;
R⁶ ist bei jedem Auftreten gleich oder verschieden H, D, F, Cl, Br, I, CHO, N(Ar)₂, C(=O)Ar, P(=O)(Ar)₂, S(=O)Ar, S(=O)₂Ar, CR⁷=CR⁷Ar, CN, NO₂, Si(R⁸)₃, B(OR⁸)₂, B(R⁸)₂, B(N(R⁸)₂)₂, OSO₂R⁸, eine geradkettige Alkyl-, Alkoxy- oder Thio-alkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R⁸ substituiert sein kann, wobei eine oder mehrere nicht benachbarte CH₂-Gruppen durch R⁸C=CR⁸, C=C, Si(R⁸)₂, Ge(R⁸)₂, Sn(R⁸)₂, C=O, C=S, C=Se, C=NR⁸, P(=O)(R⁸), SO, SO₂, NR⁸, O, S oder CONR⁸ ersetzt sein können und wobei ein oder mehrere H-Atome durch F, Cl, Br, I, CN oder NO₂ ersetzt sein können, oder ein aromatisches oder hetero-aromatisches Ringsystem mit 5 bis 60 aromatischen Ring-atomen, das jeweils durch einen oder mehrere Reste R⁸ substituiert sein kann, oder eine Aryloxy- oder Heteroaryloxy-gruppe mit 5 bis 60 aromatischen Ringatomen, die durch einen oder mehrere Reste R⁸ substituiert sein kann, oder eine Kombination dieser Systeme;
R⁷ ist bei jedem Auftreten gleich oder verschieden H, D, F oder eine lineare Alkylgruppe mit 1 bis 20 C-Atomen oder eine verzweigte oder cyclische Alkylgruppe mit 3 bis 20 C-Atomen; dabei können mehrere Reste R⁷ miteinander ein Ringsystem bilden;
R⁸ ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können;
R⁹ ist bei jedem Auftreten gleich oder verschieden H, D, F, CN, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R⁷ substituiert sein kann, wobei eine oder mehrere nicht benachbarte CH₂-Gruppen durch R⁷C=CR⁷, C=C, O oder S ersetzt sein können und wobei ein oder mehrere H-Atome durch F ersetzt sein können;und
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R⁶ substituiert sein kann, dabei können auch zwei Reste Ar, welche an dasselbe Stickstoff-, Phosphor- oder Boratom binden, durch eine Einfachbindung oder eine Brücke, ausgewählt aus B(R⁸), C(R⁸)2, Si(R⁸)₂, C=O, C=NR⁸, C=C(R⁸)₂, O, S, S=O, SO₂, N(R⁸), P(R⁸) und P(=O)R⁸, miteinander verknüpft sein;
ausgeschlossen sind; **dadurch gekennzeichnet, dass** das Molekulargewicht der funktionalen Verbindung mindestens 800 g/mol beträgt und
die Formulierung mindestens 80 Gew.-% aromatisches oder heteroaromatisches Lösungsmittel umfasst.

2. Formulierung nach Anspruch 1, **dadurch gekennzeichnet, dass** der Index k in Formel (I) eine ganze Zahl größer oder gleich 2 ist.

3. Formulierung nach Anspruch 1 oder 2, **dadurch gekennzeichnet, dass** die funktionale Verbindung der allgemeinen Formel (I) eine Glasübergangstemperatur von mindestens 70°C aufweist.

4. Formulierung nach einem oder mehreren der Ansprüche 1 bis 3, **dadurch gekennzeichnet, dass** das funktionale Strukturelement A in Formel (I) eine Einheit ist, welche Lochinjektions- und/oder Lochtransporteigenschaften aufweist.

5. Formulierung nach einem oder mehreren der Ansprüche 1 bis 4, **dadurch gekennzeichnet, dass** das funktionale Strukturelement A in Formel (I) eine Einheit ist, welche Elektroneninjektions- und/oder Elektronentransporteigenschaften aufweist.

6. Formulierung nach einem oder mehreren der Ansprüche 1 bis 5, **dadurch gekennzeichnet, dass** das funktionale Strukturelement A in Formel (I) eine Einheit ist, welche lichtemittierende Eigenschaften aufweist.

7. Formulierung nach Anspruch 6, **dadurch gekennzeichnet, dass** das funktionale Strukturelement A in Formel (I) eine Einheit mit phosphoreszierenden Eigenschaften ist.

8. Formulierung nach Anspruch 6 oder 7, **dadurch gekennzeichnet, dass** das funktionale Strukturelement A in Formel (I) eine Einheit ist, welche mindestens ein Schweratom mit einer Ordnungszahl von mehr als 36 umfasst.

9. Formulierung nach einem oder mehreren der Ansprüche 1 bis 8, **dadurch gekennzeichnet, dass** das Gewichtsverhältnis von Strukturelement A zu Strukturelement B in Formel (I) im Bereich von 2:1 bis 1:20 liegt.

## Claims

1. Formulation comprising at least one solvent and at least one functional compound of the general formula (I)
A⁅B]ₖ (I)
where
A is a functional structural element,
B is a solubility-promoting structural element and
k is an integer in the range from 1 to 20,
the solubility-promoting structural element B conforms to the general formula (L-II) 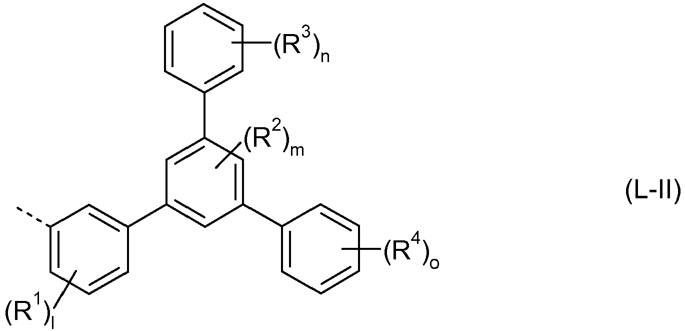 where
R¹, R², R³, R⁴ are each, independently of one another, a straight-chain alkyl or alkoxy group having 1 to 20 C atoms or a branched or cyclic alkyl or alkoxy group having 3 to 20 C atoms;
l is 0, 1, 2, 3 or 4;
m is 0, 1, 2 or 3; and
n, o are each, independently of one another, 0, 1, 2, 3, 4 or 5;
where the dashed bond indicates the bond to the functional structural element A;
where compounds of the formula (A-III) 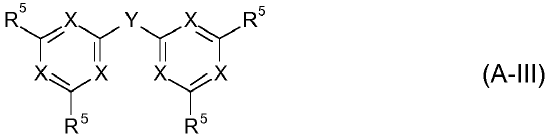 where the following applies to the symbols used:
Y is C=O or C(R⁷)₂;
X is on each occurrence, identically or differently, CR⁹ or N;
R⁵ is on each occurrence, identically or differently, hydrogen or an aromatic or heteroaromatic ring system having 5 to 60 aromatic ring atoms, which may be substituted by one or more radicals R⁶, or an N(Ar)₂, Si(Ar)₃, C(=O)Ar, OAr, ArSO, ArSO₂, P(Ar)₂, P(O)(Ar)₂ or B(Ar)₂ group,
R⁶ is on each occurrence, identically or differently, H, D, F, Cl, Br, I, CHO, N(Ar)₂, C(=O)Ar, P(=O)(Ar)₂, S(=O)Ar, S(=O)₂Ar, CR⁷=CR⁷Ar, CN, NO₂, Si(R⁸)₃, B(OR⁸)₂, B(R⁸)₂, B(N(R⁸)₂)₂, OSO₂R⁸, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R⁸, where one or more non-adjacent CH₂ groups may be replaced by R⁸C=CR⁸, C=C, Si(R⁸)₂, Ge(R⁸)₂, Sn(R⁸)₂, C=O, C=S, C=Se, C=NR⁸, P(=O)(R⁸), SO, SO₂, NR⁸, O, S or CONR⁸ and where one or more H atoms may be replaced by F, Cl, Br, I, CN or NO₂, or an aromatic or heteroaromatic ring system having 5 to 60 aromatic ring atoms, which may in each case be substituted by one or more radicals R⁸, or an aryloxy or heteroaryloxy group having 5 to 60 aromatic ring atoms, which may be substituted by one or more radicals R⁸, or a combination of these systems;
R⁷ is on each occurrence, identically or differently, H, D, F or a linear alkyl group having 1 to 20 C atoms or a branched or cyclic alkyl group having 3 to 20 C atoms; a plurality of radicals R⁷ here may form a ring system with one another;
R⁸ is on each occurrence, identically or differently, H, D or an aliphatic, aromatic and/or heteroaromatic hydrocarbon radical having 1 to 20 C atoms, in which, in addition, H atoms may be replaced by F; and
R⁹ is on each occurrence, identically or differently, H, D, F, CN, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R⁷, where one or more non-adjacent CH₂ groups may be replaced by R⁷C=CR⁷, C=C, O or S and where one or more H atoms may be replaced by F; and
Ar is on each occurrence, identically or differently, an aromatic or heteroaromatic ring system having 5 to 30 aromatic ring atoms, which may be substituted by one or more non-aromatic radicals R⁶; two radicals Ar which are bonded to the same nitrogen, phosphorus or boron atom may also be linked to one another by a single bond or a bridge selected from B(R⁸), C(R⁸)₂, Si(R⁸)₂, C=O, C=NR⁸, C=C(R⁸)₂, O, S, S=O, SO₂, N(R⁸), P(R⁸) and P(=O)R⁸;
are excluded;
**characterised in that** the molecular weight of th functional compound is at least 800 g/mol and
the formulation comprises at least 80% by weight of aromatic or heteroaromatic solvent.

2. Formulation according to Claim 1, **characterised in that** the index k in formula (I) is an integer greater than or equal to 2.

3. Formulation according to Claim 1 or 2, **characterised in that** the functional compound of the general formula (I) has a glass transition temperature of at least 70°C.

4. Formulation according to one or more of Claims 1 to 3, **characterised in that** the functional structural element A in formula (I) is a unit which has hole-injection and/or hole-transport properties.

5. Formulation according to one or more of Claims 1 to 4, **characterised in that** the functional structural element A in formula (I) is a unit which has electron-injection and/or electron-transport properties.

6. Formulation according to one or more of Claims 1 to 5, **characterised in that** the functional structural element A in formula (I) is a unit which has light-emitting properties.

7. Formulation according to Claim 6, **characterised in that** the functional structural element A in formula (I) is a unit having phosphorescent properties.

8. Formulation according to Claim 6 or 7, **characterised in that** the functional structural element A in formula (I) is a unit which contains at least one heavy atom having an atomic number of greater than 36.

9. Formulation according to one or more of Claims 1 to 8, **characterised in that** the weight ratio of structural element A to structural element B in formula (I) is in the range from 2:1 to 1:20.

## Revendications

1. Formulation comprenant au moins un solvant et au moins un composé fonctionnel de la formule générale (I) :
A⁅B]ₖ (I)
dans laquelle :
A est un élément structurel fonctionnel ;
B est un élément structurel favorisant la solubilité ; et
k est un entier dans la plage de 1 à 20 ;
l'élément structurel favorisant la solubilité B est conforme à la formule générale (L-II) : 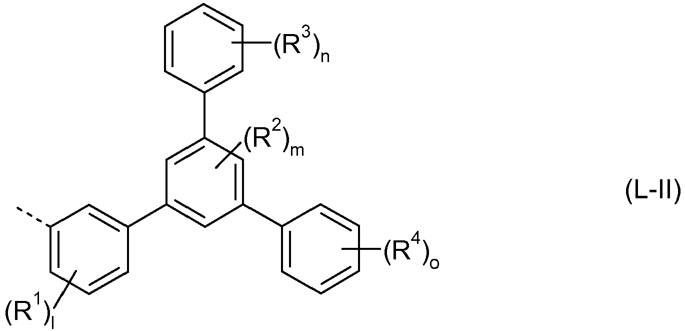 dans laquelle :
R¹, R², R³, R⁴ sont chacun, de manière indépendante les uns des autres, un groupe alkyle ou alcoxy en chaîne droite qui comporte de 1 à 20 atome(s) de C ou un groupe alkyle ou alcoxy ramifié ou cyclique qui comporte de 3 à 20 atomes de C ;
l est 0, 1, 2, 3 ou 4 ;
m est 0, 1, 2 ou 3 ; et
n, o sont chacun, de manière indépendante l'un de l'autre, 0, 1, 2, 3, 4 ou 5 ;
où la liaison en pointillés indique la liaison sur l'élément structurel fonctionnel A ;
où des composés de la formule (A-III) : 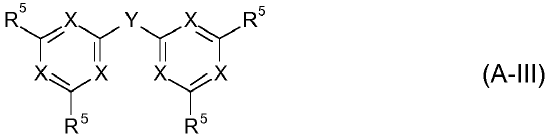 dans laquelle ce qui suit s'applique aux symboles qui sont utilisés :
Y est C=O ou C(R⁷)₂ ;
X est pour chaque occurrence, de manière identique ou différente, CR⁹ ou N ;
R⁵ est pour chaque occurrence, de manière identique ou différente, hydrogène ou un système de cycle aromatique ou hétéroaromatique qui comporte de 5 à 60 atomes de cycle aromatique, lequel peut être substitué par un radical ou par plusieurs radicaux R⁶, ou un groupe N(Ar)₂, Si(Ar)₃, C(=O)Ar, OAr, ArSO, ArSO₂, P(Ar)₂, P(O)(Ar)₂ ou B(Ar)₂ ;
R⁶ est pour chaque occurrence, de manière identique ou différente, H, D, F, Cl, Br, I, CHO, N(Ar)₂, C(=O)Ar, P(=O)(Ar)₂, S(=O)Ar, S(=O)₂Ar, CR⁷=CR⁷Ar, CN, NO₂, Si(R⁸)₃, B(OR⁸)₂, B(R⁸)2, B(N(R⁸)₂)₂, OSO₂R⁸, un groupe alkyle, alcoxy ou thio-alcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R⁸, où un ou plusieurs groupe(s) CH₂ non adjacents peut/peuvent être remplacé(s) par R⁸C=CR⁸, C=C, Si(R⁸)₂, Ge(R⁸)₂, Sn(R⁸)₂, C=O, C=S, C=Se, C=NR⁸, P(=O)(R⁸), SO, SO₂, NR⁸, O, S ou CONR⁸ et où un ou plusieurs atome(s) de H peut/peuvent être remplacé(s) par F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R⁸, où un groupe aryloxy ou hétéroaryloxy qui comporte de 5 à 60 atomes de cycle aromatique, lequel peut être substitué par un radical ou par plusieurs radicaux R⁸, ou une combinaison de ces systèmes ;
R⁷ est pour chaque occurrence, de manière identique ou différente, H, D, F ou un groupe alkyle linéaire qui comporte de 1 à 20 atome(s) de C ou un groupe alkyle ramifié ou cyclique qui comporte de 3 à 20 atomes de C ; une pluralité de radicaux R⁷ peuvent ici former un système de cycle les uns avec les autres ;
R⁸ est pour chaque occurrence, de manière identique ou différente, H, D ou un radical hydrocarbone aliphatique, aromatique et/ou hétéroaromatique qui comporte de 1 à 20 atome(s) de C, où, en outre, des atomes de H peuvent être remplacés par F ; et
R⁹ est pour chaque occurrence, de manière identique ou différente, H, D, F, CN, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R⁷, où un ou plusieurs groupe(s) CH₂ non adjacents peut/peuvent être remplacé(s) par R⁷C=CR⁷, C≡C, O ou S et où un ou plusieurs atome(s) de H peut/peuvent être remplacé(s) par F ; et
Ar est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux non aromatique(s) R⁶; deux radicaux Ar qui sont liés au même atome d'azote, au même atome de phosphore ou au même atome de bore peuvent également être liés l'un à l'autre au moyen d'une liaison simple ou d'un pont qui est sélectionné parmi B(R⁸), C(R⁸)2, Si(R⁸)₂, C=O, C=NR⁸, C=C(R⁸)₂, O, S, S=O, SO₂, N(R⁸), P(R⁸) et P(=O)R⁸ ;
sont exclus ;
**caractérisée en ce que** le poids moléculaire du composé fonctionnel est d'au moins 800 g/mol et la formulation comprend au moins 80 % en poids d'un solvant aromatique ou hétéroaromatique.

2. Formulation selon la revendication 1, **caractérisée en ce que** l'indice k dans la formule (I) est un entier supérieur ou égal à 2.

3. Formulation selon la revendication 1 ou 2, **caractérisée en ce que** le composé fonctionnel de la formule générale (I) présente une température de transition vitreuse d'au moins 70° C.

4. Formulation selon une ou plusieurs des revendications 1 à 3, **caractérisée en ce que** l'élément structurel fonctionnel A dans la formule (I) est une unité qui présente des propriétés d'injection de trous et/ou de transport de trous.

5. Formulation selon une ou plusieurs des revendications 1 à 4, **caractérisée en ce que** l'élément structurel fonctionnel A dans la formule (I) est une unité qui présente des propriétés d'injection d'électrons et/ou de transport d'électrons.

6. Formulation selon une ou plusieurs des revendications 1 à 5, **caractérisée en ce que** l'élément structurel fonctionnel A dans la formule (I) est une unité qui présente des propriétés d'émission de lumière.

7. Formulation selon la revendication 6, **caractérisée en ce que** l'élément structurel fonctionnel A dans la formule (I) est une unité qui présente des propriétés phosphorescentes.

8. Formulation selon la revendication 6 ou 7, **caractérisée en ce que** l'élément structurel fonctionnel A dans la formule (I) est une unité qui contient au moins un atome lourd qui présente un numéro atomique supérieur à 36.

9. Formulation selon une ou plusieurs des revendications 1 à 8, **caractérisée en ce que** le rapport en poids de l'élément structurel A sur l'élément structurel B de la formule (I) s'inscrit dans la plage qui va de 2:1 à 1:20.
